(19) Europäisches Patentamt
European Patent Office
Office européen des brevets

(11) Numéro de publication: **0 412 907 B1**

(12) # FASCICULE DE BREVET EUROPEEN

(45) Date de publication de fascicule du brevet: **09.11.94**

(51) Int. Cl.[5]: **C07J 41/00**, C07J 1/00, C07J 31/00, A61K 31/565

(21) Numéro de dépôt: **90402266.2**

(22) Date de dépôt: **08.08.90**

(54) **Nouveaux esters d'acides organiques avec des alcools dérivés de 19-nor stéroides et leurs sels, leur procédé de préparation et les intermédiaires de ce procédé, leur application à titre de médicaments et les compositions les renfermant.**

(30) Priorité: **08.08.89 FR 8910648**

(43) Date de publication de la demande:
**13.02.91 Bulletin 91/07**

(45) Mention de la délivrance du brevet:
**09.11.94 Bulletin 94/45**

(84) Etats contractants désignés:
**AT BE CH DE DK ES FR GB GR IT LI LU NL SE**

(56) Documents cités:
**EP-A- 0 147 361**
**EP-A- 0 192 598**
**EP-A- 0 277 676**

(73) Titulaire: **ROUSSEL-UCLAF**
**35, Boulevard des Invalides**
**F-75007 Paris (FR)**

(72) Inventeur: **Moguilewsky, Martine**
**54, rue Notre Dame de Lorette**
**F-75009 Paris (FR)**
Inventeur: **Nedelec, Lucien**
**45, Boulevard de l'Ouest**
**F-93340 Le Raincy (FR)**
Inventeur: **Nique, François**
**7, Allée Pierre Brossolette**
**F-93320 Pavillons sous Bois (FR)**
Inventeur: **Philibert, Daniel**
**16, rue Chevalier**
**F-94210 La Varenne Saint Hilaire (FR)**

(74) Mandataire: **Bourgouin, André et al**
**Département des Brevets**
**ROUSSEL UCLAF**
**111, route de Noisy**
**B.P. no 9**
**F-93230 Romainville (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 412 907 B1

Rank Xerox (UK) Business Services
(3.10/3.09/3.3.3)

## Description

La présente invention concerne de nouveaux esters d'acides organiques avec des alcools dérivés de 19-nor stéroïdes et leurs sels, leur procédé de préparation et les intermédiaires de ce procédé, leur application à titre de médicaments et les compositions les renfermant.

L'invention a pour objet, les produits de formule générale (I) :

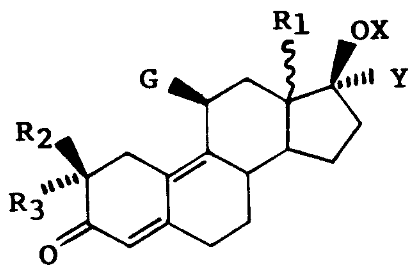

(I)

dans laquelle $R_1$ représente un radical hydrocarboné aliphatique renfermant de un à huit atomes de carbone, $R_2$ et $R_3$ identiques ou différents représentent un atome d'hydrogène ou un radical alkyle renfermant de un à quatre atomes de carbone, G représente un groupe hydrocarboné renfermant de un à dix-huit atomes de carbone et éventuellement un ou plusieurs hétéroatomes identiques ou différents, lié au noyau stéroïde par un atome de carbone et

ou bien X représente $X_A$, $X_A$ étant un atome d'hydrogène, un radical alkyle renfermant de un à huit atomes de carbone, un radical arylalkyle renfermant de sept à quinze atomes de carbone ou un radical acyle renfermant de un à huit atomes de carbone et dans ce cas Y représente $Y_A$, $Y_A$ étant un groupe

-B-O-CO-A-Z

dans lequel B représente un radical aliphatique bivalent, linéaire ou ramifié, saturé ou insaturé, renfermant de un à huit atomes de carbone, A représente ou bien un radical aliphatique bivalent, linéaire ou ramifié, saturé ou insaturé, renfermant de un à six atomes de carbone et éventuellement interrompu ou terminé par un radical aromatique bivalent, ou bien A représente un radical aromatique bivalent, et Z représente une des fonctions -COOH ou $-SO_3H$, ces fonctions pouvant être éventuellement salifiées sous forme d'un sel de métal alcalin ou alcalino-terreux, d'un sel d'ammonium ou d'un sel d'amine,

ou bien X représente $X_B$, $X_B$ étant un groupe

-CO-A-Z

dans lequel A et Z sont tels que définis précédemment et Y représente alors $Y_B$, $Y_B$ étant un des groupes choisis parmi $-C{\equiv}C-R_4$, $-CH=CH-R_4$ ou $-CH_2-CH_2-R_4$ dans lesquels $R_4$ représente un atome d'hydrogène, un atome d'halogène, un radical trialkylsilyle renfermant de trois à douze atomes de carbone, un radical alkyle renfermant de un à six atomes de carbone, linéaire ou ramifié ou un radical phényle, lesdits radicaux alkyle et phényle étant éventuellement substitués, le trait ondulé en position 13 signifie que le radical $R_1$ peut se trouver en position alpha ou béta.

$R_1$ représente de préférence un radical alkyle, linéaire ou ramifié, renfermant de un à quatre atomes de carbone comme le radical méthyle, éthyle, propyle, isopropyle ou butyle.

On préfère les produits dans lesquels $R_1$ se trouve en position béta.

Quand $R_2$ ou $R_3$ représente un radical alkyle, il s'agit de préférence du radical méthyle mais $R_2$ ou $R_3$ peut également représenter un radical éthyle, propyle, isopropyle ou butyle.

G représente notamment :

soit un radical aryle renfermant de six à quatorze atomes de carbone et éventuellement substitué par un ou plusieurs radicaux choisis parmi :

- les atomes d'halogène,
- le radical carbamoyle,
- les radicaux aliphatiques linéaires ou ramifiés, saturés ou insaturés renfermant de un à six atomes de carbone et éventuellement substitués par un ou plusieurs atomes d'halogène ou par un groupe carbamoyle, amino, alkylamino renfermant de un à quatre atomes de carbone, dialkylamino renfermant de deux à huit atomes de carbone ou par un radical hétérocyclique de trois à huit chaînons ayant au moins un atome d'azote et éventuellement un ou deux hétéroatomes choisis parmi les atomes d'oxygène ou de soufre,

- les radicaux acyles linéaires ou ramifiés renfermant de un à six atomes de carbone,
- le radical phénoxy éventuellement substitué par un groupe amino, alkylamino renfermant de un à quatre atomes de carbone, dialkylamino renfermant de deux à huit atomes de carbone ou par un radical hétérocyclique de trois à huit chaînons ayant au moins un atome d'azote et éventuellement un ou deux hétéroatomes choisis parmi les atomes d'oxygène ou de soufre,
- le radical phényle éventuellement substitué par un groupe amino, alkylamino renfermant de un à quatre atomes de carbone, dialkylamino renfermant de deux à huit atomes de carbone ou par un radical hétérocyclique de trois à huit chaînons ayant au moins un atome d'azote et éventuellement un ou deux hétéroatomes choisis parmi les atomes d'oxygène ou de soufre,
- les radicaux hétérocycliques de trois à huit chaînons comprenant au moins un atome d'azote et éventuellement un ou deux hétéroatomes choisis parmi les atomes de soufre ou d'oxygène et pouvant eux-mêmes être substitués par un ou plusieurs radicaux alkyles renfermant de un à quatre atomes de carbone,
- les radicaux $alc_1alc_2N(O)_m$-, $alc_3S(O)_p$- ou $alc_4O$- dans lesquels $alc_1$, $alc_2$, $alc_3$ et $alc_4$ identiques ou différents, représentent indépendamment les uns des autres un atome d'hydrogène, un radical alkyle linéaire ou ramifié renfermant de un à douze atomes de carbone éventuellement substitué par un groupe amino, alkylamino renfermant de un à onze atomes de carbone, dialkylamino renfermant de deux à onze atomes de carbone, trialkylsilyle renfermant de trois à onze atomes de carbone ou par un radical hétérocyclique de trois à huit chaînons comprenant au moins un atome d'azote et éventuellement un ou deux hétéroatomes choisis parmi les atomes de soufre ou d'oxygène et pouvant lui-même être substitué par un ou plusieurs radicaux alkyles renfermant de un à quatre atomes de carbone, m est égal à zéro ou un, et p est égal à zéro, un ou deux,
- les groupes trialkylsilyle renfermant de trois à douze atomes de carbone.

soit un radical aliphatique linéaire ou ramifié, saturé ou insaturé renfermant de un à dix-huit atomes de carbone et éventuellement substitué par un ou plusieurs radicaux choisis parmi :

- les atomes d'halogène,
- le radical phényle éventuellement substitué,
- les radicaux $alc_1alc_2N(O)_m$-, $alc_3S(O)_p$- ou $alc_4O$- dans lesquels $alc_1$, $alc_2$, $alc_3$, $alc_4$, m et p sont tels que définis précédemment,
- les radicaux hétérocycliques de trois à huit chaînons comprenant au moins un atome d'azote et éventuellement un ou deux hétéroatomes choisis parmi les atomes de soufre ou d'oxygène et pouvant lui-même être substitué par un ou plusieurs radicaux alkyles renfermant de un à quatre atomes de carbone,
- parmi les groupes trialkylsilyle renfermant de trois à douze atomes de carbone,

soit un radical hétérocyclique éventuellement substitué par un ou plusieurs radicaux choisis parmi :

- les atomes d'halogène,
- les radicaux alkyle linéaires ou ramifiés renfermant de un à six atomes de carbone et éventuellement substitués par un groupe carbamoyle, amino, alkylamino renfermant de un à quatre atomes de carbone, dialkylamino renfermant de deux à huit atomes de carbone ou par un radical hétérocyclique de trois à huit chaînons ayant au moins un atome d'azote et éventuellement un ou deux hétéroatomes choisis parmi les atomes d'oxygène ou de soufre,
- les radicaux $alc_1alc_2N(O)_m$-, $alc_3S(O)_p$- ou $alc_4O$- dans lesquels $alc_1$, $alc_2$, $alc_3$, $alc_4$, m et p sont tels que définis précédemment,
- les groupes trialkylsilyle renfermant de trois à douze atomes de carbone,

soit un système bicyclique condensé contenant de un à trois atomes d'azote et dont l'un est éventuellement oxydé, éventuellement substitué par un ou plusieurs radicaux choisis parmi les atomes d'halogène ou les radicaux alkyle renfermant de un à quatre atomes de carbone,

ou soit un radical cycloalkyle de trois à huit chaînons et éventuellement substitué par un ou plusieurs radicaux choisis parmi :

- les atomes d'halogène,
- le radical phényle,
- les radicaux alkyle linéaires ou ramifiés renfermant de un à six atomes de carbone et éventuellement substitués par un groupe carbamoyle, amino, alkylamino renfermant de un à quatre atomes de carbone, dialkylamino renfermant de deux à huit atomes de carbone ou par un radical hétérocyclique de trois à huit chaînons ayant au moins un atome d'azote et éventuellement un ou deux hétéroatomes

choisis parmi les atomes d'oxygène ou de soufre,

- les radicaux $alc_1alc_2N(O)_m$-, $alc_3S(O)_p$- ou $alc_4O$- dans lesquels $alc_1$, $alc_2$, $alc_3$, $alc_4$, m et p sont tels que définis précédemment,
- les groupes trialkylsilyle renfermant de trois à douze atomes de carbone.

De préférence G peut représenter un radical aryle. Ce radical aromatique peut être substitué en ortho, méta ou para par un ou plusieurs radicaux alkyles renfermant de préférence de 1 à 4 atomes de carbone ; par un ou plusieurs radicaux alkyloxy ayant préférentiellement de 1 à 4 atomes de carbone tels que les radicaux méthoxy, éthoxy, propyloxy, isopropyloxy, butyloxy, isobutyloxy, tert-butyloxy ; alkényloxy tel que vinyloxy ou 2-propényloxy ; par un ou plusieurs atomes d'halogène, de préférence chlore ou fluor ; par un ou plusieurs radicaux choisis parmi les radicaux hydroxyle, (2-amino 2-oxo éthyle), trifluorométhyle, alkylthio ayant de 1 à 4 atomes de carbone éventuellement oxydé sous forme de sulfoxyde ou de sulfone tel que méthylthio, éthylthio ; amino, alkylamino ayant de un à quatre atomes de carbone tel que méthylamino, éthylamino, dialkylamino ayant de deux à huit atomes de carbone éventuellement oxydés sous forme de N-oxyde tel que le radical diméthylamino diéthylamino ou (N-méthyl éthylamino), par un radical acyle tel que formyle, acétyle, propionyle, butyryle ou benzoyle, de préférence acétyle ; bien entendu les radicaux susbstituant le radical aryle peuvent aussi être éventuellement substitués tel que décrit précédemment ; de même le radical aryle peut être substitué par une combinaison de ces différents radicaux tel que par exemple le (3-fluoro 4-diméthylamino phényle) ;

G peut également représenter un radical hétérocyclique éventuellement substitué par les différents radicaux envisagés ci-dessus. On peut citer les radicaux thiényle, furyle, isothiényle, isofuryle, thiazolyle, isothiazolyle, oxazolyle, isoxazolyle, thiadiazolyle, pyridyle, pipéridinyle ou pyrrolidinyle ou pipérazinyle et les hétérocycles connus de l'homme de métier ;

G peut également représenter un radical bicyclique condensé comportant un noyau phényle et un noyau hétérocyclique contenant au moins un atome d'azote éventuellement substitué par les radicaux envisagés précédemment ; on peut citer le radical N-méthyl 2,3-dihydro indol-5-yle ;

G peut également représenter un radical cycloalkyle tel que cyclopropyle, cyclobutyle, cyclopentyle ou cyclohexyle, cycloalkényle tel que cyclobutényle ou cyclopropényle ;

G peut représenter également un radical comportant un noyau arylique substitué soit par une fonction amine éventuellement substituée par un ou deux radicaux alkyle ayant de 1 à 8 atomes de carbone, soit par un radical aminé incorporé dans un hétérocycle comportant éventuellement un autre hétéroatome choisi dans le groupe formé par l'oxygène, l'azote et le soufre, tel que les radicaux morpholino, pipéridinyle, pyrrolidinyle ou 4-méthyl pipérazinyle.

Le noyau arylique est alors de préférence le noyau phényle.

Comme substituant sur le noyau arylique on peut également envisager un radical amino (substitué) alkyle, tel que le radical diméthylamino méthyle, diméthylamino éthyle ; un radical amino (substitué) alkyloxy tel que le radical diméthylamino éthyloxy.

On peut également citer les radicaux comportant un atome de silicium tel que le radical triméthylsilyl phényle ou le radical [N-méthyl (1-triméthylsilyl) méthylamino] phényle.

Les radicaux précédemment cités comportant un atome d'azote peuvent être oxydés.

G peut également représenter un radical alkyle saturé ou insaturé, linéaire ou ramifié ayant de 1 à 18 atomes de carbone.

Il s'agit alors de préférence du radical méthyle, éthyle, propyle, isopropyle, butyle, isobutyle ou tert-butyle, n-pentyle, n-hexyle, 2-méthyl pentyle, 2,3-diméthyl butyle, n-heptyle, 2-méthylhexyle, 2,2-diméthylpentyle, 3,3-diméthyl pentyle, 3-éthylpentyle, n-octyle, 2,2-diméthylhexyle, 3,3-diméthylhexyle, 3-méthyl-3-éthylpentyle, nonyle, 2,4-diméthyl heptyle, ou n-décyle. Il peut s'agir également des radicaux vinyle, isopropényle, allyle, 2-méthylallyle ou isobutényle.

Les radicaux précités peuvent être substitués. Parmi les substituants possibles on peut citer les radicaux thioalkyle tels que thiométhyle ou thioéthyle ; G peut également être substitué par un ou plusieurs atomes d'halogènes tels que fluor, chlore, brome, iode, ou par les radicaux amino substitués tels que diméthylamino.

De manière générale, on préfère les produits dans lesquels le substituant G comporte un hétéroatome de préférence l'azote, l'oxygène ou le soufre.

Lorsque X représente $X_A$, $X_A$ étant un radical alkyle, il s'agit de préférence du radical méthyle, éthyle ou propyle.

Lorsque X représente $X_A$, $X_A$ étant un radical arylalkyle, il s'agit de préférence du radical benzyle ou phénéthyle.

Lorsque X représente $X_A$, $X_A$ étant un radical acyle, il s'agit de préférence du radical formyle, acétyle, propionyle, butyryle ou benzoyle.

B représente de préférence soit un radical bivalent saturé comme le radical méthylène, éthylène, triméthylène ou tétraméthylène, soit un radical bivalent insaturé comme le radical vinylène, propénylène, buténylène, éthynylène, propynylène ou butynylène.

A représente de préférence le radical méthylène, éthylène, triméthylène ou tétraméthylène ; lorsqu'il est interrompu par un cycle aromatique, A représente alors de préférence le radical :

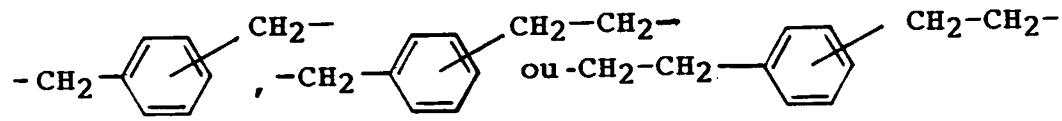

lorsqu'il est terminé par un cycle aromatique, il s'agit de préférence du radical :

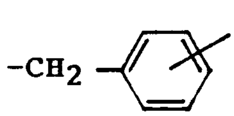

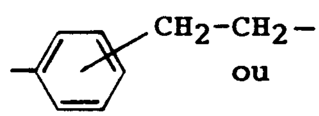

ou

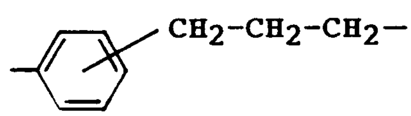

Lorsque A représente un radical aromatique bivalent, il s'agit de préférence du radical ortho-phénylène, méta-phénylène ou para-phénylène ou encore du radical

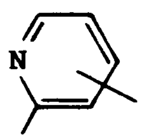

ou

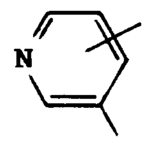

Lorsque Z représente un groupe carboxy ou sulfo salifié, il s'agit de préférence du sel de sodium, de potassium, de calcium, de magnésium, d'ammonium, ou d'un sel d'amine utilisée dans la fabrication des médicaments, comme les sels de lysine, d'arginine, de cystéïne, de bétaïne, de carnitine, de méglumine, de quinine, de sarcosine, de procaïne, d'histidine ou de N-méthyl glucamine.

Lorsque $R_4$ représente un radical alkyle, il s'agit de préférence d'un radical méthyle, éthyle, propyle, butyle, isopropyle ou isobutyle.

L'invention a notamment pour objet les produits de formule générale (I) telle que définie précédemment et répondant à la formule (I') :

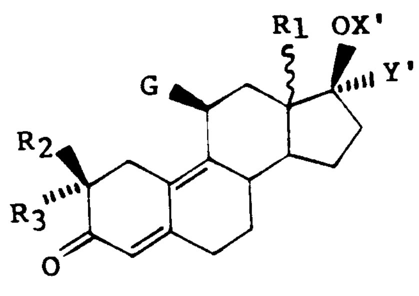

(I')

dans laquelle

<u>ou bien</u> X′ représente un atome d'hydrogène et dans ce cas Y′ représente $Y'_A$, $Y'_A$ étant un groupe :

-B′-O-CO-A′-Z′

dans lequel B′ représente un des radicaux bivalents $-CH=CH-CH_2-$ ou $-C{\equiv}C-CH_2-$, A′ représente un des radicaux bivalents $-(CH_2)_n-$ dans lequel n peut prendre une des valeurs de deux à six ou un radical ortho-, méta- ou para-phénylène et Z′ représente les fonctions carboxy ou sulfo ou leur sel de sodium,

ou bien X′ représente $X'_B$, $X'_B$ étant un groupe :

$-CO-(CH_2)_n-Z'$

dans lequel n et Z′ sont tels que définis précédemment et Y′ représente alors $Y'_B$, $Y'_B$ étant un des groupes $-C \equiv C-R'_4$ ou $-CH=CH-R'_4$ dans lesquels $R'_4$ représente un atome d'hydrogène, un atome d'halogène, un radical triméthylsilyle, ou un radical méthyle éventuellement substitué par un ou plusieurs atomes d'halogène, un radical hydroxy ou par un groupe alkyloxy, alkylthio ou alkylamino renfermant de un à quatre atomes de carbone, dialkylamino renfermant de deux à huit atomes de carbone ou trialkylsilyle renfermant de trois à douze atomes de carbone.

n prend de préférence une des valeurs de deux à quatre ; quand $R'_4$ représente un radical trialkylsilyle, il s'agit de préférence du radical triméthylsilyle.

Lorsque $R'_4$ représente le radical méthyle éventuellement substitué, il s'agit de préférence du radical méthyle, (fluorométhyle), (trifluorométhyle), (chlorométhyle), (bromométhyle), (diméthylamino méthyle), (hydroxyméthyle), (méthoxyméthyle), ou (méthylthiométhyle).

Lorsque $R'_4$ représente un atome d'halogène il s'agit de preférence d'un atome de chlore, de brome ou d'iode.

Plus précisément G représente un radical aryle substitué par un atome d'halogène, un radical aryle, un radical acyle renfermant de un à huit atomes de carbone ou par une des fonctions $-NH_2$, -NHR′ ou -NR′R″, dans lesquelles R′ et R″ identiques ou différents représentent un radical phényle, ou un radical alkyle primaire, secondaire ou tertiaire renfermant de 1 à 8 atomes de carbone, comportant éventuellement un ou plusieurs hétéroatomes, choisis parmi les atomes d'oxygène, d'azote et de soufre, ou éventuellement substitué par un hétérocycle, ou R′ et R″ représentent avec l'atome d'azote auquel ils sont liés, un radical hétérocyclique éventuellement substitué et comportant éventuellement un autre hétéroatome choisi parmi les atomes d'azote, d'oxygène et de soufre, ou bien par une des fonctions -0R‴ ou -SR‴ dans lesquelles R‴ représente un radical phényle ou un radical alkyle primaire, secondaire ou tertiaire renfermant de 1 à 8 atomes de carbone, comportant éventuellement un ou plusieurs hétéroatomes, choisis parmi les atomes d'oxygène, d'azote, de soufre ou éventuellement substitué par un hétérocycle, ou G représente un radical 2, 3 ou 4-pyridyle.

L'invention a particulièrement pour objet les produits de formules (I) et (I′) telles que définies précédemment et dans lesquelles G représente un radical phényle substitué en position 4 par un radical amino, (méthylamino), (diméthylamino) ou son N-oxyde, (diéthylamino), (dipropylamino), (N-méthyl éthylamino), (N-méthyl isopropylamino), (N-méthyl isobutylamino), (N-méthyl isopentylamino), 1-pyrrolidinyle, [2-(diméthylamino) N-méthyl éthylamino], [N-méthyl 2-(1-pyrrolidinyl) éthylamino], [N-méthyl 2-(4-morpholinyl) éthylamino], (4-méthyl 1-pipérazinyle), formyle, acétyle, méthoxy, phénoxy, [2-(diméthylamino) éthoxy], [2-(1-pyrrolidinyl) éthoxy], [2-(4-morpholinyl) éthoxy], (méthylthio), (éthylthio), (isopentylthio), [2-(diméthylamino) éthylthio], [2-(1-pyrrolidinyl) éthylthio] [2-(4-morpholinyl) éthylthio], (triméthylsilyle), méthyle, isopropyle, [(diméthylamino) méthyle], ou par un des atomes de fluor, de chlore ou de brome.

L'invention a spécialement pour objet les produits de formule (I) telle que définie précédemment, dans laquelle $R_1$ représente un radical méthyle en position 13béta et $R_2$ et $R_3$ chacun un atome d'hydrogène, et tout particulièrement ceux dont les noms suivent :

- succinate de sodium et de 21-chloro 11béta-[4-(diméthylamino) phényl] 3-oxo 19-nor 17alpha-pregna-4,9-dièn-20-yn-17béta-yle,
- succinate de sodium et de 11béta-[4-(méthylthio) phényl] 3-oxo 17alpha-(1-propynyl) estra-4,9-dièn-17béta-yle.

L'invention a également pour objet un procédé de préparation des produits de formule (I) telle que définie précédemment et caractérisé en ce que :

A - les produits de formule ($II_A$) ou de formule ($II_B$) :

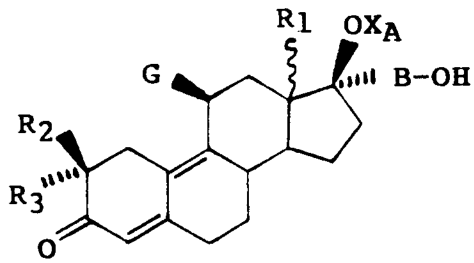

($II_A$)

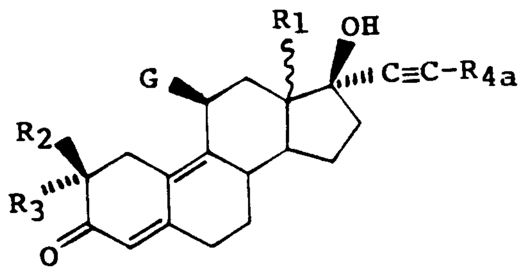

($II_B$)

dans lesquelles $X_A$, $R_1$, $R_2$, $R_3$ et G ont la signification indiquée ci-dessus et $R_{4a}$ a les valeurs indiquées ci-dessus pour $R_4$ ainsi que ces valeurs dans lesquelles les fonctions réactives sont protégées, sont soumis, dans un solvant neutre et en présence d'une base,

a) à l'action d'un produit de formule ($III_1$) :

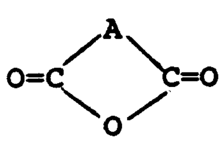

($III_1$)

pour obtenir après si nécessaire déprotection des fonctions réactives protégées et si désiré salification de la fonction carboxy, les produits de formule générale (I) répondant respectivement aux formule ($I_{A1}$) et ($I_{B1}$) :

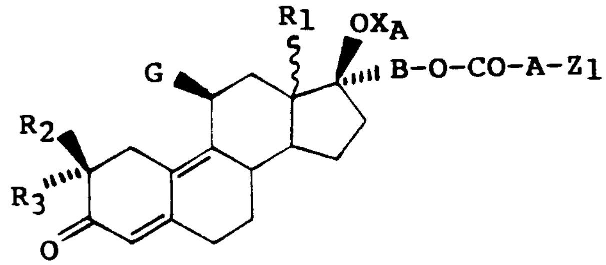

$(I_{A1})$

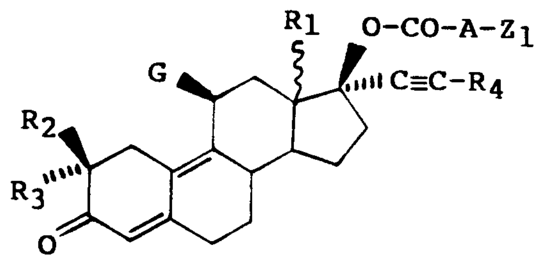

$(I_{B1})$

dans lesquelles $Z_1$ représente un radical carboxy éventuellement salifié,
b) à l'action d'un produit de formule $(III_2)$ :

HOOC-A-U $(III_2)$

ou encore d'un dérivé fonctionnel de cet acide dans laquelle U représente un des groupes -COOH, $-COOR_5$ dans lequel $R_5$ représente un radical alkyle renfermant de un à six atomes de carbone ou un radical arylalkyle renfermant de sept à douze atomes de carbone, ou -SH, pour obtenir, après si nécessaire déprotection des fonctions réactives protégées, respectivement les produits de formules $(IV_A)$ et $(IV_B)$ :

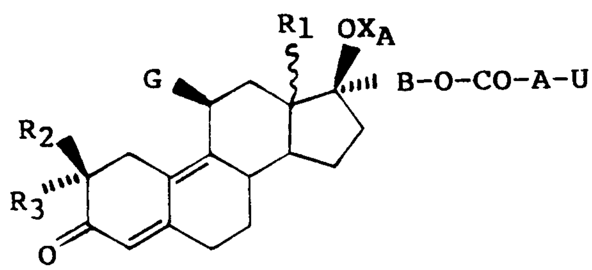

$(IV_A)$

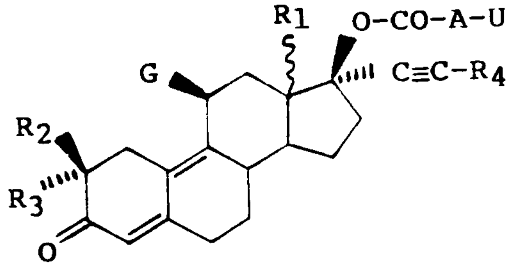

$(IV_B)$

produits de formules $(IV_A)$ et $(IV_B)$ qui :

- dans le cas où U représente le groupe carboxy libre, correspondent après salification éventuelle, aux produits de formules $(I_{A1})$ et $(I_{B1})$,
- dans le cas où U représente le groupe $-COOR_5$, correspondent aux produits de formules $(IV_C)$ et $(IV_D)$ :

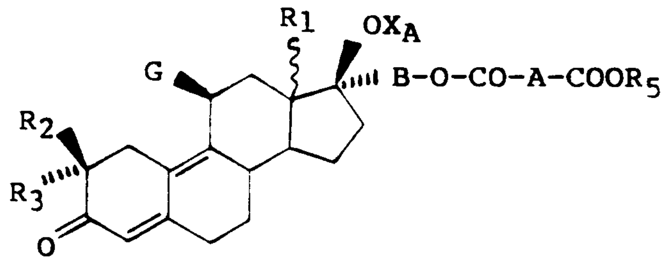

(IV$_C$)

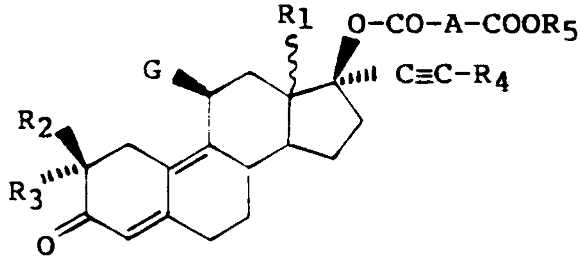

(IV$_D$)

produits de formules (IV$_C$) et (IV$_D$) qui sont hydrolysés ou saponifiés pour obtenir respectivement les produits de formules ($I_{A1}$) et ($I_{B1}$),

- dans le cas où U représente le groupe -SH, correspondent aux produits de formules (IV$_E$) et (IV$_F$) :

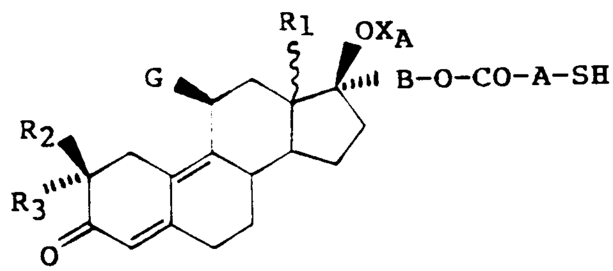

(IV$_E$)

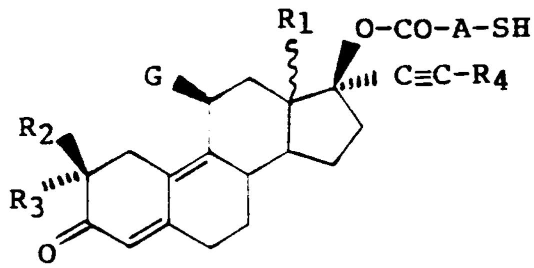

(IV$_F$)

produits de formules (IV$_E$) et (IV$_F$) qui sont oxydés et si désiré salifiés pour obtenir les produits de formule générale (I) répondant respectivement aux formules ($I_{A2}$) et ($I_{B2}$) :

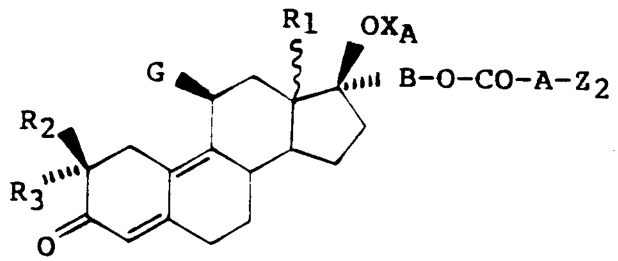

$(I_{A2})$

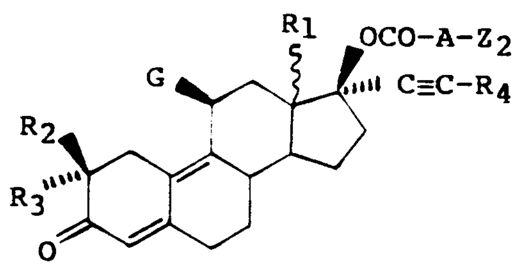

$(I_{B2})$

dans lesquelles $Z_2$ représente un groupe sulfo éventuellement salifié,

c) ou à l'action d'un produit de formule $(III_3)$ :

$HOOC\text{-}A\text{-}SO_2Cl$ $(III_3)$

ou encore d'un dérivé fonctionnel de cet acide, pour obtenir si nécessaire après déprotection des fonctions réactives contenues dans $R_{4a}$ et si désiré salification, respectivement les produits de formule $(I_{A2})$ et $(I_{B2})$ ;

en ce que :

B - les produits de formule $(I_{B1})$, $(I_{B2})$ et $(IV_D)$ sont soumis si désiré :

a) soit à un agent d'hydrogénation des triples liaisons pour obtenir respectivement les produits de formules $(I_{B3})$, $(I_{B4})$ et $(V_D)$ :

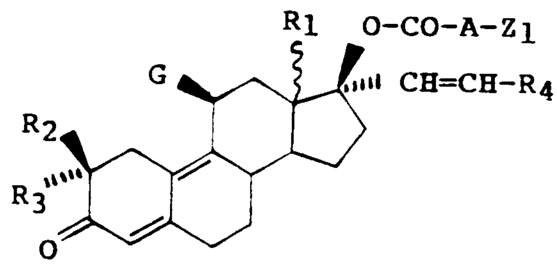

$(I_{B3})$

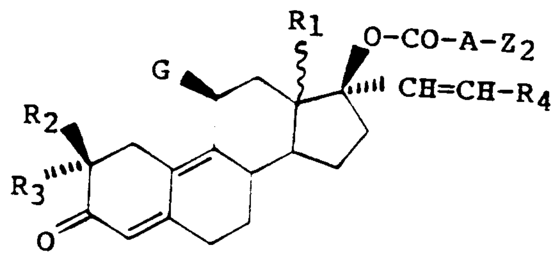

$(I_{B4})$

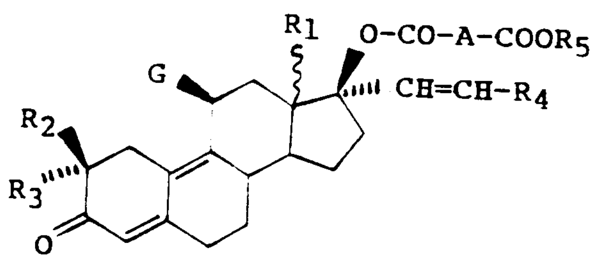

$(V_D)$

produits qui sont si désiré soumis à un agent d'hydrogénation des doubles liaisons pour obtenir respectivement des produits de formule $(I_{B5})$, $(I_{B6})$ et $(VI_D)$ :

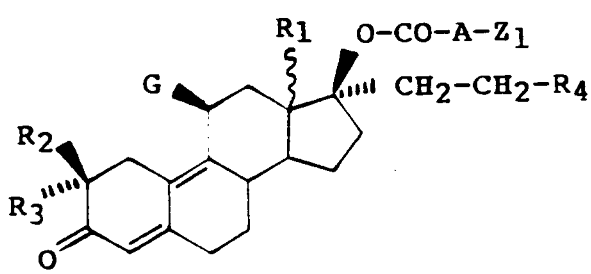

$(I_{B5})$

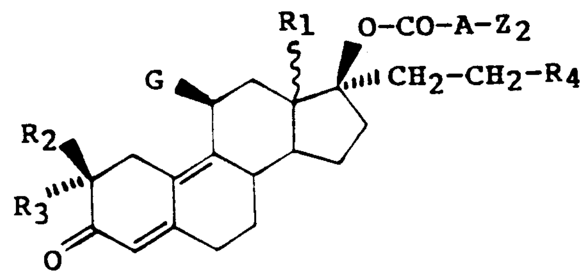

$(I_{B6})$

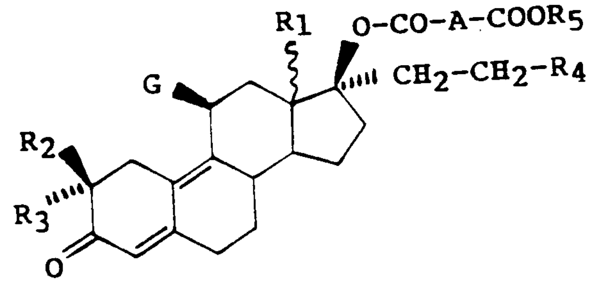

$(VI_D)$

soit à un agent d'hydrogénation directe des triples liaisons en simples liaisons pour obtenir respectivement les produits de formules ($I_{B5}$), ($I_{B6}$) et ($VI_D$).

b) les produits de formules ($V_D$) et ($VI_D$) sont soit hydrolysés, soit saponifiés pour obtenir respectivement les produits de formules ($I_{B3}$) et ($I_{B5}$).

Dans un mode de réalisation préféré du procédé ci-dessus décrit l'action de ($III_1$) sur ($II_A$) et ($II_B$) est effectuée en présence d'une base et dans un solvant neutre, la fonction hydroxy éventuellement contenue dans $R_4$ est protégée sous forme d'un groupe (2-tétrahydropyrannyloxy) par action du 2,3-dihydropyranne.

La déprotection ultérieure de cette fonction a lieu par passage sur une résine sulfonique (forme acide) du commerce ou par traitement acide. Le solvant neutre servant de milieu réactionnel peut être le chloroforme, le chlorure de méthylène, l'acétonitrile ou l'éther éthylique ; la base utilisée est de préférence une base azotée telle que la triéthylamine, la diisopropyléthylamine, la 4-diméthylaminopyridine, la pyridine ou la N-méthyl morpholine.

L'oxydation du groupe mercapto en groupe sulfo se fait par l'acide nitrique, le nitrate de baryum ou par auto-oxydation en milieu basique.

L'agent d'hydrogénation de la triple liaison en double liaison est l'hydrogène en présence d'un catalyseur tel que le palladium sur sulfate de baryum. L'agent d'hydrogénation des double ou triple liaisons en liaison simple est l'hydrogène en présence d'un catalyseur tel le palladium sur charbon actif ou le chloro tris(triphénylphosphine) rhodium.

Par dérivés fonctionnels des acides de formule ($III_2$) ou ($III_3$), on comprend les anhydrides obtenus "in situ" par action d'un chloroformiate d'alkyle comme le chloroformiate d'isobutyle, ou d'un dicycloalkylcarbodiimide comme le dicyclohexylcarbodiimide.

L'hydrolyse et la saponification de la fonction $-COOR_5$, ainsi que la salification des fonctions carboxy ou sulfo sont réalisées de façon usuelle.

Dans un mode d'exécution préféré du procédé décrit précédemment pour la préparation des produits de formule (I'),

- a) soit l'on soumet le produit de formule ($II'_A$) ou ($II'_B$) :

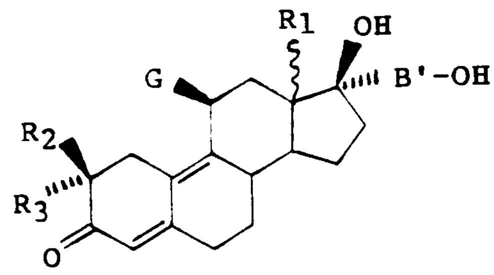

(II'A)

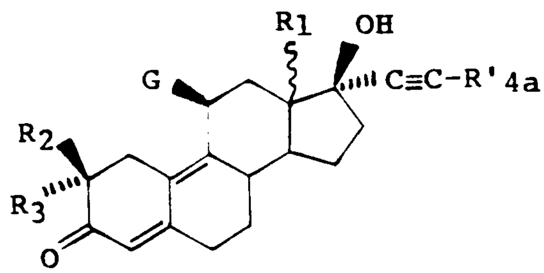

(II'B)

dans laquelle $R_1$, $R_2$, $R_3$ et G ont les valeurs indiquées ci-dessus et $R'_{4a}$ a les valeurs indiquées ci-dessus pour $R'_4$ ainsi que ces valeurs dans lesquelles les fonctions hydroxylées sont protégées, dans un solvant neutre et en présence d'une base à l'action d'un produit de formule ($III'_1$) :

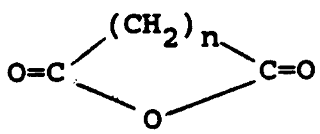

(III'1)

dans laquelle n prend une des valeurs de deux à six pour obtenir, après si nécessaire déprotection de la fonction hydroxy protégée contenue dans $R'_4$ et si désiré, salification de la fonction carboxy libre par action d'une solution de bicarbonate de sodium, respectivement le produit de formule ($I'_{A1}$) ou ($I'_{B1}$) :

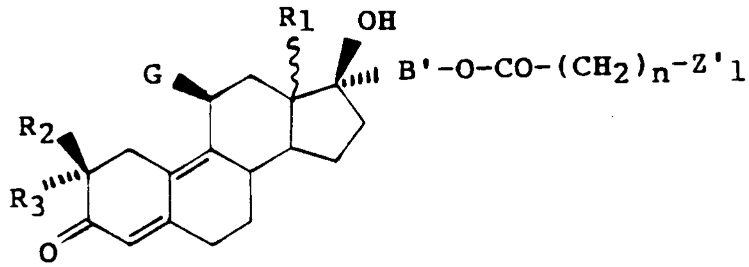

(I'$_{A1}$)

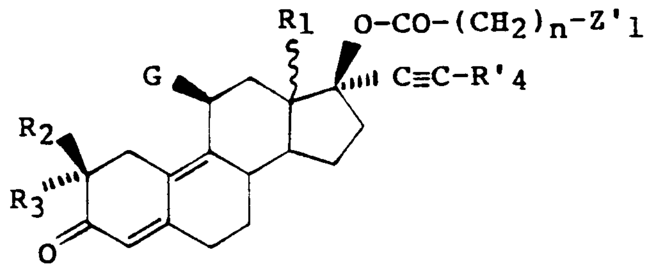

(I'$_{B1}$)

dans lesquelles $Z'_1$ représente un groupe carboxy libre ou son sel de sodium.

b) soit l'on soumet le produit de formule (II'$_A$) telle que définie ci-dessus à l'action, en présence d'une base azotée, d'un produit de formule (III'$_3$) :

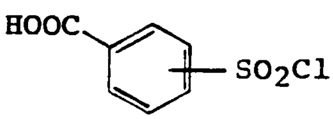

(III'$_3$)

pour obtenir, si nécessaire et si désiré, salification par une solution de bicarbonate de sodium, un produit de formule (I'$_{A2}$) :

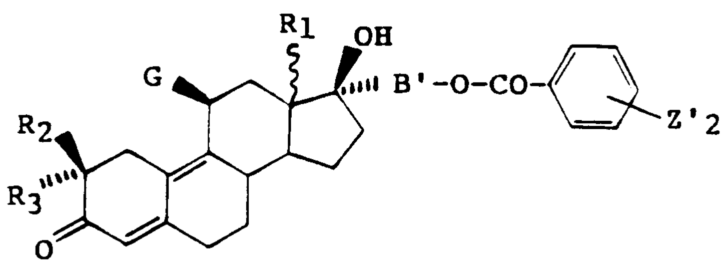

(I'$_{A2}$)

dans laquelle $Z'_2$ représente un groupe sulfo ou son sel de sodium,

produits de formule (I'$_{B1}$) que l'on soumet, si désiré, à l'action d'hydrogène en présence de catalyseur pour obtenir les produits de formule (I'$_{B3}$) :

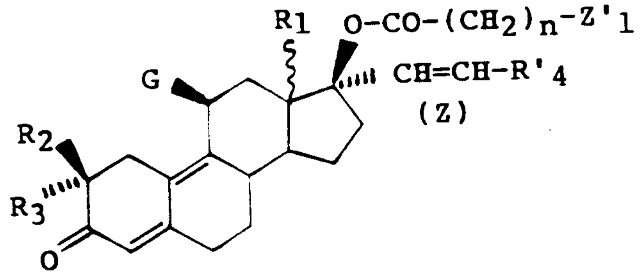

$(I'_{B3})$

Les produits de formules $(II_A)$ et $(II_B)$ utilisés pour la mise en oeuvre du procédé de préparations des produits de formule (I) sont connus de manière générale et leurs préparations sont décrites dans les brevets français n° 2377418, 2497807, 2522328, 2528434 et les brevets européens n° 057115 et 190759.

Les produits de formules $(III_1)$, $(III_2)$ et $(III_3)$ sont pour certains des produits commercialisés ; les autres peuvent être préparés par des méthodes connues de l'homme de l'art :

- ETAIX, annales de Chimie (7) 9 p. 371
- LOVEN, J. Prakt. Chemie (2) 29 p. 376
- UHLENBROEK, Recueil des Travaux Chimiques des Pays-Bas (1957) 76 p. 129, 142.

Les produits de formule générale (I) possèdent d'intéressantes propriétés pharmacologiques.

L'étude des produits sur les récepteurs hormonaux a permis de mettre en évidence des activités progestomimétiques ou anti-progestomimétiques, androgènes ou anti-androgènes.

Les produits de la présente demande de brevet peuvent également présenter des propriétés antiglucocorticoïdes et/ou glucocorticoïdes, antiprolifératives, anti-estrogènes et/ou estrogènes.

Ces produits peuvent donc être utilisés comme médicaments pour lutter principalement contre les effets secondaires des glucocorticoïdes ; ils permettent de lutter également contre les troubles dus à une hypersécrétion de glucocorticoïdes et notamment contre le vieillissement en général et plus particulièrement contre l'hypertension, le glaucome, l'athérosclérose, l'ostéoporose, le diabète, l'obésité ainsi que la dépression de l'immunité et l'insomnie.

Les produits qui possèdent des propriétés antiprogestomimétiques peuvent être utilisés pour préparer des contraceptifs originaux ou comme agents d'interruption de grossesse.

Les produits objets de la présente demande de brevet peuvent aussi être utilisés comme inducteurs de règles chez la femme et plus généralement chez les animaux femelles à sang chaud.

Ces produits sont alors administrés pendant les périodes où la progestérone joue un rôle physiologique essentiel, c'est-à-dire notamment pendant la phase lutéale du cycle, au moment de la nidation (ou implantation de l'embryon) et pendant la grossesse. Une méthode de contraception selon l'invention consiste à administrer à la femme un au moins des produits de formule (I) pendant 1 à 5 jours se situant préférentiellement à la fin du cycle. Ces produits sont administrés alors préférentiellement par la voie orale ou in vagino mais ils peuvent également être utilisés par la voie parentérale.

Les produits objets de la présente demande de brevet peuvent également être utilisés par la voie endonasale.

Ces produits possèdant des propriétés antiprogestomimétiques peuvent également être utilisés contre les dérèglements hormonaux et, par ailleurs, ils peuvent présenter un intérêt dans le traitement des tumeurs hormonodépendantes.

Leurs actions sur les sécrétions hypophysaires rendent les produits utilisables dans la ménopause.

Ces produits peuvent également être utilisés dans la synchronisation de l'estrus chez les animaux d'élevage, en particulier les bovins et les ovins.

Ces produits peuvent également être utilisés pour contrôler la fertilité des animaux familiers tels que chiens ou chats.

Les produits objets de la présente demande de brevet peuvent également présenter des propriétés progestomimétiques et peuvent ainsi être utilisés dans le traitement des aménorrhées, des dysménorrhées et des insuffisances lutéales.

Ces produits qui présentent des propriétés anti-androgènes peuvent être utilisés dans le traitement des hypertrophies et du cancer de la prostate, de l'hyperandrogénie, de l'anémie, de l'hirsutisme et de l'acné. Ils peuvent également être utilisés pour la contraception chez l'homme.

Enfin les produits objets de la présente demande de brevet qui présentent des propriétés antiprolifératives, anti-estrogènes et/ou estrogènes peuvent être utilisables dans le traitement des carcinomes hormono-dépendants, comme par exemple les carcinomes mammaires et leurs métastases. Ces propriétés

rendent les produits également utilisables dans le traitement des tumeurs bénignes du sein.

Les propriétés estrogènes que peuvent également présenter lesdits produits les rendent utilisables également dans le traitement des troubles liés à une hypofolliculinie, par exemple les aménorrhées, les dysménorrhées, les avortements répétés, les troubles prémenstruels ainsi que le traitement de la ménopause.

L'invention a donc pour objet, à titre de médicaments, les produits de formule (I) objets de la présente demande de brevet et plus particulièrement ceux de formule (I').

L'invention a tout particulièrement pour objet, à titre de médicaments, le :

- Succinate de sodium et de 21-chloro 11béta-[4-(diméthylamino) phényl] 3-oxo 19-nor 17alpha-pregna-4,9-dièn-20-yn-17béta-yle.
- Succinate de sodium et de 11béta-[4-(méthylthio) phényl] 3-oxo 17alpha-(1-propynyl) estra 4,9-dièn-17béta-yle.

La posologie utile varie en fonction de l'affection à traiter et de la voie d'administration ; elle peut varier par exemple de 10 mg à 1 g par jour chez l'adulte par voie orale.

Les nouveaux produits objets de la présente demande de brevet, tels que définis précédemment peuvent être employés pour préparer des compositions pharmaceutiques renfermant, à titre de principe actif, l'un au moins desdits produits.

Ces produits sont utilisés par voie digestive, parentérale ou locale. Ils peuvent être prescrits sous forme de comprimés simples ou dragéifiés, de gélules, de granulés, de suppositoires, d'ovules, de préparation injectables, de pommades, de crèmes, de gels, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, les dispersants ou émulsifiants, les conservateurs.

De plus, les produits de formule générale (I) dans laquelle Z représente un groupe carboxy salifié ou sulfo salifié et tout particulièrement les produits de formule (I') dans laquelle Z' représente -COONa ou $-SO_3Na$, sont très solubles dans l'eau ; parmi ceux-ci on peut citer de manière non limitative la solubilité du produit de l'exemple 18 qui est supérieure à 25 g pour 100 ml d'eau ; ceci permet leur utilisation sous forme de solutés buvables, nasaux ou auriculaires, de collyres, d'aérosols, de solutions injectables IM ou IV ou de capsules.

L'invention a donc pour objet les compositions pharmaceutiques renfermant comme principe actif au moins un produit objet de la présente demande de brevet.

L'invention a également pour objet à titre de produits industriels nouveaux les produits de formules ($IV_C$), ($IV_D$), ($IV_E$), ($IV_F$), ($V_D$) et ($VI_D$), telles que définies précédemment.

Les exemples suivants et l'étude pharmacologique illustrent l'invention sans toutefois la limiter.

**EXEMPLE 1 : Succinate acide de (Z)-3-[11béta-[4-(diméthylamino) phényl] 17béta-hydroxy 3-oxo estra-4,9-dièn-17alpha-yl] 2-propényle**

Dans un ballon muni d'une agitation magnétique, on dissout 900 mg de 11béta-[4-(diméthylamino) phényl] 17béta-hydroxy 17alpha-[(Z)-3-hydroxy 1-propényl] estra-4,9-dièn-3-one dans 9 ml de chloroforme puis on ajoute 304 mg d'anhydride succinique et 1,45 ml de triéthylamine. Le mélange ainsi formé est agité pendant 15 heures à température ambiante puis évaporé à sec, les 1,443 g de résidu obtenus sont purifiés par chromatographie sur colonne de silice (éluant : (acétate d'éthyle 90-cyclohexane 10)-acide acétique 3 %). Après recristallisation dans le mélange méthanol-eau (60-40), on obtient 695 mg du produit souhaité sous forme de cristaux jaunes. PF -~ 145 ° C.

Chromatographie sur couche mince : Rf = 0,60.

(support : KC 18 Whatman [R] ; éluant : methanol-solution aqueuse d'acétate d'ammonium 0,05 molaire (80-20)).

**EXEMPLE 2 : Succinate de sodium et de (Z)-3-[11béta-[4-(diméthylamino) phényl] 17béta-hydroxy 3-oxo estra-4,9-dièn-17alpha-yl] 2-propényle**

Dans un ballon muni d'une agitation magnétique, on dissout 93 mg de bicarbonate de sodium dans 20 ml d'eau, une solution de 639 mg du produit obtenu à l'exemple 1 dans 20 ml d'ethanol est ensuite ajoutée goutte à goutte. On chasse l'éthanol par azéotropie et la solution aqueuse ainsi obtenue est filtrée sur membrane millipore [R] (0,45 micron) puis lyophilisée. On recueille 654 mg de produit désiré sous forme

d'une poudre de couleur crème $[alpha]_D$ = +101° ± 2° (c = 1 % dans l'eau). Chromatographie sur couche mince : Rf = 0,62. (support : KC 18 Whatman R ; éluant : méthanol-solution aqueuse d'acétate d'ammonium 0,05 molaire (80-20)).

**EXEMPLE 3 : Succinate acide de 3-[11béta-[4-(diméthylamino) phényl] 17béta-hydroxy 3-oxo estra-4,9-dièn-17alpha-yl] 2-propynyle**

Dans un ballon muni d'une agitation magnétique, on dissout 900 mg de 11béta-[4-(diméthylamino) phényl] 17béta-hydroxy 17alpha-(3-hydroxy 1-propynyl) estra-4,9-dièn-3-one dans 9 ml de chloroforme, 303 mg d'anhydride succinique et 1,4 ml de triéthylamine sont ensuite ajoutés et le mélange ainsi formé est agité pendant 17 heures à température ambiante. Après évaporation à sec, les 1,685 g de produit brut sont purifiés par chromatographie sur colonne Bondapack C18 R (en éluant avec le mélange méthanol-solution aqueuse d'acétate d'ammonium 0,05 molaire (60-40)). On obtient ainsi 1,104 g du produit recherché.
Rf = 0,63 (chromatographie sur couche mince, support : KC 18 Whatman R , éluant : méthanol-solution aqueuse d'acétate d'ammonium 0,05 molaire (80-20)).

**EXEMPLE 4 : Succinate de sodium et de 3-[11béta-[4-(diméthylamino) phényl] 17béta-hydroxy 3-oxo estra-4,9-dièn-17alpha-yl] 2-propynyle**

On opère de la même manière qu'à l'exemple 2 avec 141 mg de bicarbonate de sodium dans 29 ml d'eau et 964 mg du produit préparé à l'exemple 3 dans 29 ml d'éthanol, on obtient alors 935 mg du produit recherché. $[alpha]_D$ = +55° ± 1,5° (c = 1 % dans l'eau).
Rf = 0,63 (chromatographie sur couche mince, support : KC 18 Whatman R , éluant : méthanol-solution aqueuse d'acétate d'ammonium 0,05 molaire (80-20)).

**EXEMPLE 5 : Succinate acide de 11béta-[4-(diméthylamino) phényl] 3-oxo 17alpha-(1-propynyl) estra-4,9-dièn-17béta-yle**

Le milieu réactionnel est préparé en ajoutant 2,15 g d'anhydride succinique, 2,2 ml de triéthylamine et 215 mg de 4-(diméthylamino) pyridine à une solution de 2,15 g de 11béta-[4-(diméthylamino) phényl] 17béta-hydroxy 17alpha-(1-propynyl) estra-4,9-dièn-3-one, dans 22 ml de chloroforme, puis chauffé au reflux pendant 42 heures et 430 mg de 4-(diméthylamino) pyridine et 4,4 ml de triéthylamine sont rajoutés. Le reflux est poursuivi pendant 26 heures et la solution est alors versée sur un mélange eau-glace. Après décantation de la phase organique, celle-ci est lavée à l'eau puis séchée et le chloroforme est distillé pour conduire à un extrait sec de couleur brune. La phase aqueuse est acidifiée avec de l'acide chlorhydrique 0,5 N puis neutralisée par addition d'acétate de sodium. On extrait de nouveau à l'acétate d'éthyle et la nouvelle phase organique est lavée, à l'eau, séchée et après distillation du solvant conduit à un résidu que l'on réunit avec le précédent. Le produit est purifié sur colonne de silice en éluant avec un mélange éther-acétate d'éthyle (9-1) à 3 % d'acide acétique et recristallisé deux fois dans un mélange éther-chlorure de méthylène. On obtient 1,435 g du produit recherché. PF -~ 165°.
$[alpha]_D$ = +97° (c = 0,8 % dans $CHCl_3$).
Rf -~ 0,40 (chromatographie sur couche mince, support : $SiO_2$, éluant : éther 9-acétate d'éthyle 1-acide acétique 3 %).

**EXEMPLE 6 : Succinate de sodium et de 11béta-[4-(diméthylamino) phényl] 3-oxo 17alpha-(1-propynyl) estra-4,9-dièn-17béta-yle**

Dans un ballon muni d'une agitation magnétique on introduit 3 g du produit préparé à l'exemple 5 et 94 ml d'éthanol et verse ensuite une solution de 433 mg de bicarbonate de sodium dans 94 ml d'eau. Après 30 minutes d'agitation à température ambiante, l'éthanol est chassé par azéotropie et la solution restante est filtrée sur membrane millipore R (0,45 microns) et lyophilisée. On obtient 2,88 g du produit recherché.
$[alpha]_D$ = +48,5° ± 1,5° (c = 1 % dans l'eau).
Rf = 0,54 (chromatographie sur couche mince, support : KC 18 Whatman R , éluant : méthanol-solution aqueuse d'acétate d'ammonium 0,05 molaire (80-20)).

**EXEMPLE 7 : Succinate acide de 11béta-[4-(diméthylamino) phényl] 3-oxo 17alpha-[(Z)-1-propényl] estra-4,9-dièn-17béta-yle**

Dans un ballon muni d'une agitation magnétique, on met 2,462 g du produit préparé à l'exemple 5 et ajoute 150 ml d'acétate d'éthyle à 2 % de pyridine, 15 ml d'eau et 50 mg d'hydroxyde de Palladium à 10 % sur sulfate de baryum. Après hydrogénation sous agitation pendant 5 heures et demie, le milieu réactionnel est filtré, la pyridine est chassée et la solution restante évaporée à sec. Le résidu ainsi obtenu est purifié par deux chromatographies successives sur colonne Bondapack C 18 en éluant avec le mélange méthanol-solution aqueuse d'acétate d'ammomium 0,05 molaire (65-35), on obtient 576 mg du produit recherché Rf = 0,50 (chromatographie sur couche mince ; support : KC 18 Whatman $^R$ , éluant : méthanol-solution aqueuse d'acétate d'ammonium 0,05 molaire (80-20)).

**EXEMPLE 8 : Succinate de sodium et de 11béta-[4-(diméthylamino) phényl] 3-oxo 17alpha-[(Z)-1-propényl] estra-4,9-dièn-17béta-yle**

En opérant de la même manière qu'à l'exemple 2 avec une solution de 100 mg de bicarbonate de sodium dans 21 ml d'eau et 665 mg du produit préparé à l'exemple 7 dans 21 ml d'éthanol, on obtient 583 mg du produit recherché.
$[alpha]_D$ = +56,5° ± 1,5° (c = 1 % dans l'eau).
Rf = 0,50 (chromatographie sur couche mince, support KC 18 Whatman $^R$ , éluant : méthanol-solution aqueuse d'acétate d'ammonium 0,05 molaire (80-20)).

**EXEMPLE 9 : Succinate acide de 21-chloro 11béta-[4-(diméthylamino) phényl] 3-oxo 19-nor 17alpha-prégna-4,9-dièn-20-yn-17béta-yle**

Dans un ballon muni d'une agitation magnétique, on dissout 1,854 g de 21-chloro 11béta-[4-(diméthylamino) phényl] 17béta-hydroxy 17alpha-pregna-4,9-dièn-20-yn-3-one dans 18,5 ml de chloroforme puis ajoute 2,497 g d'anhydride succinique 7 ml de triéthylamine et 0,936 g de 4-(diméthylamino) pyridine. Le mélange ainsi formé est chauffé au reflux pendant 42 heures. On coule ensuite le milieu réactionnel dans 31 ml d'une solution d'acide chlorhydrique 2N puis ramène le pH à 6-7 par addition d'acétate de sodium. On sépare la phase chloroformique puis on extrait à nouveau deux fois par du chloroforme. Les extraits recueillis sont réunis, lavés à l'eau, séchés sur sulfate de sodium puis concentrés sous pression réduite, on obtient 3,85 g d'un résidu marron que l'on purifie par chromatographie sur colonne de Kieselgel en éluant d'abord à l'éther éthylique puis avec le mélange éther éthylique à 3 % d'acide acétique. On obtient 1,8 g de produit brut que l'on recristallise dans le mélange chlorure de méthylène-éther éthylique puis dans le mélange chlorure de méthylène-éther éthylique. On obtient 1,21 g de produit attendu, PF ~ 165°C.
$[alpha]_D$ = +63° ± 1,5° (c = 0,90 dans $CHCl_3$).
Chromatographie sur couche mince : Rf = 0,53.
(support : KC18 Whatman $^R$ ; éluant : méthanol-solution aqueuse d'acétate d'ammonium 0,05 molaire (80-20)).

**EXEMPLE 10 : Succinate de sodium et de 21-chloro 11béta-[4-(diméthylamino) phényl] 3-oxo 19-nor 17alpha-prégna-4,9-dièn-20-yn-17béta-yle**

Dans un ballon muni d'une agitation magnétique, on mélange 817 mg du produit préparé à l'exemple 9 et 25 ml d'éthanol ; une solution de 113 mg de bicarbonate de sodium dans 25 ml d'eau est alors versée goutte à goutte et le milieu réactionnel est agité pendant 30 minutes à température ambiante. L'éthanol est ensuite chassé par azéotropie, la solution restante est filtrée sur membrane millipore $^R$ (0,45 microns) puis lyophilisée. On obtient 813 mg de lyophilisat qui correspond au produit recherché.
$[alpha]_D$ = +16,5° ± 1° (c = 1 % dans $H_2O$).
Rf = 0,54 (chromatographie sur couche mince ; support : KC 18 Whatman $^R$ , éluant : méthanol-solution aqueuse d'acétate d'ammonium 0,05 molaire (80-20)).

**EXEMPLE 11 : Succinate acide de 11béta-[4-(méthylthio) phényl] 3-oxo 17alpha-(1-propynyl) estra-4,9-dièn-17béta-yle**

Dans un ballon muni d'une agitation magnétique et d'un réfrigérant, 1,5 g de 17béta-hydroxy 11béta-[4-(méthylthio) phényl] 17alpha-(1-propynyl) estra-4,9-dièn-3-one, 15,3 ml de chloroforme sont mélangés puis

1,86 g d'anhydride succinique, 6 ml de triéthylamine et 794 mg de 4-(diméthylamino) pyridine sont ajoutés et le tout est chauffé au reflux pendant 94 heures, versé dans de l'acide chlorhydrique 1N et extrait au chloroforme. La phase chloroformique est lavée à l'eau, séchée sur sulfate de sodium et le solvant est éliminé sous pression réduite à 40 °C. On obtient 2,26 g de produit brut que l'on chromatographie sur une colonne de silice Kieselgel 60H [R] (éluant : (chlorure de méthylène 97,5-méthanol 2,5)-acide acétique 1 %). Après recristallisation dans le mélange chlorure de méthylène-éther isopropylique, il se forme 826 mg de cristaux du produit recherché. PF = 158 °C.
Rf = 0,61 (chromatographie sur couche mince, support KC 18 Whatman [R] , éluant : melange éthanol-solution aqueuse d'acétate d'ammonium 0,05 molaire (70-30)).

**EXEMPLE 12 : Succinate de sodium et de 11béta-[4-(méthylthio) phényl] 3-oxo 17alpha-(1-propynyl) estra-4,9-dièn-17béta-yle**

En opérant de la même manière qu'à l'exemple 2 avec 108 mg de bicarbonate de sodium dans 21,5 ml d'eau et 719 mg du produit préparé à l'exemple 11 dans 21,5 ml d'éthanol, on obtient 720 mg d'un lyophilisat correspondant au produit recherché.
$[alpha]_D$ = +74,5 ° ± 1,5 ° (c = 1 % dans l'eau).
Rf = 0,61 (chromatographie sur couche mince, support KC 18 Whatman [R] , éluant : éthanol-solution aqueuse d'acétate et ammonium 0,05 molaire (70-30)).

**EXEMPLE 13 : Acide 3-[3-[11béta-[4-(diméthylamino) phényl] 17béta-hydroxy 3-oxo estra-4,9-dièn-17alpha-yl] 2-propynyloxycarbonyl] benzènesulfonique**

On dissout 1 g de 11béta-[4-(diméthylamino) phényl] 17béta-hydroxy 17alpha-(3-hydroxy 1-propynyl) estra-4,9-dièn-3-one dans 10 ml de chloroforme, ajoute 1,5 g du complexe pyridine-acide 3-(chlorosulfonyl) benzoïque puis 1,25 ml de triéthylamine, porte au reflux pendant 45 minutes, ajoute de nouveau 0,5 g du complexe précédent et poursuit le chauffage au reflux pendant 30 minutes. Après refroidissement et évaporation du solvant, le résidu est dissous dans l'eau, on distille alors la solution en présence du toluène. L'extrait sec obtenu est dissous dans le chloroforme et filtré sur une colonne de silice, on élue avec du chloroforme à 5 % d'éthanol puis avec du chloroforme à 10 % d'éthanol. On recueille une fraction acide de laquelle on isole 1,7 g de produit souhaité sous forme de résine jaune pâle.

**EXEMPLE 14 : 3-[3-[11béta-[4-(diméthylamino) phényl] 17béta-hydroxy 3-oxo estra-4,9-dièn-17alpha-yl] 2-propynyloxycarbonyl] benzènesulfonate de sodium**

25ml d'une solution éthanolique d'acétate de sodium (0,24 mmole/ml) sont ajoutés à la fraction acide obtenue à l'exemple précédent et la solution ainsi obtenue est évaporée à sec. L'extrait sec est repris dans du méthanol à 33 % d'eau et chromatographié sur une colonne LICHROSORB KC 18 [R] (éluants : méthanol-eau (3-6), méthanol-eau (1-1) puis methanol-eau (6-3)). Les éluants sont regroupés, refiltrés, évaporés à sec et la poudre obtenue est séchée pour donner 1,35 g du produit recherché.
$[alpha]_D$ = +107 ° (c = 0,2 % dans l'éthanol).

| Microanalyse | | | | | | | | |
|---|---|---|---|---|---|---|---|---|
| Calculé<br>trouvé | C% | 66,34<br>66,6 | H% | 5,87<br>6,30 | N% | 2,25<br>2,50 | S% | 4,92<br>4,7 |

**EXEMPLE 15 : Succinate acide de 11béta-(4-acétyl phényl) 3-oxo 17alpha-(1-propynyl) estra-4,9-dièn-17béta-yle.**

1,8 g de 11béta-(4-acétyl phényl) 17béta-hydroxy 17alpha-(1-propynyl) estra-4,9-dièn-3-one sont dissous dans 18 ml de chloroforme puis on ajoute 2,54 g d'anhydride succinique, 7 ml de triéthylamine et 0,95 g de 4-diméthylamino pyridine ; la solution est portée au reflux et laissée à cette température pendant 70 heures. Après refroidissement à température ambiante, le milieu réactionnel est coulé dans de l'acide chlorhydrique 2N, extrait au chloroforme, la phase organique lavée à l'eau, séchée sur sulfate de sodium et évaporée à sec sous vide ; le produit brut ainsi obtenu est chromatographié sur une colonne de silice Kieselgel 60 [R] en éluant d'abord avec de l'éther éthylique puis avec de l'éther éthylique à 3 % d'acide

acétique. On obtient 1,37 g de produit brut que l'on purifie par cristallisation dans un mélange chlorure de méthylène-éther éthylique puis recristallisation dans un mélange identique. 1,027 g du produit recherché sont alors recueillis. PF -~ 168 °C.
Rf = 0,32 (chromatographie sur couche mince ; support : $SiO_2F_{254}$ Merck 60 [R] ; éluant : mélange éther éthylique-acétate d'éthyle à 3 % d'acide acétique (90-10)).

**EXEMPLE 16 : Succinate de sodium et de 11béta-(4-acétyl phényl) 3-oxo 17alpha-(1-propynyl) estra-4,9-dièn-17béta-yle**

0,874 g du produit préparé à l'exemple 15 sont dissous dans 30 ml d'éthanol ; cette solution est ajoutée goutte à goutte à une solution de 0,132 g de bicarbonate de sodium dans 30 ml d'eau ; l'éthanol est ensuite chassé par azéotropie, la solution aqueuse obtenue filtrée sur membrane millipore [R] (0,45 microns) puis lyophilisée. On obtient ainsi 0,908 g du sel de sodium recherché.
$[alpha]_D$ = + 67 ° ± 1,5 ° (C = 1 % dans l'eau).
Rf = 0,73 (chromatographie sur couche mince ; support : KC18 Whatman [R] ; éluant : mélange méthanol-solution aqueuse d'acétate d'ammonium 0,05 molaire (80-20)).

**EXEMPLE 17 : Succinate acide de 11béta-[4-(N-méthyl isopropylamino) phényl] 3-oxo 17alpha-(1-propynyl) estra-4,9-dièn-17béta-yle**

Une solution contenant 2,159 g de 11béta-[4-(N-méthyl isopropylamino) phényl] 17béta-hydroxy 17alpha-(1-propynyl) estra-4,9-dièn-3-one, 22 ml de triéthylamine, 2,52 g d'anhydride succinique, 8,1 ml de triéthylamine et 1,07 g de 4-diméthylamino pyridine est portée au reflux et laissée à cette temperature pendant 24 heures. 0,339 g d'anhydride succinique sont rajoutés et le reflux continué pendant 74 heures. Après refroidissement le milieu réactionnel est coulé dans 36 ml d'acide chlorhydrique 2N et le pH ajusté à 6 par addition d'acétate de sodium ; après décantation de la phase organique, la phase aqueuse est réextraite au chloroforme. Les phases chloroformiques réunies sont lavées à l'eau, séchées sur sulfate de sodium et évaporées à sec sous vide. Le résidu obtenu est chromatographié sur une colonne de 300 g de silice Kieselgel 60 [R] en éluant d'abord avec de l'éther éthylique puis de l'éther éthylique à 3 % d'acide acétique. On obtient 1,493 g de produit brut que l'on recristallise dans le mélange chlorure de methylène-éther éthylique. 1,082 g du succinate acide recherché sont ainsi obtenus. PF -~ 155 °C.
Rf = 0,47 (chromatographie sur couche mince ; support : KC18 Whatman [R] ; éluant : mélange méthanol-solution aqueuse d'acétate d'ammonium 0,05 molaire (80-20)).

**EXEMPLE 18 : Succinate de sodium et de 11béta-[4-(N-méthyl isopropylamino) phényl] 3-oxo 17alpha-(1-propynyl) estra-4,9-dièn-17béta-yle**

On opère de la même manière qu'à l'exemple 16 avec 0,128 g de bicarbonate de sodium dans 30 ml d'eau et 0,933 g du produit préparé à l'exemple 17 dans 30 ml d'éthanol ; 0,915 g du sel de sodium recherché sont ainsi obtenus :
$[alpha]_D$ = + 40,5 ° ± 1,5 ° (C = 1 % dans l'eau).
Rf = 0,47 (chromatographie sur couche mince, support KC18 Whatman [R] ; éluant : mélange méthanol-solution aqueuse d'acétate d'ammonium 0,05 molaire (80-20)).

**EXEMPLE 19 : Compositions pharmaceutiques.**

On a préparé les comprimés répondant à la formule suivante :

| | |
|---|---|
| Produit de l'exemple 10 | 50 mg |
| Excipient (talc, amidon, stéarate de magnésium) q.s. pour un comprimé terminé à | 120 mg |

**EXEMPLE 20 :**

On a préparé le collyre répondant à la formule suivante :

| | |
|---|---|
| Produit de l'exemple 10<br>Excipient (eau distillée, chlorure de sodium, méthyl cellulose, borate de sodium) | 2 g<br>100 ml |

## ETUDE PHARMACOLOGIOUE

**1) Mesure de l'affinité relative de liaison pour les récepteurs des hormones stéroïdiennes :**

Récepteur glucocorticoïde du thymus de rat :

Des rats mâles de 160 à 200 g sont surrénalectomisés. 4 à 8 jours après cette ablation, les animaux sont sacrifiés, et les thymus sont prélevés et homogénéisés à 0°C à l'aide d'un Potter téflon-verre dans un tampon (TSD) Tris 10 mM, saccharose 0,25 M, dithiothreitol 2 mM, HCl pH 7,4 (1 g de tissu pour 10 ml de TSD). L'homogénat est ensuite ultracentrifugé (105 000 g x 90 mn) à 0°C. Des aliquotes du surnageant ainsi obtenu, "cytosol", sont incubées à 0°C pendant 4 heures et 24 heures avec une concentration constante (T= 2,5 nM) de dexaméthasone tritiée en présence de concentrations croissantes (0 - 2.500 nM) de dexaméthasone froide ou du produit froid à tester. La concentration de la dexaméthasone tritiée liée (B) est ensuite mesurée dans chaque incubat par la technique d'adsorption au charbon-dextran.

Récepteur progestérone de l'utérus de lapin :

Des lapines impubères d'environ 1 kg reçoivent une application cutanée de 25 ug d'estradiol. Cinq jours après ce traitement, les animaux sont sacrifiés, les utérus sont prélevés, pesés et homogénéisés à 0°C à l'aide d'un Potter teflon-verre dans un tampon (TS) Tris 10 mM, saccharose 0,25 M, HCl pH 7,4 (1 g de tissu pour 50 ml de TS). L'homogénat est ensuite ultra-centrifugé (105 000 g x 90 mn) à 0°C. Des aliquotes du surnageant ainsi obtenu, "cytosol" sont incubées à 0°C pendant 2 heures et 24 heures avec une concentration constante (T = 5 nm) de R 5020 tritié (17,21-diméthyl-19-nor-4,9-prègnadièn-3,20-dione), progestomimétique puissant très affine pour le récepteur de la progestérone, en présence de concentrations croissantes (0 - 2500 nM) de progestérone froide, ou du produit à tester.

La concentration de R 5020 tritié lié (B) est ensuite mesurée dans chaque incubat par la technique d'adsorption au charbon-dextran.

Récepteur androgène de la prostate de rat

Des rats mâles de 160 à 200 g sont castrés. 24 heures après la castration, les animaux sont sacrifiés, les prostates sont prélevées, pesées et homogénéisées à 0°C, à l'aide d'un Potter téflon-verre dans un tampon (TSDPM) Tris 10 mM, saccharose 0,25 M, phénylméthanesulfonylfluoride 0,1 nM, molybdate 20 mM, dithiothréitol 2 mM, HCl pH 7,4 (1 g de tissu pour 5 ml de TSDPM). L'homogénat est ensuite ultracentrifugé (105 000 g x 60 mn) à 0°C. Des aliquotes du surnageant ainsi obtenu, "cytosol", sont incubées à 0°C avec une concentration constante (0 - 1000 nM) de testostérone froide ou du produit à tester. Après 1/2 heure et 24 heures d'incubation, la concentration de testostérone tritiée liée (B) est mesurée dans chaque incubat par la technique d'absorption au charbon-dextran.

Calcul de l'affinité relative de liaison :

Le calcul de l'affinité relative de liaison (ARL) est identique pour tous les récepteurs.

On trace les 2 courbes suivantes : le pourcentage de l'hormone tritiée liée $\frac{B}{T}$ en fonction du logarithme de la concentration de l'hormone de référence froide et $\frac{B}{T}$ en fonction du logarithme de la concentration du produit froid testé.

On détermine la droite d'équation $I_{50} = (\frac{B}{T} \max + \frac{B}{T} \min)/2$.

$\frac{B}{T}$ max = Pourcentage de l'hormone tritiée liée pour une incubation de cette hormone tritiée à la concentration (T).

$\frac{B}{T}$ min = Pourcentage de l'hormone tritiée liée pour une incubation de cette hormone tritiée à la

concentration (T) en présence d'un grand excès d'hormone froide ($2500.10^{-9}$ M).

Les intersections de la droite $I_{50}$ et des courbes permettent d'évaluer les concentrations de l'hormone de référence froide (CH) et du produit froid testé (CX) qui inhibent de 50 % la liaison de l'hormone tritiée sur le récepteur.

L'affinité relative de liaison (ARL) du produit testé est déterminée par l'équation

$$ARL = 100 \frac{(CH)}{(CX)}$$

Les résultats obtenus sont les suivants :

| Exemples | Progestérone | | Glucocorticoïde | | Androgène | |
|---|---|---|---|---|---|---|
| | 2H | 24H | 4H | 24H | 0,5H | 5H |
| EX. 5 | 2 | 8 | 28 | 77 | 0,6 | 1 |
| EX. 12 | 1,2 | 3,6 | 9,5 | 24 | 0,8 | 0,3 |
| EX. 4 | 14 | 26 | 31,5 | 27 | 5,1 | 2,8 |
| EX. 2 | 21 | 88 | 62 | 48 | 12 | 39 |
| EX. 10 | 3 | 13 | 37 | 77 | 1,9 | 8,6 |
| EX. 6 | 2 | 6 | 26 | 54 | 2,2 | 4,1 |

Les produits des exemples 12 et 10 présentent une bonne activité anti-progestérone.

**2) Activité anti-glucocorticoïde vis-à-vis de la dexaméthasone**

Etude in vitro

Incorporation d'uridine dans les thymocytes de rat

Les glucocorticoïdes provoquent au niveau du tissu lymphoïde une inhibition de l'incorporation des nucléosides. La mesure de l'incorporation d'uridine radioactive dans les thymocytes en présence d'un produit à tester permet d'évaluer son activité glucocorticoïde.

Méthode

Elle s'inspire de la technique décrite par Dausse et Coll (3). Le thymus d'un rat surrénalectomisé (160 à 180 g) est prélevé, émincé et homogénéisé lentement à l'aide d'un homogénéiseur téflon-verre dans une solution de Hanks. La suspension cellulaire obtenue est filtrée sur gaze, puis centrifugée à 800 g x 10 mn. On procède ensuite à une nouvelle centrifugation à 800 g x 10 mn. Le culot ainsi obtenu est remis en suspension dans un milieu nutritif (M.E.M. Gibco) et la concentration cellulaire est ajustée à environ $20.10^{6}$ cellules par ml. Des aliquotes de 250 ul sont alors incubées sous carbogène pendant 3 heures à 37 °C avec $5.10^{-8}$ M de dexaméthasone en présence ou non de concentrations croissantes de produit ($10^{-8}$ M à $10^{-6}$ M). On ajoute ensuite dans chaque incubat 0,1 uCi d'uridine tritiée et on continue l'incubation pendant 1 heure. Les incubats sont ensuite refroidis et additionnés de 1 ml d'une solution froide d'acide trichloracétique (TCA) à 5 % poids/volume. Les précipités sont recueillis sur des filtres Whatman GF/C et sont lavés par 4 x 2 ml de TCA à 5 % glacé. La radioactivité retenue sur les filtres (représentant l'uridine tritiée incorporée dans les thymocytes) est mesurée à l'aide d'un spectomètre à scintillation liquide.

**Thymocytes**

**% de l'inhibition de l'incorporation d'uridine**

**Concentration molaire du produit à tester**

| Produit de l'exemple | $10^{-6}M$ | $10^{-7}M$ | $10^{-8}M$ |
|---|---|---|---|
| 5 | 135 | 100 | 28 |
| 12 | 104 | 62 | 8 |
| 4 | 22 | 2,5 | 0 |
| 2 | 68 | 1 | 0 |
| 10 | 78 | 19 | 0 |
| 6 | 78 | 27 | 0 |

Les produits des exemples 5 et 12 présentent une bonne activité anti-glucocorticoïde.

## Revendications

**Revendications pour les Etats contractants suivants : AT, BE, CH, DE, DK, FR, GB, IT, LI, LU, NL, SE**

**1.** Les produits de formule générale (I) :

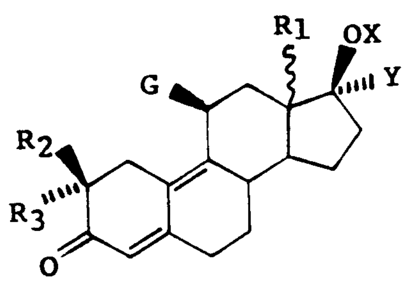

(I)

dans laquelle $R_1$ représente un radical hydrocarboné aliphatique renfermant de un à huit atomes de carbone, $R_2$ et $R_3$ identiques ou différents représentent un atome d'hydrogène ou un radical alkyle renfermant de un à quatre atomes de carbone, G représente un groupe hydrocarboné renfermant de un à dix-huit atomes de carbone et éventuellement un ou plusieurs hétéroatomes identiques ou différents, lié au noyau stéroïde par un atome de carbone et
ou bien X représente $X_A$, $X_A$ étant un atome d'hydrogène, un radical alkyle renfermant de un à huit atomes de carbone, un radical arylalkyle renfermant de sept à quinze atomes de carbone ou un radical acyle renfermant de un à huit atomes de carbone et dans ce cas Y représente $Y_A$, $Y_A$ étant un groupe

-B-O-CO-A-Z

dans lequel B représente un radical aliphatique bivalent, linéaire ou ramifié, saturé ou insaturé, renfermant de un à huit atomes de carbone, A représente ou bien un radical aliphatique bivalent, linéaire ou ramifié, saturé ou insaturé, renfermant de un à six atomes de carbone et eventuellement interrompu ou terminé par un radical aromatique bivalent, ou bien A représente un radical aromatique bivalent, et Z représente une des fonctions -COOH ou $-SO_3H$, ces fonctions pouvant être éventuellement salifiées sous forme d'un sel de métal alcalin ou alcalino-terreux, d'un sel d'ammonium ou d'un sel d'amine,

ou bien X représente $X_B$, $X_B$ étant un groupe

-CO-A-Z

dans lequel A et Z sont tels que définis précédemment et Y représente alors $Y_B$, $Y_B$ étant un des groupes choisis parmi $-C{\equiv}C-R_4$, $-CH=CH-R_4$ ou $-CH_2-CH_2-R_4$ dans lesquels $R_4$ représente un atome d'hydrogène, un atome d'halogène, un radical trialkylsilyle renfermant de trois à douze atomes de carbone, un radical alkyle renfermant de un à six atomes de carbone, linéaire ou ramifié ou un radical phényle, lesdits radicaux alkyle et phényle étant éventuellement substitués, le trait ondulé en position 13 signifie que le radical $R_1$ peut se trouver en position alpha ou béta.

**2.** Les produits de formule générale (I) telle que définie à la revendication 1, répondant à la formule (I') :

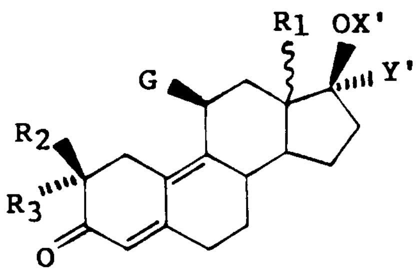

(I')

dans laquelle
ou bien X' représente un atome d'hydrogène et dans ce cas Y' représente $Y'_A$, $Y'_A$ étant un groupe :

-B'-O-CO-A'-Z'

dans lequel B' représente un des radicaux bivalents $-CH=CH-CH_2-$ ou $-C{\equiv}C-CH_2-$, A' représente un des radicaux bivalents $-(CH_2)_n-$ dans lequel n peut prendre une des valeurs de deux à six ou un radical ortho-, méta- ou para-phénylène et Z' représente les fonctions carboxy ou sulfo ou leur sel de sodium, ou bien X' représente $X'_B$, $X'_B$ étant un groupe :

$-CO-(CH_2)_n-Z'$

dans lequel n et Z' sont tels que définis précédemment et Y' représente alors $Y'_B$, $Y'_B$ étant un des groupes $-C{\equiv}C-R'_4$ ou $-CH=CH-R'_4$ lesquels $R'_4$ représente un atome d'hydrogène, un atome d'halogène, un radical triméthylsilyle, ou un radical méthyle éventuellement substitué par un ou plusieurs atomes d'halogène, un radical hydroxy ou par un groupe alklyloxy, alkylthio ou alkylamino renfermant de un à quatre atomes de carbone, dialkylamino renfermant de deux à huit atomes de carbone ou trialkylsilyle renfermant de trois à douze atomes de carbone.

**3.** Les produits de formule générale (I) telle que définie aux revendications 1 et 2 dans laquelle G représente un radical aryle substitué par un atome d'halogène, un radical aryle, un radical acyle renfermant de un à huit atomes de carbone ou par une des fonctions $-NH_2$, -NHR' ou -NR'R", dans lesquelles R' et R" identiques ou différents représentent un radical phényle, ou un radical alkyle primaire, secondaire ou tertiaire renfermant de 1 à 8 atomes de carbone, comportant éventuellement un ou plusieurs hétéroatomes, choisis parmi les atomes d'oxygène, d'azote et de soufre, ou éventuellement substitué par un hétérocycle, ou R' et R" représentent avec l'atome d'azote auquel ils sont liés, un radical hétérocyclique éventuellement substitué et comportant éventuellement un autre hétéroatome choisi parmi les atomes d'azote, d'oxygène et de soufre, ou bien par une des fonctions -0R‴ ou -SR‴ dans lesquelles R‴ représente un radical phényle ou un radical alkyle primaire, secondaire ou tertiaire renfermant de 1 à 8 atomes de carbone, comportant éventuellement un ou plusieurs hétéroatomes, choisis parmi les atomes d'oxygène, d'azote, de soufre ou éventuellement substitué par un hétérocycle, ou G représente un radical 2, 3 ou 4-pyridyle.

**4.** Les produits de formule générale (I) telle que définie à la revendication 3, dans laquelle G représente un radical phényle substitué en position 4 par un radical amino, (méthylamino), (diméthylamino) ou son N-oxyde, (diéthylamino), (dipropylamino), (N-méthyl éthylamino), (N-méthyl isopropylamino), (N-méthyl isobutylamino), (N-méthyl isopentylamino), 1-pyrrolidinyle, [2-(diméthylamino) N-méthyl éthylamino], [N-méthyl 2-(1-pyrrolidinyl) éthylamino], [N-méthyl 2-(4-morpholinyl) éthylamino], (4-méthyl 1-pipérazinyle), formyle, acétyle, méthoxy, phénoxy, [2-(diméthylamino) éthoxy], [2-(1-pyrrolidinyl) éthoxy], [2-(4-morpholinyl) éthoxy], (méthylthio), (éthylthio), (isopentylthio), [2-(diméthylamino) éthylthio], [2-(1-pyrrolidinyl) éthylthio] [2-(4-morpholinyl) éthylthio], (triméthylsilyle), méthyle, isopropyle, [(diméthylamino) méthyle], ou par un des atomes de fluor, de chlore ou de brome.

**5.** Les produits de formule générale (I) telle que définie l'une des revendications 1 à 4 dans laquelle $R_1$ représente un radical méthyle en position 13béta et $R_2$ et $R_3$ chacun un atome d'hydrogène.

**6.** Les produits de formule générale (I) telle que définie à la revendication 5 dont les noms suivent :
- succinate de sodium et de 21-chloro 11béta-[4-(diméthylamino) phényl] 3-oxo 19-nor 17alpha-pregna-4,9-dièn-20-yn-17béta-yle,
- succinate de sodium et de 11béta-[4-(méthylthio) phényl] 3-oxo 17alpha-(1-propynyl) estra-4,9-dièn-17béta-yle.

**7.** Procédé de préparation des produits de formule générale (I) telle que définie à la revendication 1 caractérisé en ce que

A - les produits de formule ($II_A$) ou de formule ($II_B$) :

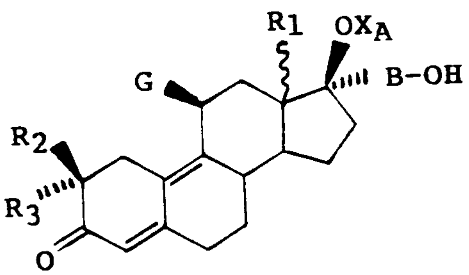

($II_A$)

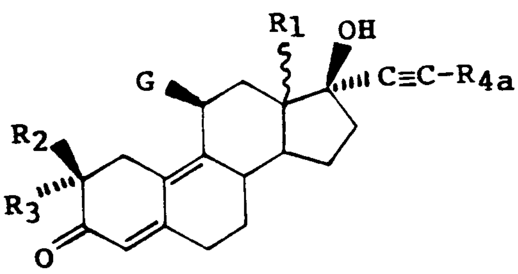

($II_B$)

dans lesquelles $R_1$, $R_2$, $R_3$ et G ont la signification indiquée à la revendication 1 et $R_{4a}$ a les valeurs indiquées à la revendication 1 pour $R_4$ ainsi que ces valeurs dans lesquelles les fonctions réactives sont protégées, sont soumis, dans un solvant neutre et en présence d'une base,

a) à l'action d'un produit de formule ($III_1$) :

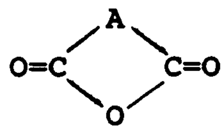

($III_1$)

pour obtenir après si nécessaire déprotection des fonctions réactives protégées et si désiré salification de la fonction carboxy, les produits de formule générale (I) répondant respectivement

aux formule ($I_{A1}$) et ($I_{B1}$) :

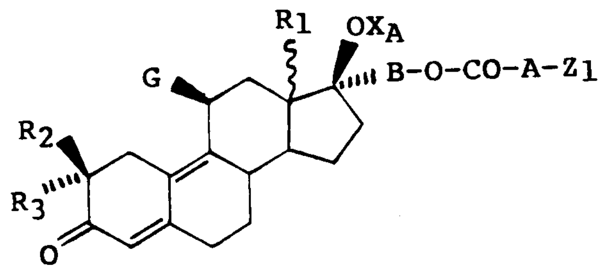

($I_{A1}$)

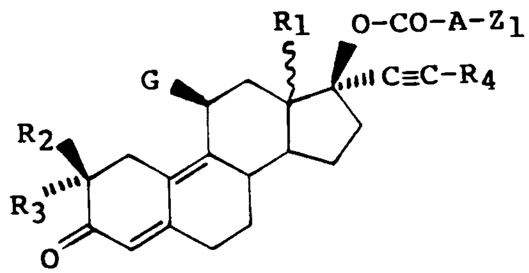

($I_{B1}$)

dans lesquelles $Z_1$ représente un radical carboxy éventuellement salifié,
b) à l'action d'un produit de formule ($III_2$) :

HOOC-A-U ($III_2$)

ou encore d'un dérivé fonctionnel de cet acide dans laquelle U représente un des groupes -COOH, -$COOR_5$ dans lequel $R_5$ représente un radical alkyle renfermant de un à six atomes de carbone ou un radical arylalkyle renfermant de sept à douze atomes de carbone, ou -SH, pour obtenir, après si nécessaire déprotection des fonctions réactives protégées, respectivement les produits de formules ($IV_A$) et ($IV_B$) :

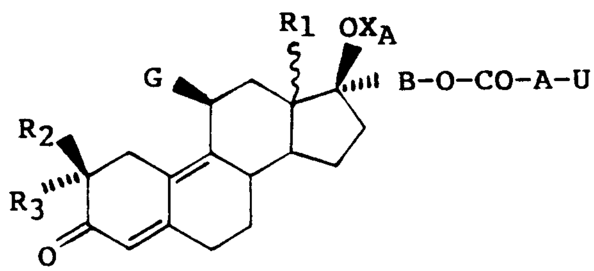

($IV_A$)

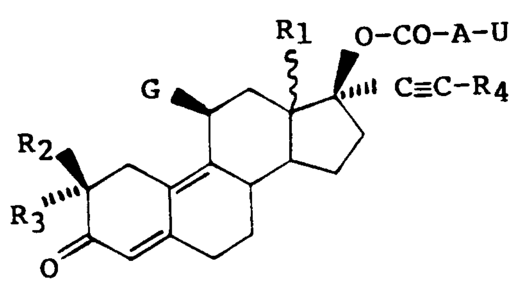

($IV_B$)

produits de formules ($IV_A$) et ($IV_B$) qui :

- dans le cas où U représente le groupe carboxy libre, correspondent après salification éventuelle, aux produits de formules ($I_{A1}$) et ($I_{B1}$),

- dans le cas où U représente le groupe $-COOR_5$, correspondent aux produits de formules ($IV_C$) et ($IV_D$) :

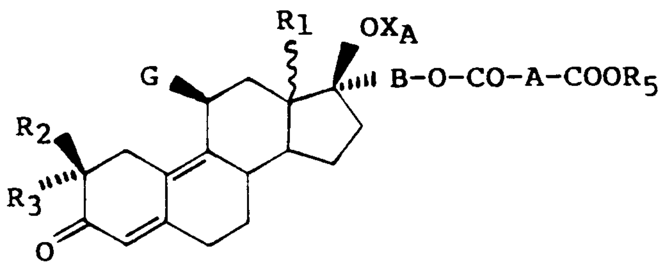

($IV_C$)

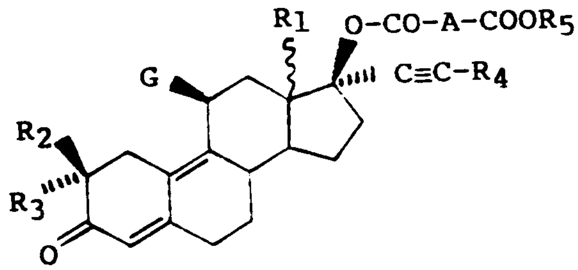

($IV_D$)

produits de formules ($IV_C$) et ($IV_D$) qui sont hydrolysés ou saponifiés pour obtenir respectivement les produits de formules ($I_{A1}$) et ($I_{B1}$),

- dans le cas où U représente le groupe -SH, correspondent aux produits de formules ($IV_E$) et ($IV_F$) :

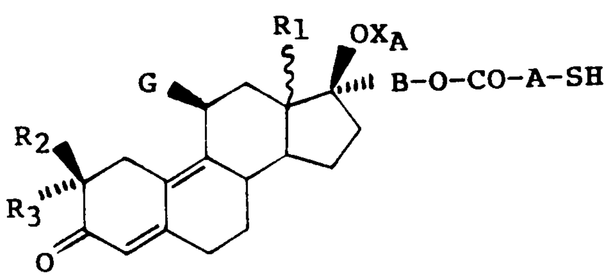

($IV_E$)

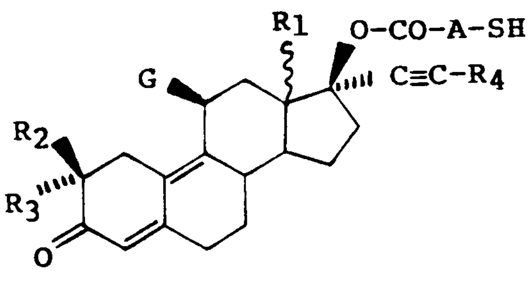

($IV_F$)

produits de formules ($IV_E$) et ($IV_F$) qui sont oxydés et si désiré salifiés pour obtenir les produits de formule générale (I) répondant respectivement aux formules ($I_{A2}$) et ($I_{B2}$) :

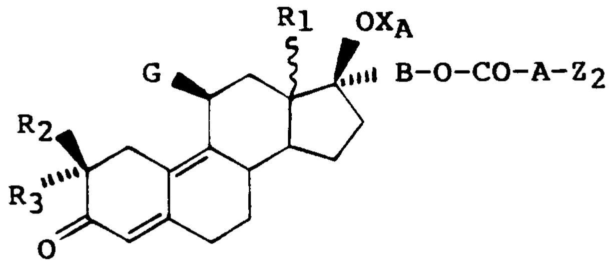

$(I_{A2})$

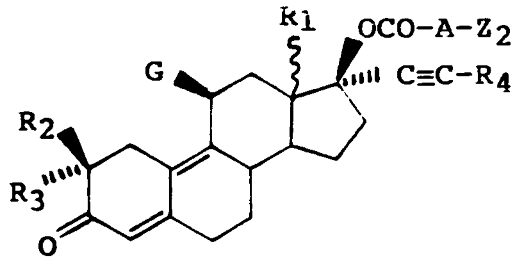

$(I_{B2})$

dans lesquelles $Z_2$ représente un groupe sulfo éventuellement salifié,

c) ou à l'action d'un produit de formule ($III_3$) :

$HOOC\text{-}A\text{-}SO_2Cl$ ($III_3$)

ou encore d'un dérivé fonctionnel de cet acide, pour obtenir si nécessaire après déprotection des fonctions réactives contenues dans $R_{4a}$ et si désiré salification, respectivement les produits de formule ($I_{A2}$) et ($I_{B2}$) ;

en ce que :

B - les produits de formule ($I_{B1}$), ($I_{B2}$) et ($IV'_D$) sont soumis si désiré :

a) soit à un agent d'hydrogénation des triples liaisons pour obtenir respectivement les produits de formules ($I_{B3}$), ($I_{B4}$) et ($V_D$) :

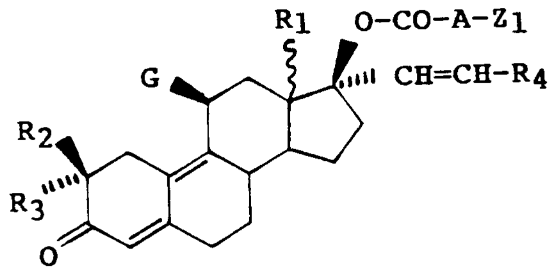

$(I_{B3})$

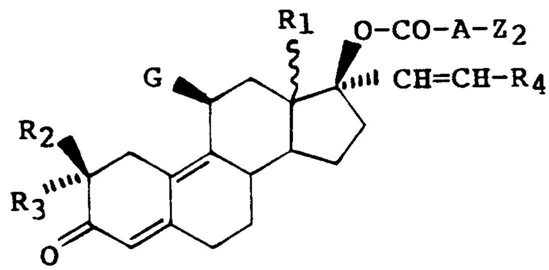

$(I_{B4})$

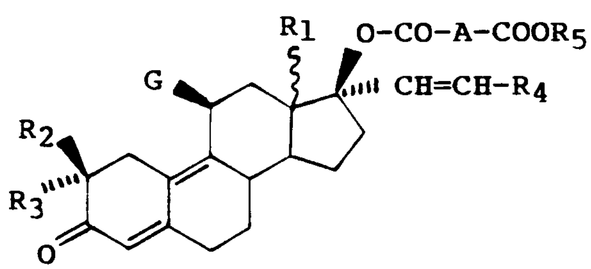

$(V_D)$

produits qui sont si désiré soumis à un agent d'hydrogénation des doubles liaisons pour obtenir respectivement des produits de formule $(I_{B5})$, $(I_{B6})$ et $(VI_D)$ :

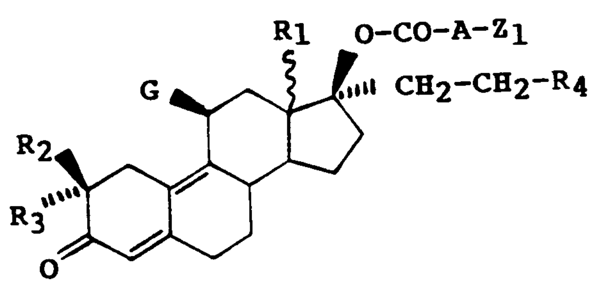

$(I_{B5})$

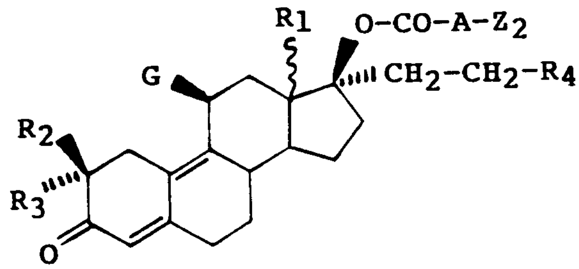

($I_{B6}$)

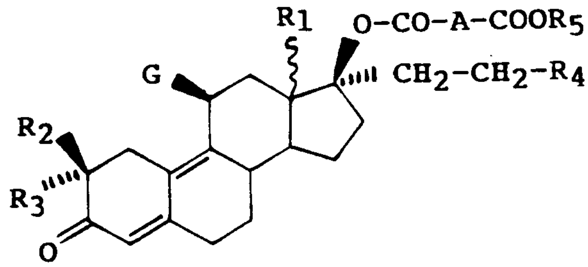

($VI_D$)

soit à un agent d'hydrogénation directe des triples liaisons en simples liaisons pour obtenir respectivement les produits de formules ($I_{B5}$), ($I_{B6}$) et ($VI_D$) ;

b) les produits de formules ($V_D$) et ($VI_D$) sont soit hydrolysés, soit saponifiés pour obtenir respectivement les produits de formules ($I_{B3}$) et ($I_{B5}$).

**8.** Procédé selon la revendication 7 de préparation des produits de formule (I') tels que définis à la revendication 2 caractérisé en ce que :

- a) soit l'on soumet le produit de formule ($II'_A$) ou ($II'_B$) :

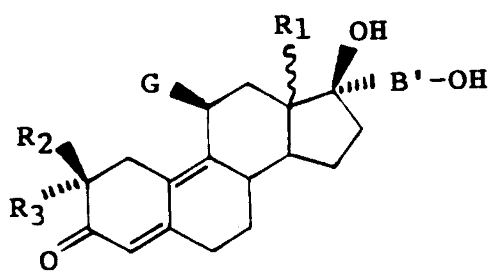

($II'_A$)

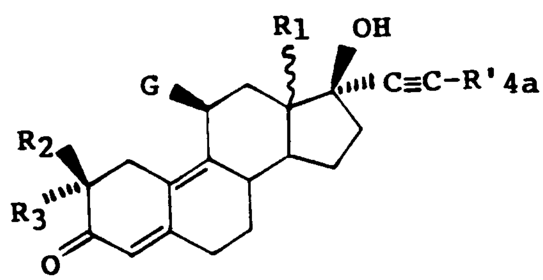

($II'_B$)

dans laquelle $R_1$, $R_2$, $R_3$ et G ont les valeurs indiquées à la revendication 2 et $R'_{4a}$ a les valeurs indiquées à la revendication 2 pour $R'_4$ ainsi que ces valeurs dans lesquelles les fonctions hydroxylées sont protégées, dans un solvant neutre et en présence d'une base à l'action d'un produit de formule ($III'_1$) :

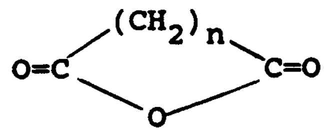

(III'1)

dans laquelle n prend une des valeurs de deux à six pour obtenir, après si nécessaire déprotection de la fonction hydroxy protégée contenue dans R'$_4$ et si désiré, salification de la fonction carboxy libre par action d'une solution de bicarbonate de sodium, respectivement le produit de formule (I'$_{A1}$) ou (I'$_{B1}$) :

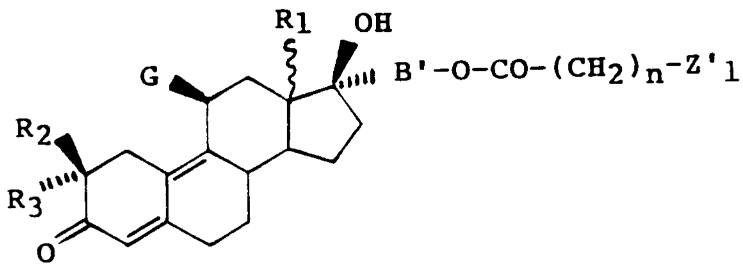

(I'A1)

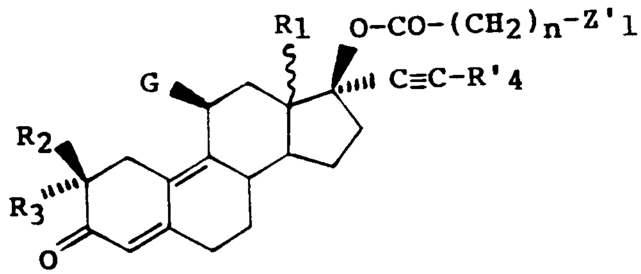

(I'B1)

dans laquelle Z'$_1$ représente un groupe carboxy libre ou son sel de sodium.

b) soit l'on soumet le produit de formule (II'$_A$) telle que définie ci-dessus à l'action en présence d'une base azotée d'un produit de formule (III'$_3$) :

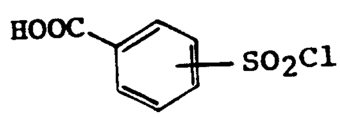

(III'3)

pour obtenir, si nécessaire et si désiré, salification par une solution de bicarbonate de sodium, un produit de formule (I'$_{A2}$) :

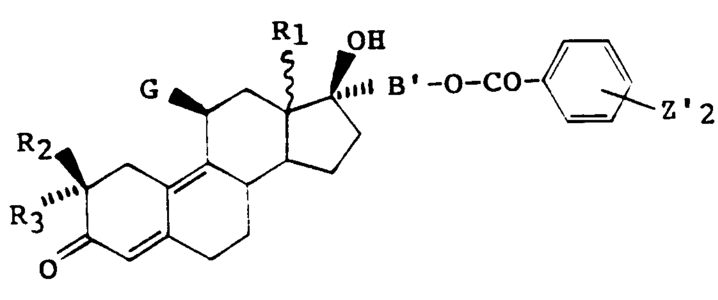

(I'A2)

dans lesquelles Z'$_2$ représente un groupe sulfo ou son sel de sodium ;

produit de formule (I'$_{B1}$) que l'on soumet, si désiré, à l'action d'hydrogène en présence de catalyseur

pour obtenir les produits de formule ($I'_{B3}$) :

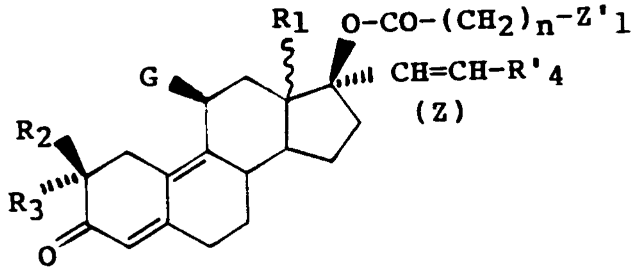

($I'_{B3}$)

**9.** A titre de médicaments, les produits de formule générale (I) telle que définie à la revendication 1.

**10.** A titre de médicaments, les produits de formule générale (I) telle que définie à l'une quelconque des revendications 2 à 6.

**11.** Compositions pharmaceutiques renfermant comme principe actif, l'un au moins des médicaments définis selon la revendication 9 ou 10.

**12.** A titre de produits industriels nouveaux, les produits de formules ($IV_C$), ($IV_D$), ($IV_E$), ($IV_F$), ($V_D$) et ($VI_D$).

**Revendications pour l'Etat contractant suivant : ES**

**1.** Procédé pour préparer les produits de formule générale (I) :

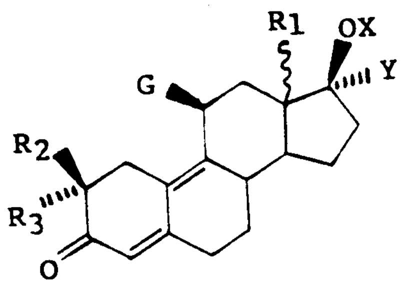

(I)

dans laquelle $R_1$ représente un radical hydrocarboné aliphatiue renfermant de un à huit atomes de carbone, $R_2$ et $R_3$ identiques ou différents représentent un atome d'hydrogène ou un radical alkyle renfermant de un à quatre atomes de carbone, G représente un groupe hydrocarboné renfermant de un à dix-huit atomes de carbone et éventuellement un ou plusieurs hétéroatomes identiques ou différents, lié au noyau stéroïde par un atome de carbone et

ou bien X représente $X_A$, $X_A$ étant un atome d'hydrogène, un radical alkyle renfermant de un à huit atomes de carbone, un radical arylalkyle renfermant de sept à quinze atomes de carbone ou un radical acyle renfermant de un à huit atomes de carbone et dans ce cas Y représente $Y_A$, $Y_A$ étant un groupe

-B-O-CO-A-Z

dans lequel B représente un radical aliphatique bivalent, linéaire ou ramifié, saturé ou insaturé, renfermant de un à huit atomes de carbone, A représente ou bien un radical aliphatique bivalent, linéaire ou ramifié, saturé ou insaturé, renfermant de un à six atomes de carbone et éventuellement interrompu ou terminé par un radical aromatique bivalent, ou bien A représente un radical aromatique bivalent, et Z représente une des fonctions -COOH ou $-SO_3H$, ces fonctions pouvant être éventuellement salifiées sous forme d'un sel de métal alcalin ou alcalino-terreux, d'un sel d'ammonium ou d'un sel d'amine,

ou bien X représente $X_B$, $X_B$ étant un groupe

-CO-A-Z

dans lequel A et Z sont tels que définis précédemment et Y représente alors $Y_B$, $Y_B$ étant un des groupes choisis parmi $-C{\equiv}C-R_4$, $-CH=CH-R_4$ ou $-CH_2-CH_2-R_4$ dans lesquels $R_4$ représente un atome d'hydrogène, un atome d'halogène, un radical trialkylsilyle renfermant de trois à douze atomes de carbone, un radical alkyle renfermant de un à six atomes de carbone, linéaire ou ramifié ou un radical phényle, lesdits radicaux alkyle et phényle étant éventuellement substitués, le trait ondulé en position 13 signifie que le radical $R_1$ peut se trouver en position alpha ou béta, caractérisé en ce que

A - les produits de formule ($II_A$) ou de formule ($II_B$) :

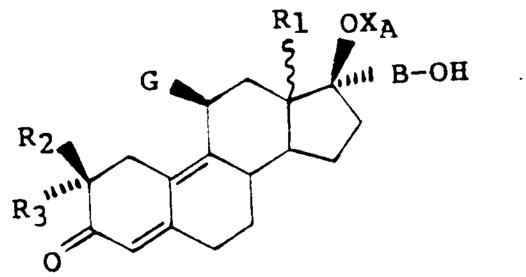

($II_A$)

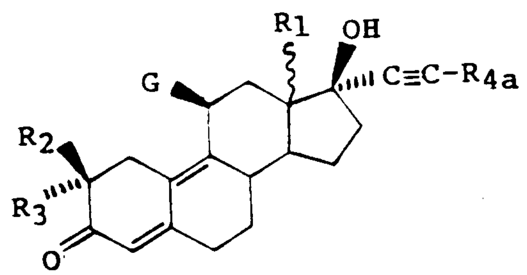

($II_B$)

dans lesquelles $R_1$, $R_2$, $R_3$ et G ont la signification indiquée à la revendication 1 et $R_{4a}$ a les valeurs indiquées à la revendication 1 pour $R_4$ ainsi que ces valeurs dans lesquelles les fonctions réactives sont protégées, sont soumis, dans un solvant neutre et en présence d'une base,

a) à l'action d'un produit de formule ($III_1$) :

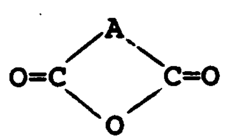

($III_1$)

pour obtenir après si nécessaire déprotection des fonctions réactives protégées et si désiré salification de la fonction carboxy, les produits de formule générale (I) répondant respectivement aux formule ($I_{A1}$) et ($I_{B1}$) :

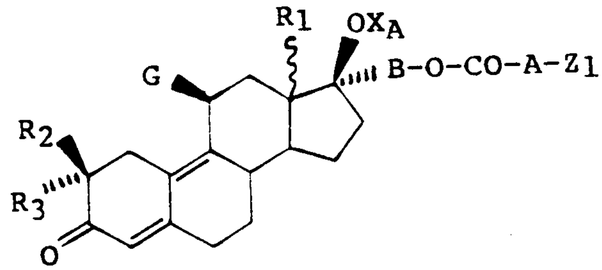

$(I_{A1})$

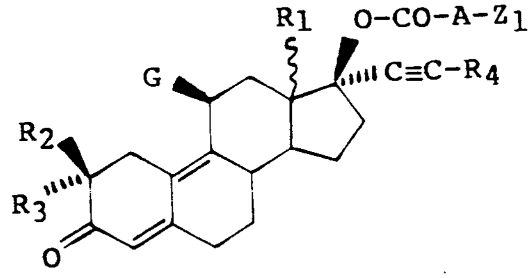

$(I_{B1})$

dans lesquelles $Z_1$ représente un radical carboxy éventuellement salifié,
b) à l'action d'un produit de formule $(III_2)$ :

HOOC-A-U $(III_2)$

ou encore d'un dérivé fonctionnel de cet acide dans laquelle U représente un des groupes -COOH, $-COOR_5$ dans lequel $R_5$ représente un radical alkyle renfermant de un à six atomes de carbone ou un radical arylalkyle renfermant de sept à douze atomes de carbone, ou -SH, pour obtenir, après si necessaire déprotection des fonctions réactives protégées, respectivement les produits de formules $(IV_A)$ et $(IV_B)$ :

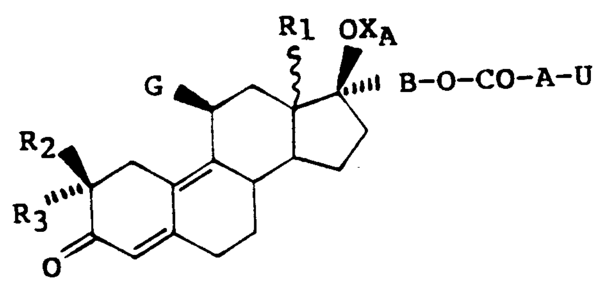

$(IV_A)$

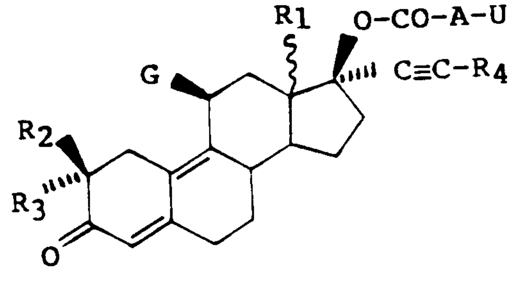

$(IV_B)$

produits de formules $(IV_A)$ et $(IV_B)$ qui :

- dans le cas où U représente le groupe carboxy libre, correspondent après salification éventuelle, aux produits de formules $(I_{A1})$ et $(I_{B1})$,

- dans le cas où U représente le groupe $-COOR_5$, correspondent aux produits de formules ($IV_C$) et ($IV_D$) :

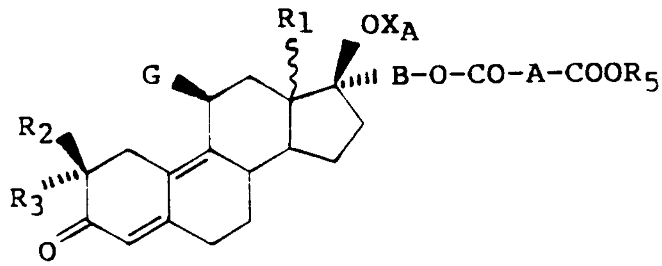

($IV_C$)

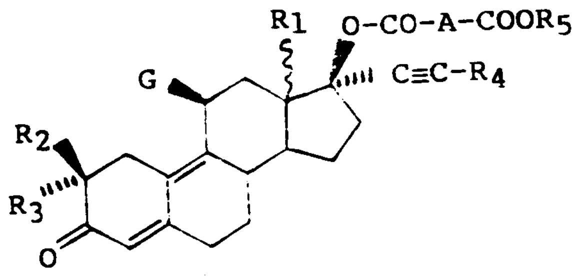

($IV_D$)

produits de formules ($IV_C$) et ($IV_D$) qui sont hydrolysés ou saponifiés pour obtenir respectivement les produits de formules ($I_{A1}$) et ($I_{B1}$),

- dans le cas où U représente le groupe -SH, correspondent aux produits de formules ($IV_E$) et ($IV_F$) :

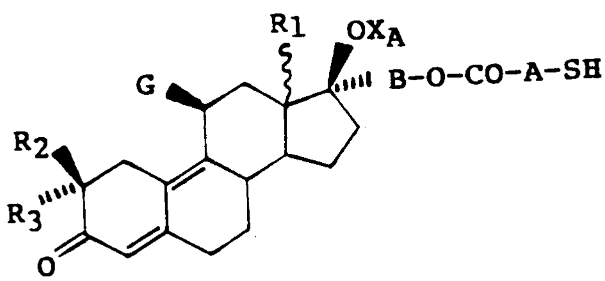

($IV_E$)

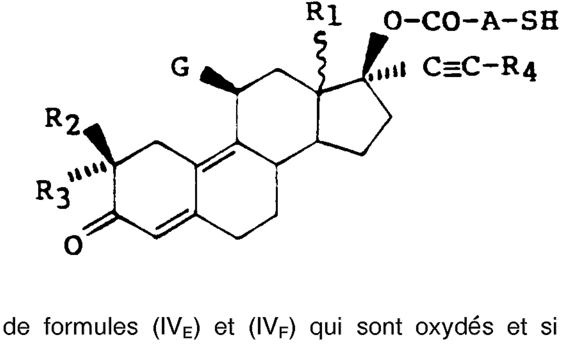

($IV_F$)

produits de formules ($IV_E$) et ($IV_F$) qui sont oxydés et si désiré salifiés pour obtenir les produits de formule générale (I) répondant respectivement aux formules ($I_{A2}$) et ($I_{B2}$) :

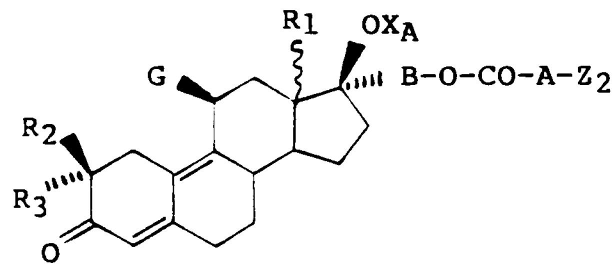

$(I_{A2})$

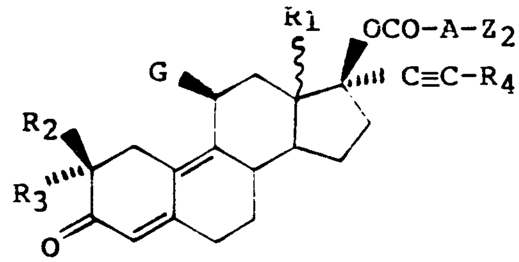

$(I_{B2})$

dans lesquelles $Z_2$ représente un groupe sulfo éventuellement salifié,

c) ou à l'action d'un produit de formule $(III_3)$ :

$HOOC\text{-}A\text{-}SO_2Cl$ $(III_3)$

ou encore d'un dérivé fonctionnel de cet acide, pour obtenir si nécessaire après déprotection des fonctions réactives contenues dans $R_{4a}$ et si désiré salification, respectivement les produits de formule $(I_{A2})$ et $(I_{B2})$ ;

en ce que :

B - les produits de formule $(I_{B1})$, $(I_{B2})$ et $(IV'_D)$ sont soumis si désiré :

a) soit à un agent d'hydrogénation des triples liaisons pour obtenir respectivement les produits de formules $(I_{B3})$, $(I_{B4})$ et $(V_D)$ :

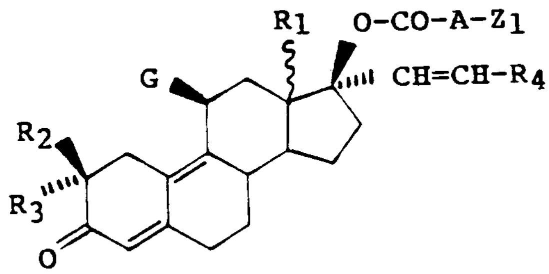

$(I_{B3})$

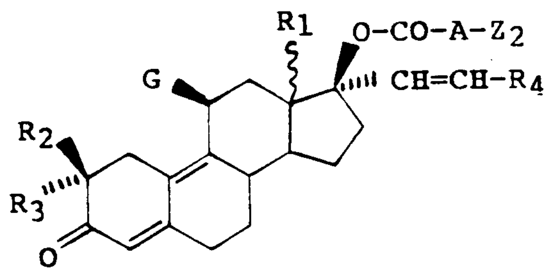

$(I_{B4})$

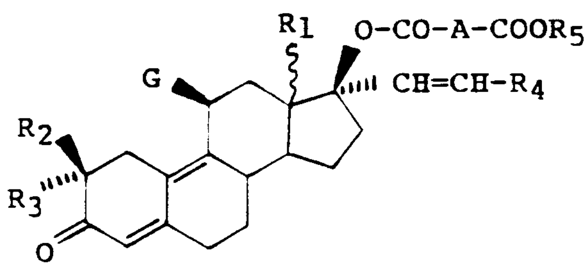

$(V_D)$

produits qui sont si désiré soumis à un agent d'hydrogénation des doubles liaisons pour obtenir respectivement des produits de formule $(I_{B5})$, $(I_{B6})$ et $(VI_D)$ :

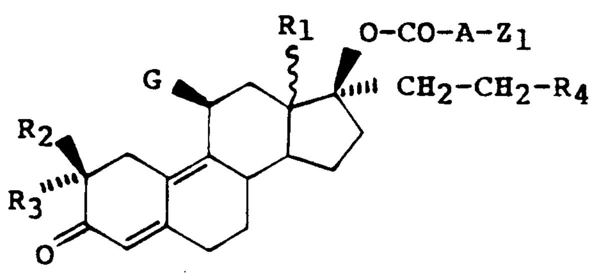

$(I_{B5})$

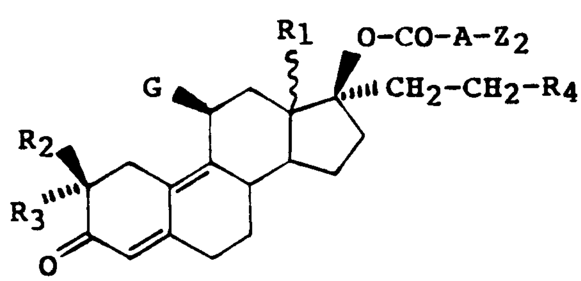

$(I_{B6})$

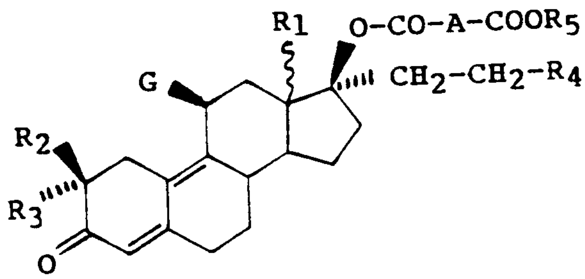

(VI$_D$)

soit à un agent d'hydrogénation directe des triples liaisons en simples liaisons pour obtenir respectivement les produits de formules ($I_{B5}$), ($I_{B6}$) et ($VI_D$) ;

b) les produits de formules ($V_D$) et ($VI_D$) sont soit hydrolysés, soit saponifiés pour obtenir respectivement les produits de formules ($I_{B3}$) et ($I_{B5}$).

**2.** Procédé pour préparer les produits de formule générale (I) telle que définie à la revendication 1, répondant à la formule (I') :

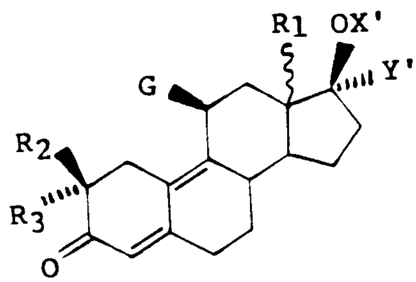

(I')

dans laquelle
ou bien X' représente un atome d'hydrogène et dans ce cas Y' représente $Y'_A$, $Y'_A$ étant un groupe :

-B'-O-CO-A'-Z'

dans lequel B' représente un des radicaux bivalents $-CH=CH-CH_2-$ ou $-C{\equiv}C-CH_2-$, A' représente un des radicaux bivalents $-(CH_2)_n-$ dans lequel n peut prendre une des valeurs de deux à six ou un radical ortho-, méta- ou para-phénylène et Z' représente les fonctions carboxy ou sulfo ou leur sel de sodium,
ou bien X' représente $X'_B$, $X'_B$ étant un groupe :

$-CO-(CH_2)_n-Z'$

dans lequel n et Z' sont tels que définis précédemment et Y' représente alors $Y'_B$, $Y'_B$ étant un des groupes $-C{\equiv}C-R'_4$ ou $-CH=CH-R'_4$ dans lesquels $R'_4$ représente un atome d'hydrogène, un atome d'halogène, un radical triméthylsilyle, ou un radical méthyle éventuellement substitué par un ou plusieurs atomes d'halogène, un radical hydroxy ou par un groupe alklyloxy, alkylthio ou alkylamino renfermant de un à quatre atomes de carbone, dialkylamino renfermant de deux à huit atomes de carbone ou trialkylsilyle renfermant de trois à douze atomes de carbone, caractérisé en ce que :

- a) soit l'on soumet le produit de formule ($II'_A$) ou ($II'_B$) :

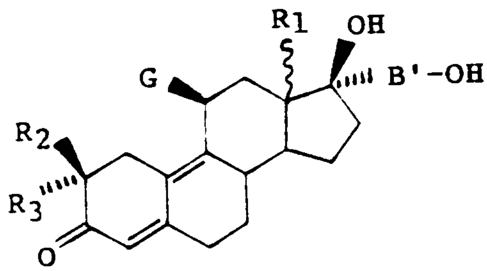

($II'_A$)

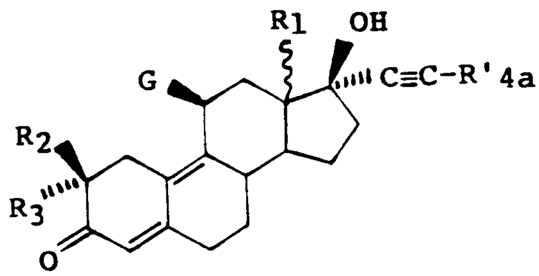

($II'_B$)

dans laquelle $R_1$, $R_2$, $R_3$ et G ont les valeurs indiquées à la revendication 2 et $R'_{4a}$ a les valeurs indiquées à la revendication 2 pour $R'_4$ ainsi que ces valeurs dans lesquelles les fonctions hydroxylées sont protégées, dans un solvant neutre et en présence d'une base à l'action d'un produit de formule ($III'_1$) :

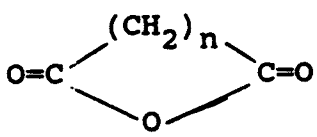

($III'_1$)

dans laquelle n prend une des valeurs de deux à six pour obtenir, après si necessaire déprotection de la fonction hydroxy protégée contenue dans $R'_4$ et si désiré, salification de la fonction carboxy libre par action d'une solution de bicarbonate de sodium, respectivement le produit de formule ($I'_{A1}$) ou ($I'_{B1}$) :

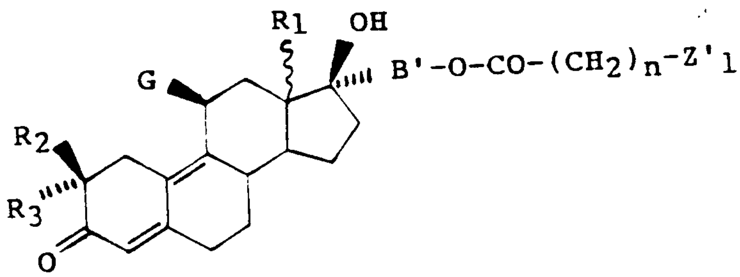

(I'A1)

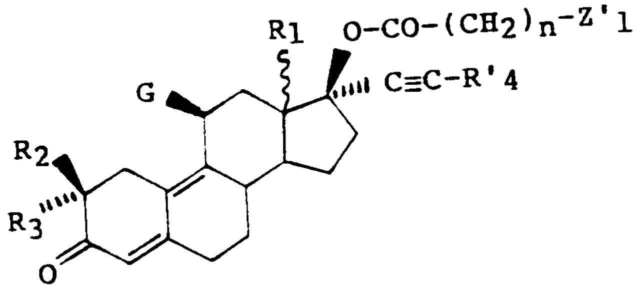

(I'B1)

dans laquelle $Z'_1$ représente un groupe carboxy libre ou son sel de sodium,

b) soit l'on soumet le produit de formule ($II'_A$) telle que définie ci-dessus à l'action en présence d'une base azotée d'un produit de formule ($III'_3$) :

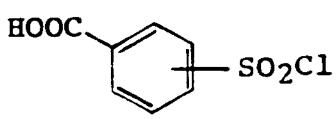

(III'3)

pour obtenir, si nécessaire et si désiré, salification par une solution de bicarbonate de sodium, un produit de formule ($I'_{A2}$) :

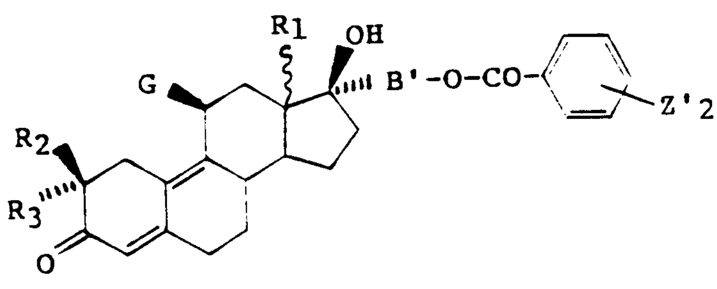

(I'A2)

dans lesquelles $Z'_2$ représente un groupe sulfo ou son sel de sodium ;

produit de formule ($I'_{B1}$) que l'on soumet, si désiré, à l'action d'hydrogène en présence de catalyseur pour obtenir les produits de formule ($I'_{B3}$) :

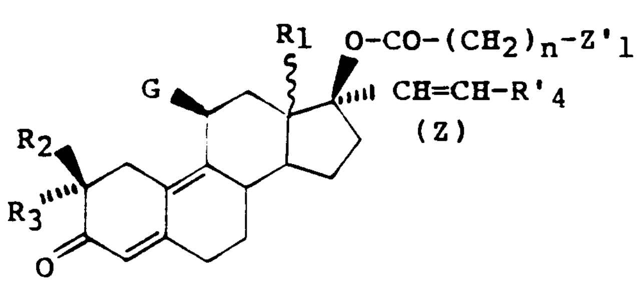

(I'B3)

3. Procédé selon la revendication 1 ou 2, caractérisé en ce que l'on utilise au départ un produit de formule ($II_A$) ou ($II_B$) ou ($II'_A$) ou ($II'_B$) dans laquelle G représente un radical aryle substitué par un atome d'halogène, un radical aryle, un radical acyle renfermant de un à huit atomes de carbone ou par une des fonctions $-NH_2$, -NHR′ ou -NR′R″, dans lesquelles R′ et R″ identiques ou différents représentent un radical phényle, ou un radical alkyle primaire, secondaire ou tertiaire renfermant de 1 à 8 atomes de carbone, comportant éventuellement un ou plusieurs hétéroatomes, choisis parmi les atomes d'oxygène, d'azote et de soufre, ou éventuellement substitué par un hétérocycle, ou R′ et R″ représentent avec l'atome d'azote auquel ils sont liés, un radical hétérocyclique éventuellement substitué et comportant éventuellement un autre hétéroatome choisi parmi les atomes d'azote, d'oxygène et de soufre, ou bien par une des fonctions -0R‴ ou -SR‴ dans lesquelles R‴ représente un radical phényle ou un radical alkyle primaire, secondaire ou tertiaire renfermant de 1 à 8 atomes de carbone, comportant éventuellement un ou plusieurs hétéroatomes, choisis parmi les atomes d'oxygène, d'azote, de soufre ou éventuellement substitué par un hétérocycle, ou G représente un radical 2, 3 ou 4-pyridyle.

4. Procédé selon la revendication 3, caractérisé en ce que l'on utilise au départ un produit de formule ($II_A$) ou ($II_B$) ou ($II'_A$) ou ($II'_B$) dans laquelle G représente un radical phényle substitué en position 4 par un radical amino, (méthylamino), (diméthylamino) ou son N-oxyde, (diéthylamino), (dipropylamino), (N-méthyl éthylamino), (N-méthyl isopropylamino), (N-méthyl isobutylamino), (N-méthyl isopentylamino), 1-pyrrolidinyle, [2-(diméthylamino) N-méthyl éthylamino], [N-méthyl 2-(1-pyrrolidinyl) éthylamino], [N-méthyl 2-(4-morpholinyl) éthylamino], (4-méthyl 1-pipérazinyle), formyle, acétyle, méthoxy, phénoxy, [2-(diméthylamino) éthoxy], [2-(1-pyrrolidinyl) éthoxy], [2-(4-morpholinyl) éthoxy], (méthylthio), (éthylthio), (isopentylthio), [2-(diméthylamino) éthylthio], [2-(1-pyrrolidinyl) éthylthio] [2-(4-morpholinyl) éthylthio], (triméthylsilyle), méthyle, isopropyle, [(diméthylamino) méthyle], ou par un des atomes de fluor, de chlore ou de brome.

5. Procédé selon l'une quelconque des revendications 1 à 4, caractérisé en ce que l'on utilise au départ un produit de formule ($II_A$) ou ($II_B$) ou ($II'_A$) ou ($II'_B$) dans laquelle $R_1$ représente un radical méthyle en position 13béta et $R_2$ et $R_3$ chacun un atome d'hydrogène.

**Revendications pour l'Etat contractant suivant : GR**

1. Procédé pour préparer les produits de formule générale (I) :

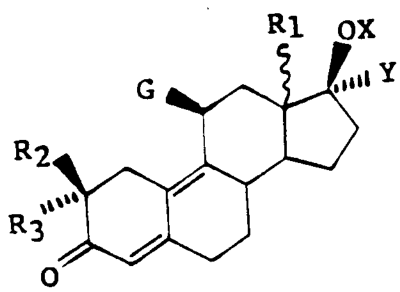

(I)

dans laquelle $R_1$ représente un radical hydrocarboné aliphatiue renfermant de un à huit atomes de carbone, $R_2$ et $R_3$ identiques ou différents représentent un atome d'hydrogène ou un radical alkyle renfermant de un à quatre atomes de carbone, G représente un groupe hydrocarboné renfermant de un à dix-huit atomes de carbone et éventuellement un ou plusieurs hétéroatomes identiques ou différents, lié au noyau stéroïde par un atome de carbone et

ou bien X représente $X_A$, $X_A$ étant un atome d'hydrogène, un radical alkyle renfermant de un à huit atomes de carbone, un radical arylalkyle renfermant de sept à quinze atomes de carbone ou un radical acyle renfermant de un à huit atomes de carbone et dans ce cas Y représente $Y_A$, $Y_A$ étant un groupe

-B-O-CO-A-Z

dans lequel B représente un radical aliphatique bivalent, linéaire ou ramifié, saturé ou insaturé, renfermant de un à huit atomes de carbone, A représente ou bien un radical aliphatique bivalent, linéaire ou ramifié, saturé ou insaturé, renfermant de un à six atomes de carbone et éventuellement

interrompu ou terminé par un radical aromatique bivalent, ou bien A représente un radical aromatique bivalent, et Z représente une des fonctions -COOH ou $-SO_3H$, ces fonctions pouvant être éventuellement salifiées sous forme d'un sel de métal alcalin ou alcalino-terreux, d'un sel d'ammonium ou d'un sel d'amine,
ou bien X représente $X_B$, $X_B$ étant un groupe

-CO-A-Z

dans lequel A et Z sont tels que définis précédemment et Y représente alors $Y_B$, $Y_B$ étant un des groupes choisis parmi $-C \equiv C-R_4$, $-CH=CH-R_4$ ou $-CH_2-CH_2-R_4$ dans lesquels $R_4$ représente un atome d'hydrogène, un atome d'halogène, un radical trialkylsilyle renfermant de trois à douze atomes de carbone, un radical alkyle renfermant de un à six atomes de carbone, linéaire ou ramifié ou un radical phényle, lesdits radicaux alkyle et phényle étant éventuellement substitués, le trait ondulé en position 13 signifie que le radical $R_1$ peut se trouver en position alpha ou béta, caractérisé en ce que

A - les produits de formule ($II_A$) ou de formule ($II_B$) :

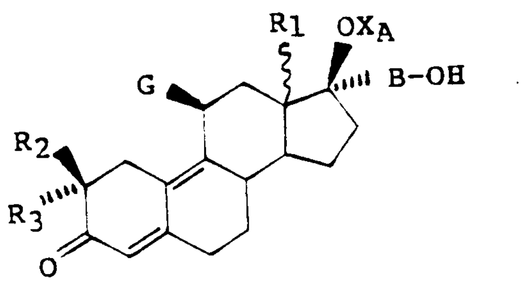

($II_A$)

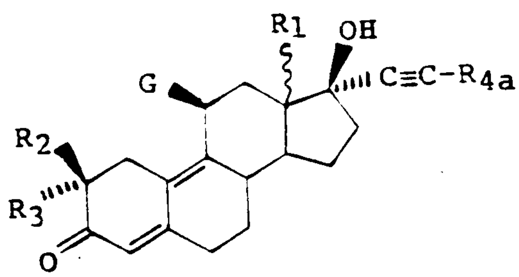

($II_B$)

dans lesquelles $R_1$, $R_2$, $R_3$ et G ont la signification indiquée à la revendication 1 et $R_{4a}$ a les valeurs indiquées à la revendication 1 pour $R_4$ ainsi que ces valeurs dans lesquelles les fonctions réactives sont protégées, sont soumis, dans un solvant neutre et en présence d'une base,

a) à l'action d'un produit de formule ($III_1$) :

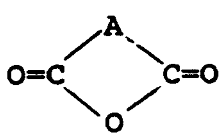

($III_1$)

pour obtenir après si nécessaire déprotection des fonctions réactives protégées et si désiré salification de la fonction carboxy, les produits de formule générale (I) répondant respectivement aux formule ($I_{A1}$) et ($I_{B1}$) :

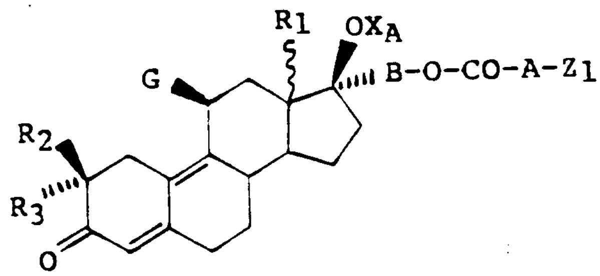

$(I_{A1})$

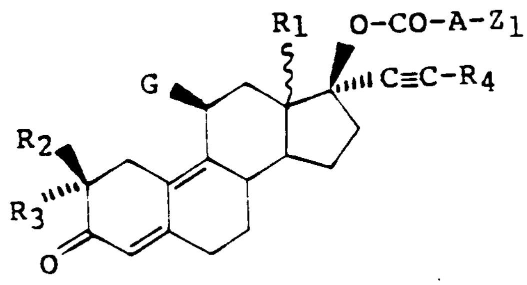

$(I_{B1})$

dans lesquelles $Z_1$ représente un radical carboxy éventuellement salifié,
b) à l'action d'un produit de formule $(III_2)$ :

HOOC-A-U $(III_2)$

ou encore d'un dérivé fonctionnel de cet acide dans laquelle U représente un des groupes -COOH, -$COOR_5$ dans lequel $R_5$ représente un radical alkyle renfermant de un à six atomes de carbone ou un radical arylalkyle renfermant de sept à douze atomes de carbone, ou -SH, pour obtenir, après si nécessaire déprotection des fonctions réactives protégées, respectivement les produits de formules $(IV_A)$ et $(IV_B)$ :

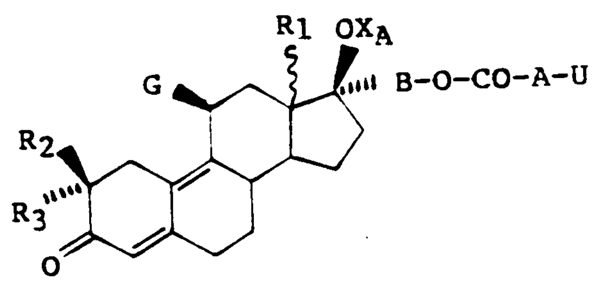

$(IV_A)$

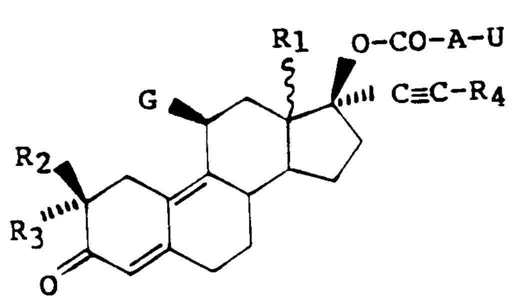

$(IV_B)$

produits de formules $(IV_A)$ et $(IV_B)$ qui :

- dans le cas où U represente le groupe carboxy libre, correspondent après salification éventuelle, aux produits de formules $(I_{A1})$ et $(I_{B1})$,

- dans le cas où U représente le groupe $-COOR_5$, correspondent aux produits de formules ($IV_C$) et ($IV_D$) :

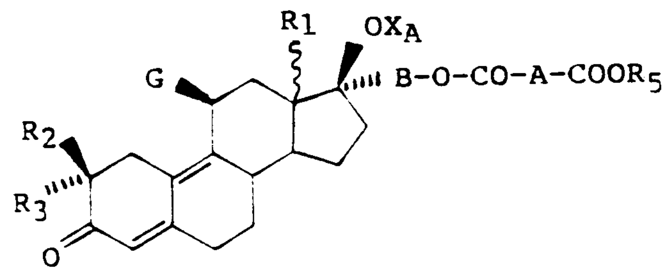

($IV_C$)

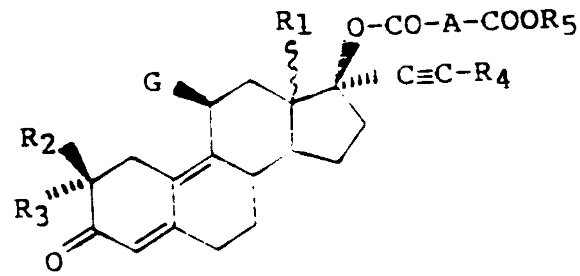

($IV_D$)

produits de formules ($IV_C$) et ($IV_D$) qui sont hydrolysés ou saponifiés pour obtenir respectivement les produits de formules ($I_{A1}$) et ($I_{B1}$),

- dans le cas où U représente le groupe -SH, correspondent aux produits de formules ($IV_E$) et ($IV_F$) :

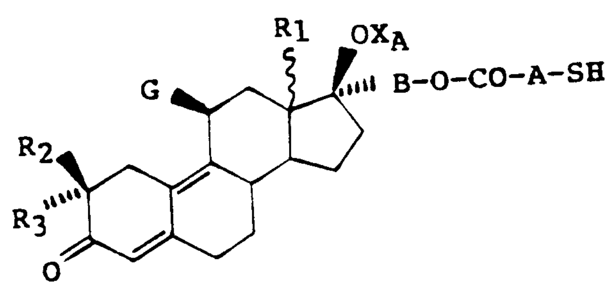

($IV_E$)

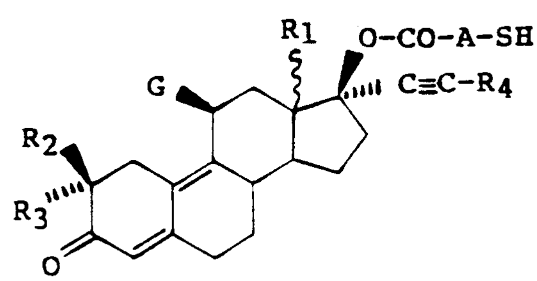

($IV_F$)

produits de formules ($IV_E$) et ($IV_F$) qui sont oxydés et si désire salifiés pour obtenir les produits de formule générale (I) répondant respectivement aux formules ($I_{A2}$) et ($I_{B2}$) :

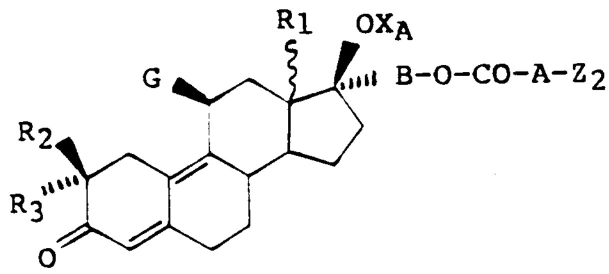

$(I_{A2})$

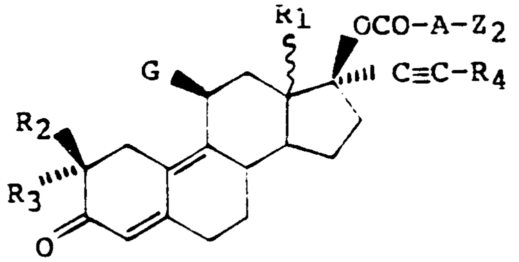

$(I_{B2})$

dans lesquelles $Z_2$ représente un groupe sulfo éventuellement salifié,

c) ou à l'action d'un produit de formule $(III_3)$ :

$HOOC\text{-}A\text{-}SO_2Cl$ $(III_3)$

ou encore d'un dérivé fonctionnel de cet acide, pour obtenir si nécessaire après déprotection des fonctions reactives contenues dans $R_{4a}$ et si désiré salification, respectivement les produits de formule $(I_{A2})$ et $(I_{B2})$ ;

en ce que :

B - les produits de formule $(I_{B1})$, $(I_{B2})$ et $(IV'_D)$ sont soumis si désiré :

a) soit à un agent d'hydrogénation des triples liaisons pour obtenir respectivement les produits de formules $(I_{B3})$, $(I_{B4})$ et $(V_D)$ :

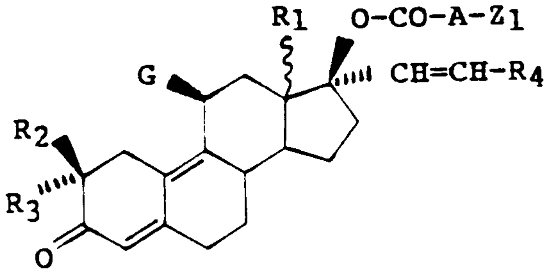

$(I_{B3})$

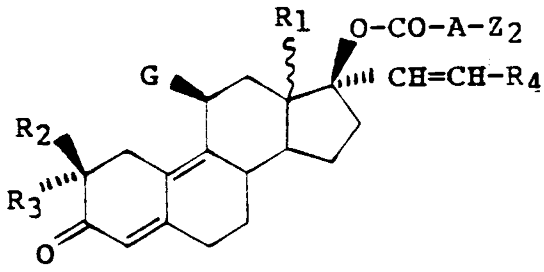

$(I_{B4})$

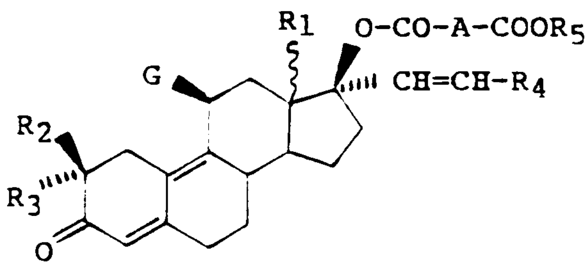

$(V_D)$

produits qui sont si désiré soumis à un agent d'hydrogénation des doubles liaisons pour obtenir respectivement des produits de formule $(I_{B5})$, $(I_{B6})$ et $(VI_D)$ :

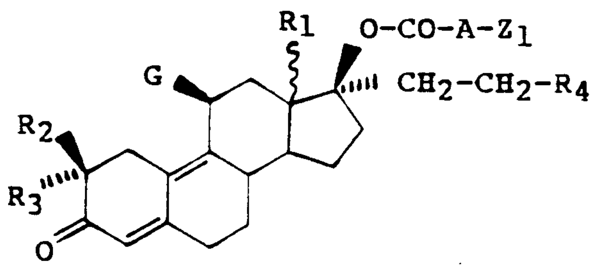

$(I_{B5})$

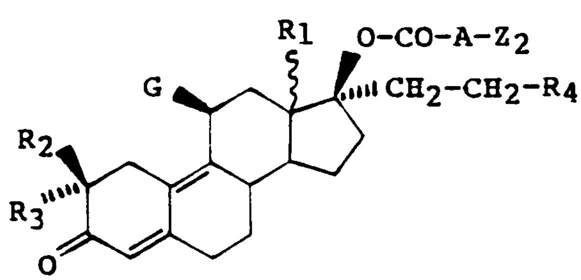

$(I_{B6})$

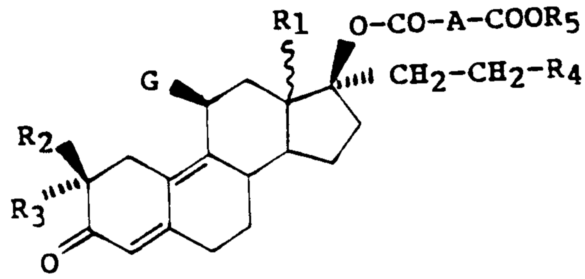

(VI$_D$)

soit à un agent d'hydrogénation directe des triples liaisons en simples liaisons pour obtenir respectivement les produits de formules ($I_{B5}$), ($I_{B6}$) et ($VI_D$) ;

b) les produits de formules ($V_D$) et ($VI_D$) sont soit hydrolysés, soit saponifiés pour obtenir respectivement les produits de formules ($I_{B3}$) et ($I_{B5}$).

**2.** Procédé pour préparer les produits de formule générale (I) telle que définie à la revendication 1, répondant à la formule (I') :

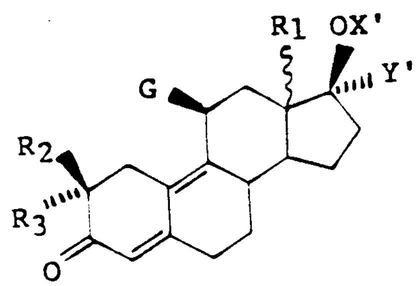

(I')

dans laquelle

ou bien X' représente un atome d'hydrogène et dans ce cas Y' représente $Y'_A$, $Y'_A$ étant un groupe :

-B'-O-CO-A'-Z'

dans lequel B' représente un des radicaux bivalents $-CH=CH-CH_2-$ ou $-C\equiv C-CH_2-$, A' représente un des radicaux bivalents $-(CH_2)_n-$ dans lequel n peut prendre une des valeurs de deux à six ou un radical ortho-, méta- ou para-phénylène et Z' représente les fonctions carboxy ou sulfo ou leur sel de sodium, ou bien X' représente $X'_B$, $X'_B$ étant un groupe :

$-CO-(CH_2)_n-Z'$

dans lequel n et Z' sont tels que définis précédemment et Y' représente alors $Y'_B$, $Y'_B$ étant un des groupes $-C\equiv C-R'_4$ ou $-CH=CH-R'_4$ dans lesquels $R'_4$ représente un atome d'hydrogène, un atome d'halogène, un radical triméthylsilyle, ou un radical methyle éventuellement substitue par un ou plusieurs atomes d'halogène, un radical hydroxy ou par un groupe alklyloxy, alkylthio ou alkylamino renfermant de un à quatre atomes de carbone, dialkylamino renfermant de deux à huit atomes de carbone ou trialkylsilyle renfermant de trois à douze atomes de carbone, caractérisé en ce que :

- a) soit l'on soumet le produit de formule ($II'_A$) ou ($II'_B$) :

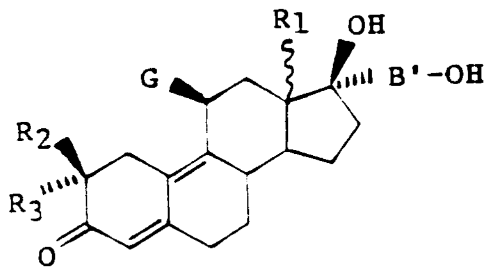

($II'_A$)

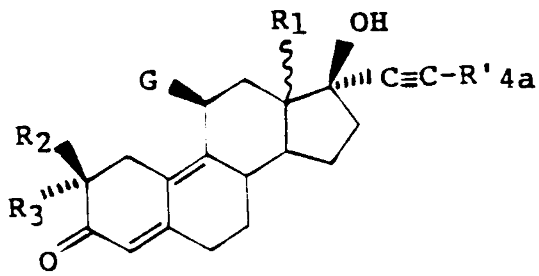

($II'_B$)

dans laquelle $R_1$, $R_2$, $R_3$ et G ont les valeurs indiquées à la revendication 2 et $R'_{4a}$ valeurs indiquées à la revendication 2 pour $R'_4$ ainsi que ces valeurs dans lesquelles les fonctions hydroxylées sont protégées, dans un solvant neutre et en présence d'une base à l'action d'un produit de formule ($III'_1$) :

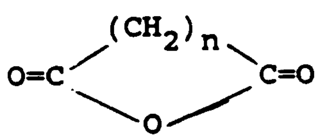

($III'_1$)

dans laquelle n prend une des valeurs de deux à six pour obtenir, après si nécessaire déprotection de la fonction hydroxy protégée contenue dans $R'_4$ et si désiré, salification de la fonction carboxy libre par action d'une solution de bicarbonate de sodium, respectivement le produit de formule ($I'_{A1}$) ou ($I'_{B1}$) :

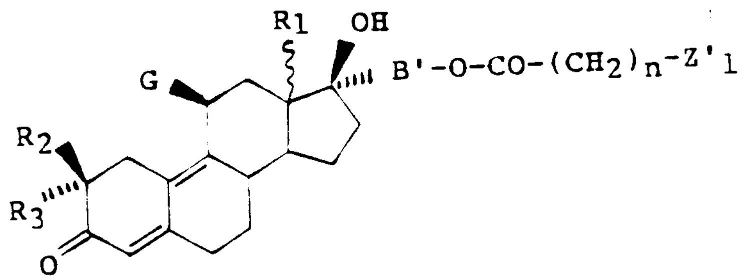

(I'$_{A1}$)

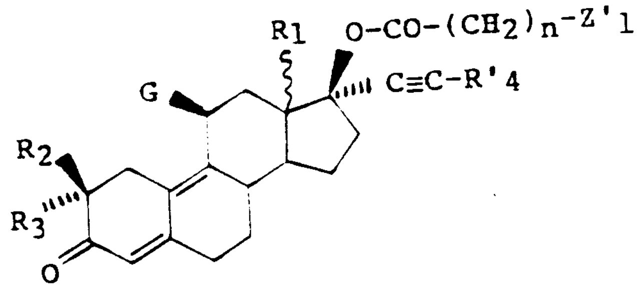

(I'$_{B1}$)

dans laquelle $Z'_1$ représente un groupe carboxy libre ou son sel de sodium,
b) soit l'on soumet le produit de formule (II'$_A$) telle que définie ci-dessus à l'action en présence d'une base azotée d'un produit de formule (III'$_3$) :

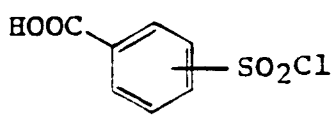

(III'$_3$)

pour obtenir, si nécessaire et si désiré, salification par une solution de bicarbonate de sodium, un produit de formule (I'$_{A2}$) :

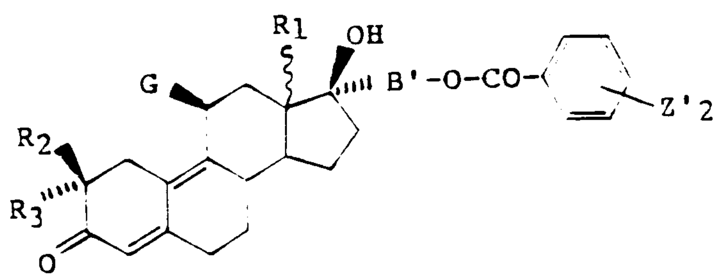

(I'$_{A2}$)

dans lesquelles $Z'_2$ représente un groupe sulfo ou son sel de sodium ;
produit de formule (I'$_{B1}$) que l'on soumet, si désiré, à l'action d'hydrogène en présence de catalyseur pour obtenir les produits de formule (I'$_{B3}$) :

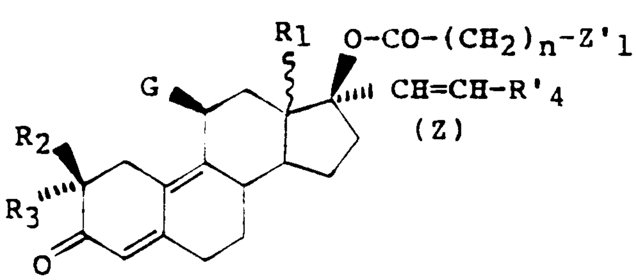

(I'$_{B3}$)

**3.** Procédé selon la revendication 1 ou 2, caractérisé en ce que l'on utilise au départ un produit de formule ($II_A$) ou ($II_B$) ou ($II'_A$) ou ($II'_B$) dans laquelle G représente un radical aryle substitué par un atome d'halogène, un radical aryle, un radical acyle renfermant de un à huit atomes de carbone ou par une des fonctions $-NH_2$, -NHR′ ou -NR′R″, dans lesquelles R′ et R″ identiques ou différents représentent un radical phényle, ou un radical alkyle primaire, secondaire ou tertiaire renfermant de 1 à 8 atomes de carbone, comportant éventuellement un ou plusieurs hétéroatomes, choisis parmi les atomes d'oxygène, d'azote et de soufre, ou éventuellement substitué par un hétérocycle, ou R′ et R″ représentent avec l'atome d'azote auquel ils sont liés, un radical hétérocyclique éventuellement substitué et comportant éventuellement un autre hétéroatome choisi parmi les atomes d'azote, d'oxygène et de soufre, ou bien par une des fonctions -0R‴ ou -SR‴ dans lesquelles R‴ représente un radical phényle ou un radical alkyle primaire, secondaire ou tertiaire renfermant de 1 à 8 atomes de carbone, comportant éventuellement un ou plusieurs hétéroatomes, choisis parmi les atomes d'oxygène, d'azote, de soufre ou éventuellement substitué par un hétérocycle, ou G représente un radical 2, 3 ou 4-pyridyle.

**4.** Procédé selon la revendication 3, caractérisé en ce que l'on utilise au départ un produit de formule ($II_A$) ou ($II_B$) ou ($II'_A$) ou ($II'_B$) dans laquelle G représente un radical phényle substitué en position 4 par un radical amino, (méthylamino), (diméthylamino) ou son N-oxyde, (diéthylamino), (dipropylamino), (N-méthyl éthylamino), (N-méthyl isopropylamino), (N-méthyl isobutylamino), (N-méthyl isopentylamino), 1-pyrrolidinyle, [2-(diméthylamino) N-méthyl éthylamino], [N-méthyl 2-(1-pyrrolidinyl) éthylamino], [N-méthyl 2-(4-morpholinyl) éthylamino], (4-méthyl 1-pipérazinyle), formyle, acétyle, méthoxy, phénoxy, [2-(diméthylamino) éthoxy], [2-(1-pyrrolidinyl) éthoxy], [2-(4-morpholinyl) éthoxy], (méthylthio), (éthylthio), (isopentylthio), [2-(diméthylamino) éthylthio], [2-(1-pyrrolidinyl) éthylthio] [2-(4-morpholinyl) éthylthio], (triméthylsilyle), méthyle, isopropyle, [(dimethylamino) méthyle], ou par un des atomes de fluor, de chlore ou de brome.

**5.** Procédé selon l'une quelconque des revendications 1 à 4, caractérisé en ce que l'on utilise au départ un produit de formule ($II_A$) ou ($II_B$) ou ($II'_A$) ou ($II'_B$)dans laquelle $R_1$ représente un radical méthyle en position 13béta et $R_2$ et $R_3$ chacun un atome d'hydrogène.

**6.** Procédé selon l'une quelconque des revendications 1 à 5, caractérisé en ce que l'on prépare l'un des produits dont les noms suivent :
- succinate de sodium et de 21-chloro 11béta-[4-(diméthylamino) phényl] 3-oxo 19-nor 17alpha-pregna-4,9-dièn-20-yn-17béta-yle,
- succinate de sodium et de 11béta-[4-(méthylthio) phényl] 3-oxo 17alpha-(1-propynyl) estra-4,9-dièn-17béta-yle.

**7.** Procédé de préparation de compositions pharmaceutiques, caractérisé en ce que l'on met à titre de principe actif l'un au moins des produits de formule (I) ou l'un au moins de leurs sels pharmaceutiquement acceptables tels que définis à la revendication 1 sous une forme destinée à cet usage.

**8.** Procédé de préparation de compositions pharmaceutiques, caractérisé en ce que l'on met à titre de principe actif l'un au moins des produits de formule (I′) ou l'un au moins de leurs sels pharmaceutiquement acceptables tels que définis à la revendication 2 sous une forme destinée à cet usage.

**9.** Procédé selon la revendication 8, caractérisé en ce que le principe actif est choisi dans le groupe constitué par les produits nommés à la revendication 6 et leurs sels pharmaceutiquement acceptables.

**10.** A titre de produits industriels nouveaux, les produits de formules ($IV_C$), ($IV_D$), ($IV_E$), ($IV_F$), ($V_D$) et ($VI_D$).

## Claims

**Claims for the following Contracting States : AT, BE, CH, DE, DK, FR, GB, IT, LI, LU, NL, SE**

**1.** The products of general formula (I):

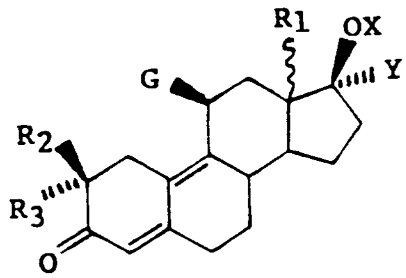

(I)

in which $R_1$ represents an aliphatic hydrocarbon radical containing 1 to 8 carbon atoms, $R_2$ and $R_3$, identical or different, represent a hydrogen atom or an alkyl radical containing 1 to 4 carbon atoms, G represents a hydrocarbon group containing 1 to 18 carbon atoms and optionally one or more identical or different heteroatoms, linked to the steroid nucleus by a carbon atom and
either X represents $X_A$, $X_A$ being a hydrogen atom, an alkyl radical containing 1 to 8 carbon atoms, an arylalkyl radical containing 7 to 15 carbon atoms or an acyl radical containing 1 to 8 carbon atoms and in this case Y represents $Y_A$, $Y_A$ being a

-B-O-CO-A-Z

group in which B represents a saturated or unsaturated, linear or branched bivalent aliphatic radical, containing 1 to 8 carbon atoms, A represents either a saturated or unsaturated, linear or branched bivalent aliphatic radical containing 1 to 6 carbon atoms and optionally interrupted or terminated by a bivalent aromatic radical, or A represents a bivalent aromatic radical, and Z represents one of the -COOH or $-SO_3H$ functions, these functions being able to be optionally salified in the form of an alkali metal or alkaline-earth salt, an ammonium salt or an amine salt,
or X represents $X_B$, $X_B$ being a

-CO-A-Z

group in which A and Z are as defined previously and Y then represents $Y_B$, $Y_B$ being one of the groups chosen from $-C{\equiv}C-R_4$, $-CH=CH-R_4$ or $-CH_2-CH_2-R_4$ in which $R_4$ represents a hydrogen atom, a halogen atom, a trialkylsilyl radical containing 3 to 12 carbon atoms, a linear or branched alkyl radical containing 1 to 6 carbon atoms or a phenyl radical, the said alkyl and phenyl radicals being optionally substituted, the wavy line in position 13 means that the $R_1$ radical can be found in alpha or beta position.

**2.** The products of general formula (I) as defined in claim 1, corresponding to formula (I'):

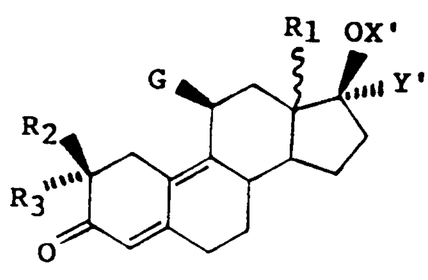

(I')

in which
either X' represents a hydrogen atom and in this case Y' represents $Y'_A$, $Y'_A$ being a group:

-B'-O-CO-A'-Z'

in which B' represents one of the bivalent radicals $-CH=CH-CH_2-$ or $C\equiv C-CH_2-$, A' represents one of the bivalent radicals $-(CH_2)_n-$ in which n can take one of the values 2 to 6 or an ortho-, meta- or para-phenylene radical and Z' represents the carboxy or sulpho functions or their sodium salt,
or X' represents $X'_B$, $X'_B$ being a group:

$-CO-(CH_2)_n-Z'$

in which n and Z' are as defined previously and Y' then represents $Y'_B$, $Y'_B$ being one of the $-C\equiv C-R'_4$ or $-CH=CH-R'_4$ groups in which $R'_4$ represents a hydrogen atom, a halogen atom, a trimethylsilyl radical, or a methyl radical optionally substituted by one or more halogen atoms, a hydroxy radical or by an alklyloxy, alkylthio or alkylamino group containing 1 to 4 carbon atoms, a dialkylamino group containing 2 to 8 carbon atoms or a trialkylsilyl group containing 3 to 12 carbon atoms.

**3.** The products of general formula (I) as defined in claims 1 and 2 in which G represents an aryl radical substituted by a halogen atom, an aryl radical, an acyl radical containing 1 to 8 carbon atoms or by one of the $-NH_2$, -NHR' or -NR'R'' functions, in which R' and R'', identical or different, represent a phenyl radical, or a primary, secondary or tertiary alkyl radical, containing 1 to 8 carbon atoms, optionally containing one or more heteroatoms, chosen from oxygen, nitrogen and sulphur atoms, or optionally substituted by a heterocycle, or R' and R'' represent with the nitrogen atom to which they are linked, an optionally substituted heterocyclic radical optionally containing another heteroatom chosen from nitrogen, oxygen and sulphur atoms, or by one of the -OR''' or -SR''' functions in which R''' represents a phenyl radical or a primary, secondary or tertiary alkyl radical containing 1 to 8 carbon atoms, optionally containing one or more heteroatoms, chosen from oxygen, nitrogen, sulphur atoms or optionally substituted by a heterocycle, or G represents a 2, 3 or 4-pyridyl radical.

**4.** The products of general formula (I) as defined in claim 3, in which G represents a phenyl radical substituted in position 4 by an one of the following radicals: amino, (methylamino), (dimethylamino) or its N-oxide, (diethylamino), (dipropylamino), (N-methyl ethylamino), (N-methyl isopropylamino), (N-methyl isobutylamino), (N-methyl isopentylamino), 1-pyrrolidinyl, [2-(dimethylamino) N-methyl ethylamino], [N-methyl 2-(1-pyrrolidinyl) ethylamino], [N-methyl 2-(4-morpholinyl) ethylamino], (4-methyl 1-piperazinyl), formyl, acetyl, methoxy, phenoxy, [2-(dimethylamino) ethoxy], [2-(1-pyrrolidinyl) ethoxy], [2-(4-morpholinyl) ethoxy], (methylthio), (ethylthio), (isopentylthio), [2-(dimethylamino) ethylthio], [2-(1-pyrrolidinyl) ethylthio] [2-(4-morpholinyl) ethylthio], (trimethylsilyl), methyl, isopropyl, [(dimethylamino) methyl], or by one of the fluorine, chlorine or bromine atoms.

**5.** The products of general formula (I) as defined in one of claims 1 to 4 in which $R_1$ represents a methyl radical in position 13beta and $R_2$ and $R_3$ each represent a hydrogen atom.

**6.** The products of general formula (I) as defined in claim 5 the names of which follow:
- sodium and 21-chloro 11beta-[4-(dimethylamino) phenyl] 3-oxo 19-nor 17alpha-pregna-4,9-dien-20-yn-17beta-yl succinate,
- sodium and 11beta-[4-(methylthio) phenyl] 3-oxo 17alpha-(1-propynyl) estra-4,9-dien-17beta-yl succinate.

**7.** Preparation process for products of general formula (I) as defined in claim 1 characterized in that

A - the products of formula ($II_A$) or formula ($II_B$):

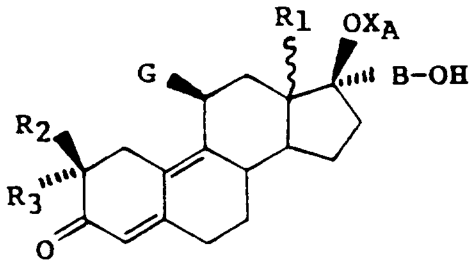

($II_A$)

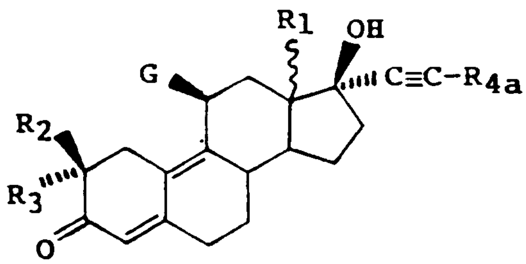

($II_B$)

in which $R_1$, $R_2$, $R_3$ and G have the meaning indicated in claim 1 and $R_{4a}$ has the values indicated in claim 1 for $R_4$ as well as those values in which the reactive functions are protected, are subjected, in a neutral solvent and in the presence of a base,

a) to the action of a product of formula ($III_1$):

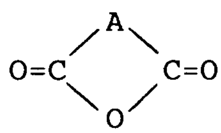

($III_1$)

in order to obtain after if necessary deprotection of the protected reactive functions and if desired salification of the carboxy function, the products of general formula (I) corresponding to formulae ($I_{A1}$) and ($I_{B1}$) respectively:

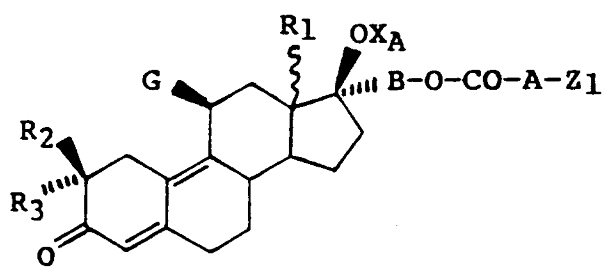

($I_{A1}$)

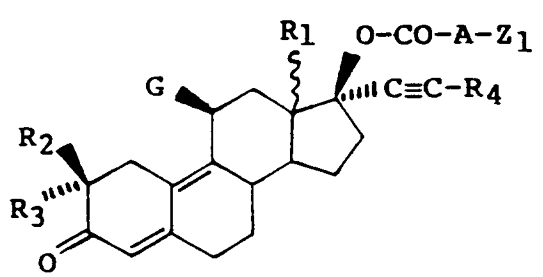

($I_{B1}$)

in which $Z_1$ represents an optionally salified carboxy radical,
b) to the action of a product of formula ($III_2$):

HOOC-A-U ($III_2$)

or also a functional derivative of this acid in which U represents one of the -COOH, $-COOR_5$ groups in which $R_5$ represents an alkyl radical containing 1 to 6 carbon atoms or an arylalkyl radical containing 7 to 12 carbon atoms, or -SH, in order to obtain, after if necessary deprotection of the protected reactive functions, the products of formulae ($IV_A$) and ($IV_B$) respectively:

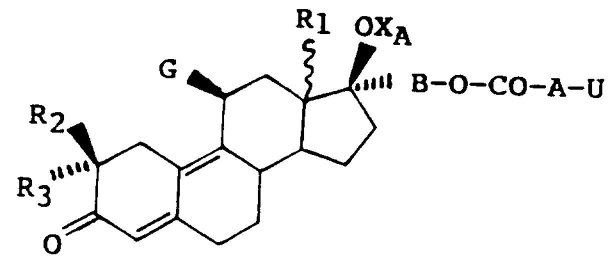

($IV_A$)

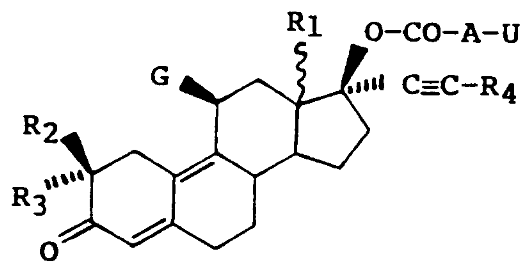

($IV_B$)

which products of formulae ($IV_A$) and ($IV_B$):
- in the case where U represents the free carboxy group, correspond, after optional salification, to the products of formulae ($I_{A1}$) and ($I_{B1}$),
- in the case where U represents the $-COOR_5$ group, correspond to the products of formulae ($IV_C$) and ($IV_D$):

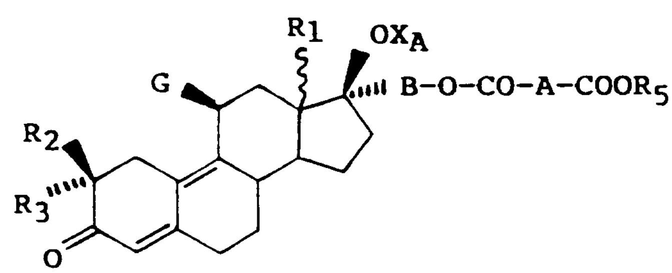

($IV_C$)

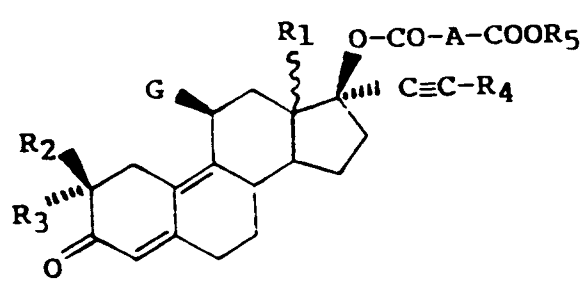

($IV_D$)

which products of formulae ($IV_C$) and ($IV_D$) are hydrolyzed or saponified in order to obtain the products of formulae ($I_{A1}$) and ($I_{B1}$) respectively,

- in the case where U represents the -SH group, correspond to the products of formulae ($IV_E$) and ($IV_F$):

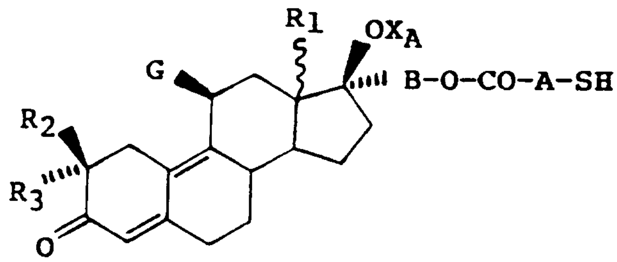

($IV_E$)

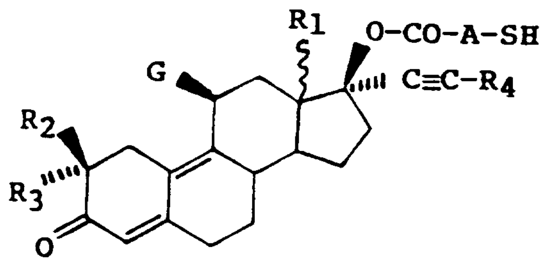

($IV_F$)

which products of formulae ($IV_E$) and ($IV_F$) are oxidized and if desired salified in order to obtain the products of general formula (I) corresponding to formulae ($I_{A2}$) and ($I_{B2}$) respectively:

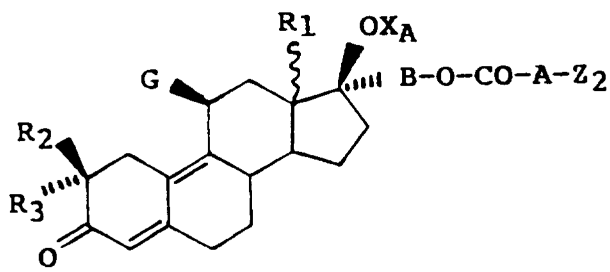

($I_{A2}$)

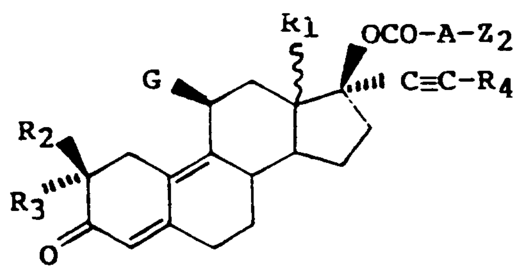

($IB_2$)

in which $Z_2$ represents an optionally salified sulpho group,

c) or to the action of a product of formula ($III_3$):

$HOOC-A-SO_2Cl$ ($III_3$)

or also a functional derivative of this acid, in order to obtain if necessary after deprotection of the reactive functions contained in $R_{4a}$ and if desired salification, the products of formulae ($I_{A2}$) and

($I_{B2}$) respectively;

in that:

B - the products of formulae ($I_{B1}$), ($I_{B2}$) and ($IV'_D$) are subjected if desired:

a) either to a hydrogenation agent of the triple bonds in order to obtain the products of formulae ($I_{B3}$), ($I_{B4}$) and ($V_D$) respectively:

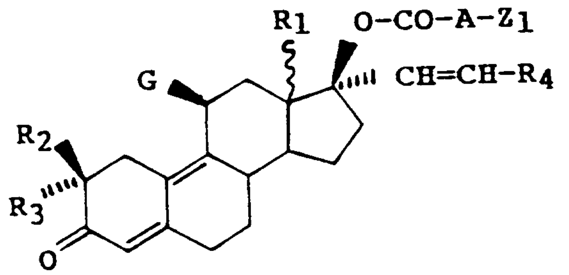

($I_{B3}$)

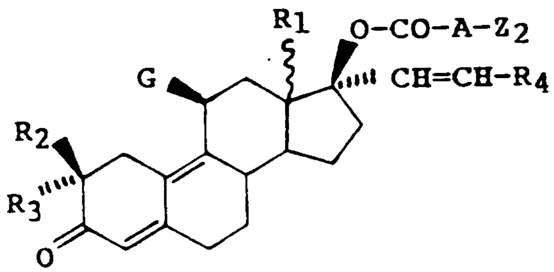

($I_{B4}$)

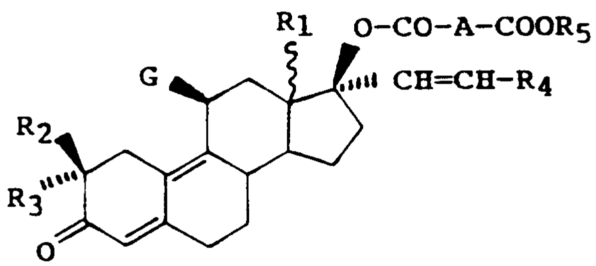

($V_D$)

which products are if desired subjected to a hydrogenation agent of the double bonds in order to obtain the products of formulae ($I_{B5}$), ($I_{B6}$) and ($VI_D$) respectively:

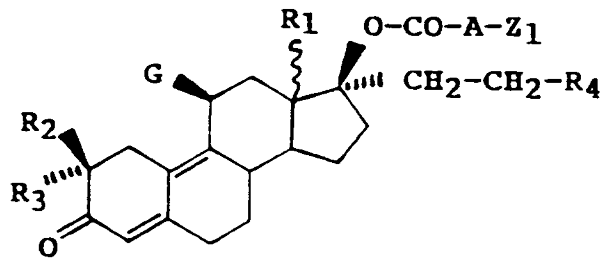

$(I_{B5})$

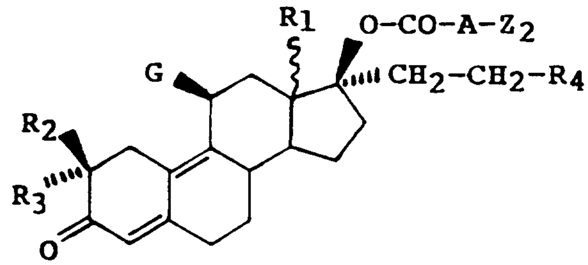

$(I_{B6})$

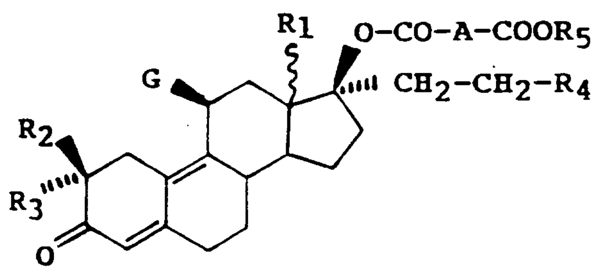

$(VI_D)$

or to a direct hydrogenation agent of the triple bonds into single bonds in order to obtain the products of formulae $(I_{B5})$, $(I_{B6})$ and $(VI_D)$ respectively;

b) the products of formulae $(V_D)$ and $(VI_D)$ are either hydrolyzed, or saponified, in order to obtain the products of formulae $(I_{B3})$ and $(I_{B5})$ respectively.

**8.** Process according to claim 7 for the preparation of the products of formula (I') as defined in claim 2 characterized in that:

- a) either the product of formula $(II'_A)$ or $(II'_B)$:

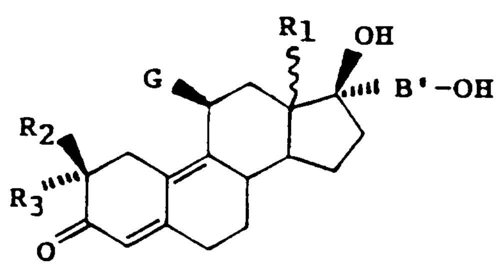

$(II'_A)$

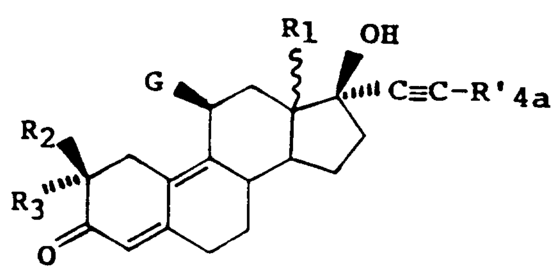

$(II'_B)$

in which $R_1$, $R_2$, $R_3$ and G have the values indicated in claim 2 and $R'_{4a}$ has the values indicated in claim 2 for $R'_4$ as well as those values in which the hydroxylated functions are protected, in a neutral solvent and in the presence of a base, is subjected to the action of a product of formula ($III'_1$):

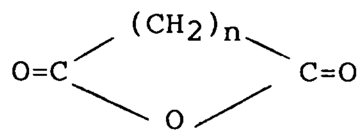

($III'_1$)

in which n takes one of the values from 2 to 6, in order to obtain, after if necessary deprotection of the protected hydroxy function contained in $R'_4$ and if desired, salification of the free carboxy function by the action of a sodium bicarbonate solution, the product of formula ($I'_{A1}$) or ($I'_{B1}$) respectively:

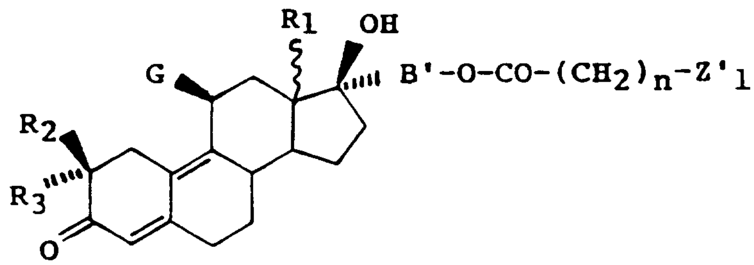

($I'_{A1}$)

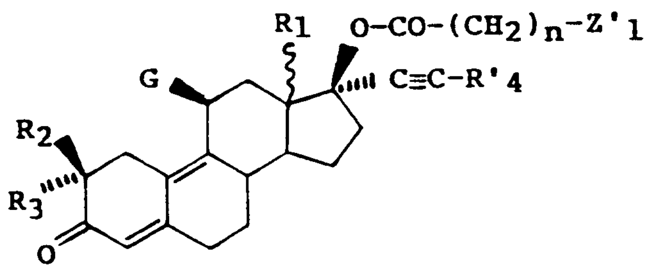

($I'_{B1}$)

in which $Z'_1$ represents a free carboxy group or its sodium salt;

b) or the product of formula ($II'_A$) as defined above is subjected, in the presence of a nitrogenous base, to the action of a product of formula ($III'_3$):

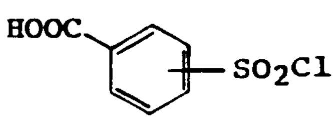

($III'_3$)

in order to obtain, if necessary and if desired, after salification by a sodium bicarbonate solution, a product of formula ($I'_{A2}$):

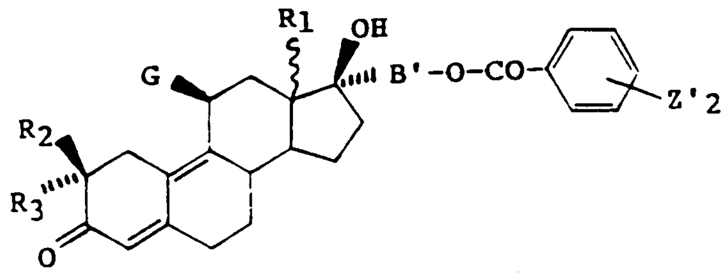

(I'A2)

in which $Z'_2$ represents a sulpho group or its sodium salt; which product of formula ($I'_{B1}$) is subjected, if desired, to the action of hydrogen in the presence of a catalyst in order to obtain the products of formula ($I'_{B3}$):

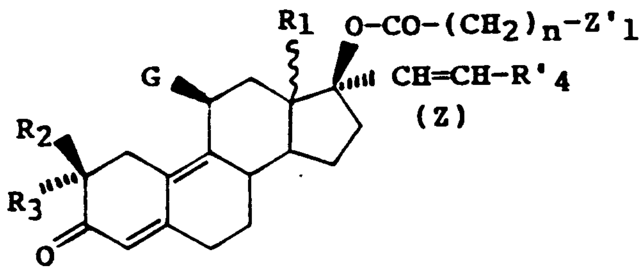

(I'B3)

**9.** As medicaments, the products of general formula (I) as defined in claim 1.

**10.** As medicaments, the products of general formula (I) as defined in any one of claims 2 to 6.

**11.** Pharmaceutical compositions containing as active ingredient, at least one of the medicaments defined according to claim 9 or 10.

**12.** As new industrial products, the products of formulae ($IV_C$), ($IV_D$), ($IV_E$), ($IV_F$), ($V_D$) and ($VI_D$).

**Claims for the following Contracting State : ES**

**1.** Process for preparing the products of general formula (I):

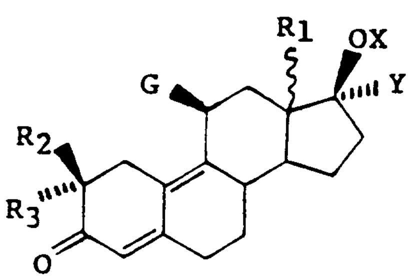

(I)

in which $R_1$ represents an aliphatic hydrocarbon radical containing 1 to 8 carbon atoms, $R_2$ and $R_3$, identical or different, represent a hydrogen atom or an alkyl radical containing 1 to 4 carbon atoms, G represents a hydrocarbon group containing 1 to 18 carbon atoms and optionally one or more identical or different heteroatoms, linked to the steroid nucleus by a carbon atom and
either X represents $X_A$, $X_A$ being a hydrogen atom, an alkyl radical containing 1 to 8 carbon atoms, an arylalkyl radical containing 7 to 15 carbon atoms or an acyl radical containing 1 to 8 carbon atoms and in this case Y represents $Y_A$, $Y_A$ being a

-B-O-CO-A-Z

group in which B represents a saturated or unsaturated, linear or branched bivalent aliphatic radical, containing 1 to 8 carbon atoms, A represents either a saturated or unsaturated, linear or branched bivalent aliphatic radical containing 1 to 6 carbon atoms and optionally interrupted or terminated by a bivalent aromatic radical, or A represents a bivalent aromatic radical, and Z represents one of the -COOH or $-SO_3H$ functions, these functions being able to be optionally salified in the form of an alkali metal or alkaline-earth salt, an ammonium salt or an amine salt,
or X represents $X_B$, $X_B$ being a

-CO-A-Z

group in which A and Z are as defined previously and Y then represents $Y_B$, $Y_B$ being one of the groups chosen from $-C{\equiv}C-R_4$, $-CH=CH-R_4$ or $-CH_2-CH_2-R_4$ in which $R_4$ represents a hydrogen atom, a halogen atom, a trialkylsilyl radical containing 3 to 12 carbon atoms, a linear or branched alkyl radical containing 1 to 6 carbon atoms or a phenyl radical, the said alkyl and phenyl radicals being optionally substituted, the wavy line in position 13 means that the $R_1$ radical can be found in alpha or beta position, characterized in that

A - the products of formula ($II_A$) or formula ($II_B$):

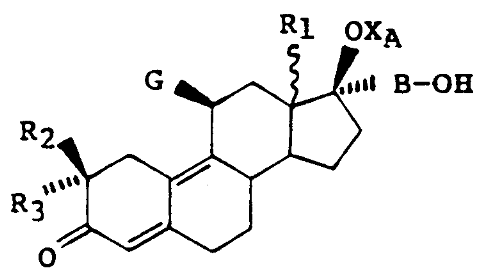

($II_A$)

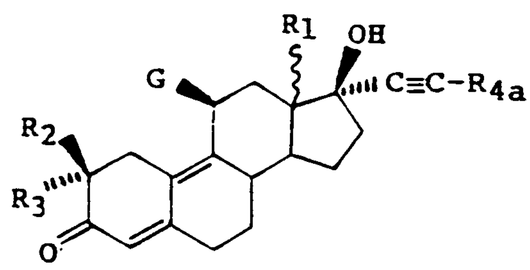

($II_B$)

in which $R_1$, $R_2$, $R_3$ and G have the meaning indicated in claim 1 and $R_{4a}$ has the values indicated in claim 1 for $R_4$ as well as these values in which the reactive functions are protected, are subjected, in a neutral solvent and in the presence of a base,

a) to the action of a product of formula ($III_1$):

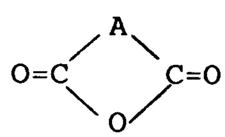

($III_1$)

in order to obtain after if necessary deprotection of the protected reactive functions and if desired salification of the carboxy function, the products of general formula (I) corresponding to formulae ($I_{A1}$) and ($I_{B1}$) respectively:

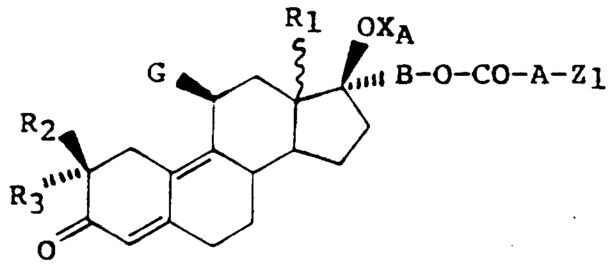

($I_{A1}$)

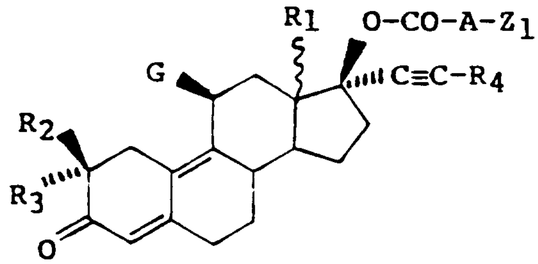

($I_{B1}$)

in which $Z_1$ represents an optionally salified carboxy radical,
b) to the action of a product of formula ($III_2$):

HOOC-A-U ($III_2$)

or also a functional derivative of this acid in which U represents one of the -COOH, $-COOR_5$ groups in which $R_5$ represents an alkyl radical containing 1 to 6 carbon atoms or an arylalkyl radical containing 7 to 12 carbon atoms, or -SH, in order to obtain, after if necessary deprotection of the protected reactive functions, the products of formulae ($IV_A$) and ($IV_B$) respectively:

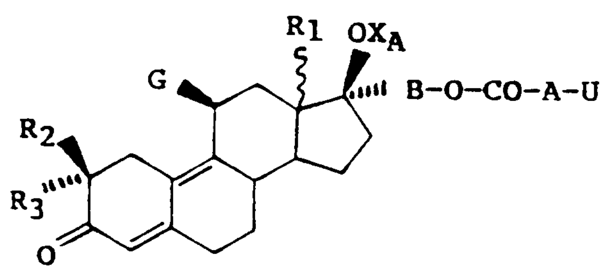

($IV_A$)

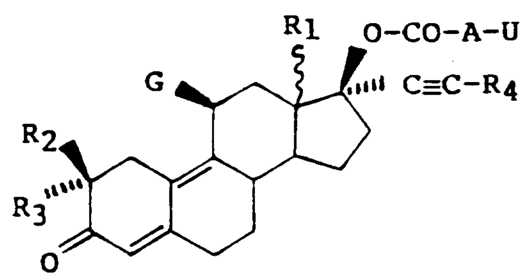

($IV_B$)

which products of formulae ($IV_A$) and ($IV_B$):
- in the case where U represents the free carboxy group, correspond, after optional salification, to the products of formulae ($I_{A1}$) and ($I_{B1}$),
- in the case where U represents the $-COOR_5$ group, correspond to the products of formulae ($IV_C$) and ($IV_D$):

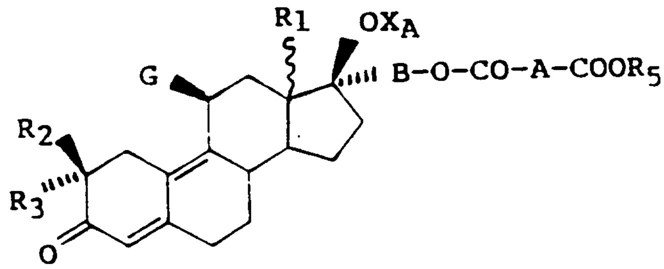

(IV$_C$)

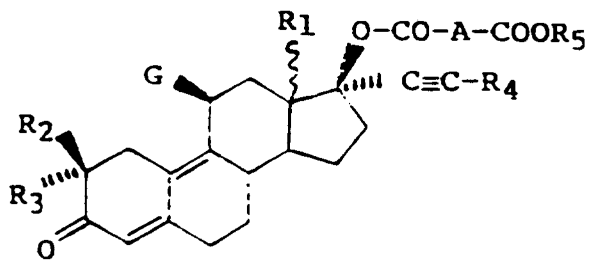

(IV$_D$)

which products of formulae (IV$_C$) and (IV$_D$) are hydrolyzed or saponified in order to obtain the products of formulae ($I_{A1}$) and ($I_{B1}$) respectively,

- in the case where U represents the -SH group, correspond to the products of formulae (IV$_E$) and (IV$_F$):

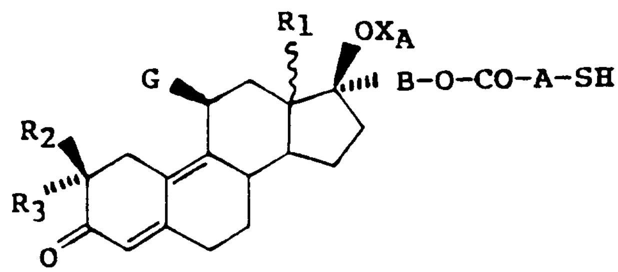

(IV$_E$)

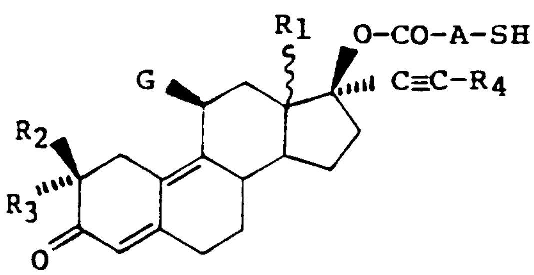

(IV$_F$)

which products of formulae (IV$_E$) and (IV$_F$) are oxidized and if desired salified in order to obtain the products of general formula (I) corresponding to formulae ($I_{A2}$) and ($I_{B2}$) respectively:

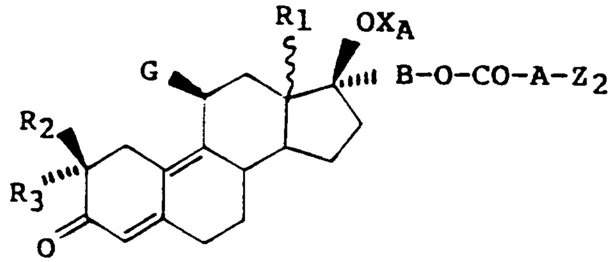

$(I_{A2})$

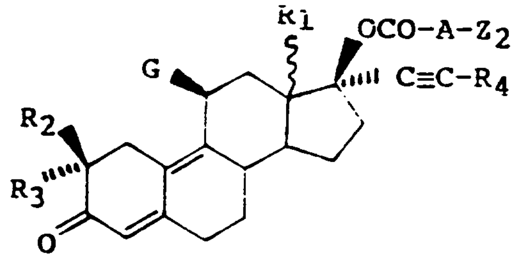

$(IB_2)$

in which $Z_2$ represents an optionally salified sulpho group,

c) or to the action of a product of formula $(III_3)$:

$HOOC\text{-}A\text{-}SO_2Cl$ $(III_3)$

or also a functional derivative of this acid, in order to obtain if necessary after deprotection of the reactive functions contained in $R_{4a}$ and if desired salification, the products of formulae $(I_{A2})$ and $(I_{B2})$ respectively;

in that:

B - the products of formulae $(I_{B1})$, $(I_{B2})$ and $(IV'_D)$ are subjected if desired:

a) either to a hydrogenation agent of the triple bonds in order to obtain the products of formulae $(I_{B3})$, $(I_{B4})$ and $(V_D)$ respectively:

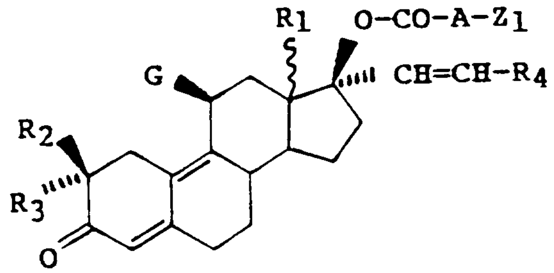

$(I_{B3})$

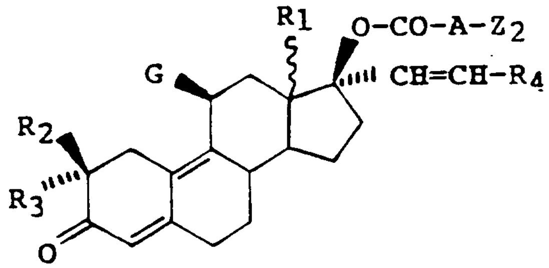

$(I_{B4})$

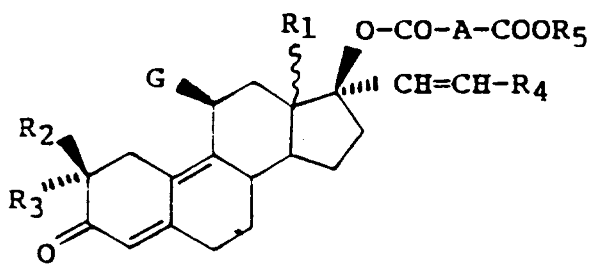

$(V_D)$

which products are if desired subjected to a hydrogenation agent of the double bonds in order to obtain the products of formulae $(I_{B5})$, $(I_{B6})$ and $(VI_D)$ respectively:

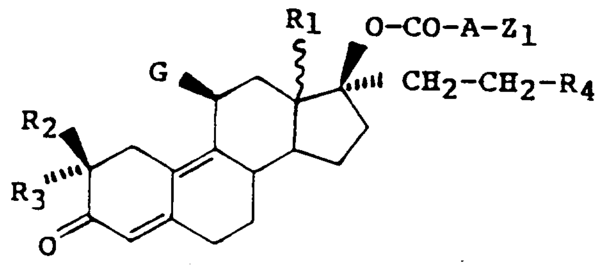

$(I_{B5})$

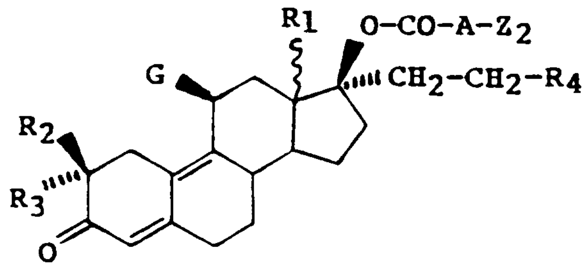

$(I_{B6})$

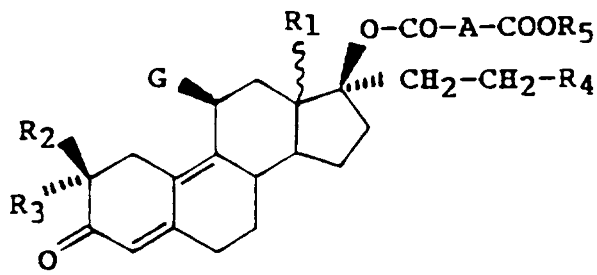

$(VI_D)$

or to a direct hydrogenation agent of the triple bonds into single bonds in order to obtain the products of formulae $(I_{B5})$, $(I_{B6})$ and $(VI_D)$ respectively;

b) the products of formulae $(V_D)$ and $(VI_D)$ are either hydrolyzed, or saponified, in order to obtain the products of formulae $(I_{B3})$ and $(I_{B5})$ respectively.

**2.** Process for preparing the products of general formula (I) as defined in claim 1, corresponding to formula (I'):

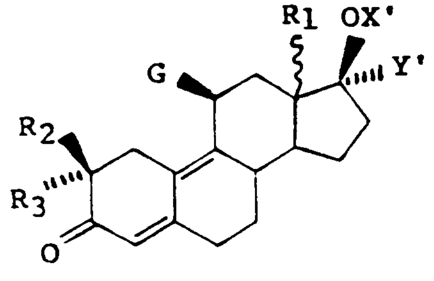

(I')

in which

either X' represents a hydrogen atom and in this case Y' represents $Y'_A$, $Y'_A$ being a group:

-B'-O-CO-A'-Z'

in which B' represents one of the bivalent radicals $-CH=CH-CH_2-$ or $C{\equiv}C-CH_2-$, A' represents one of the bivalent radicals $-(CH_2)_n-$ in which n can take one of the values 2 to 6 or an ortho-, meta- or para-phenylene radical and Z' represents the carboxy or sulpho functions or their sodium salt,

or X' represents $X'_B$, $X'_B$ being a group:

$-CO-(CH_2)_n-Z'$

in which n and Z' are as defined previously and Y' then represents $Y'_B$, $Y'_B$ being one of the $-C \equiv C-R'_4$ or $-CH=CH-R'_4$ groups in which $R'_4$ represents a hydrogen atom, a halogen atom, a trimethylsilyl radical, or a methyl radical optionally substituted by one or more halogen atoms, a hydroxy radical or by an alklyloxy, alkylthio or alkylamino group containing 1 to 4 carbon atoms, a dialkylamino group containing 2 to 8 carbon atoms or a trialkylsilyl group containing 3 to 12 carbon atoms, characterized in that:

- a) either the product of formula ($II'_A$) or ($II'_B$):

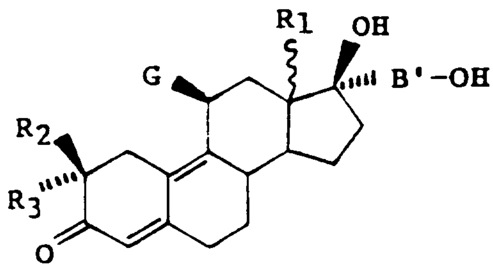

($II'_A$)

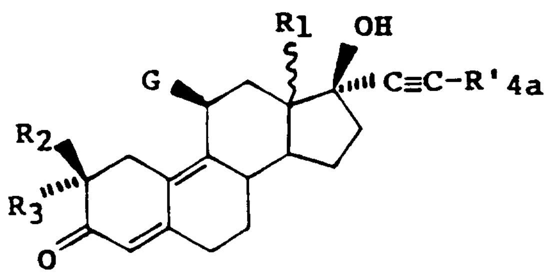

($II'_B$)

in which $R_1$, $R_2$, $R_3$ and G have the values indicated in claim 2 and $R'_{4a}$ has the values indicated in claim 2 for $R'_4$ as well as those values in which the hydroxylated functions are protected, in a neutral solvent and in the presence of a base, is subjected to the action of a product of formula ($III'_1$):

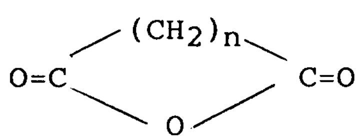

($III'_1$)

in which n takes one of the values from 2 to 6, in order to obtain, after if necessary deprotection of the protected hydroxy function contained in $R'_4$ and if desired, salification of the free carboxy function by the action of a sodium bicarbonate solution, the product of formula ($I'_{A1}$) or ($I'_{B1}$) respectively:

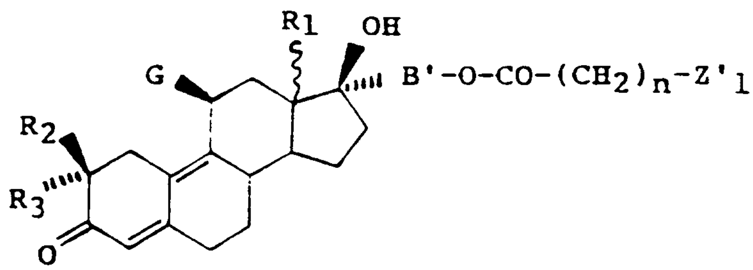

(I'A1)

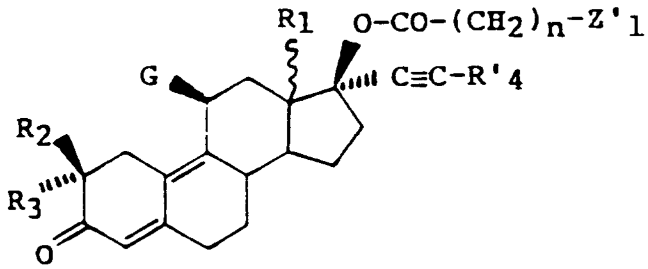

(I'B1)

in which $Z'_1$ represents a free carboxy group or its sodium salt;

b) or the product of formula ($II'_A$) as defined above is subjected, in the presence of a nitrogenous base, to the action of a product of formula ($III'_3$):

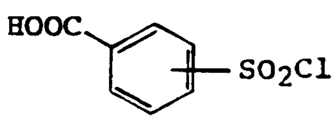

(III'3)

in order to obtain, if necessary and if desired, after salification by a sodium bicarbonate solution, a product of formula ($I'_{A2}$):

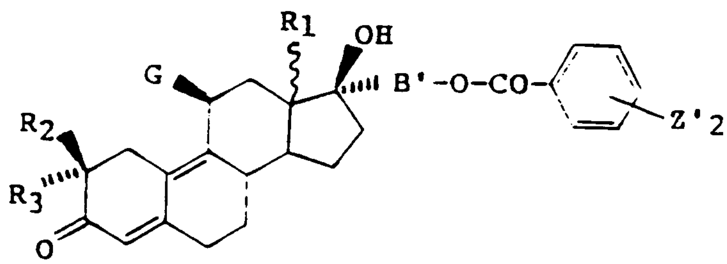

(I'A2)

in which $Z'_2$ represents a sulpho group or its sodium salt; which product of formula ($I'_{B1}$) is subjected, if desired, to the action of hydrogen in the presence of a catalyst in order to obtain the products of formula ($I'_{B3}$):

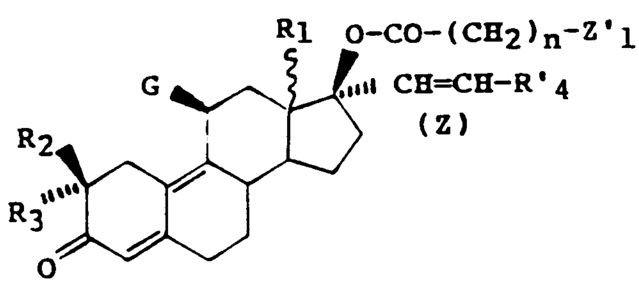

(I'B3)

3. Process according to claim 1 or 2, characterized in that a product of formula ($II_A$) or ($II_B$) or ($II'_A$) or ($II'_B$) is used at the start in which G represents an aryl radical substituted by a halogen atom, an aryl radical, an acyl radical containing 1 to 8 carbon atoms or by one of the $-NH_2$, -NHR' or -NR'R" functions, in which R' and R", identical or different, represent a phenyl radical, or a primary, secondary or tertiary alkyl radical containing 1 to 8 carbon atoms, optionally containing one or more heteroatoms, chosen from oxygen, nitrogen and sulphur atoms, or optionally substituted by a heterocycle, or R' and R" represent with the nitrogen atom to which they are linked, an optionally substituted heterocyclic radical and optionally containing another heteroatom chosen from nitrogen, oxygen and sulphur atoms, or by one of the -OR''' or -SR''' functions in which R''' represents a phenyl radical or a primary, secondary or tertiary alkyl radical containing 1 to 8 carbon atoms, optionally containing one or more heteroatoms, chosen from oxygen, nitrogen, sulphur atoms or optionally substituted by a heterocycle, or G represents a 2, 3 or 4-pyridyl radical.

4. Process according to claim 3, characterized in that a product of formula ($II_A$) or ($II_B$) or ($II'_A$) or ($II'_B$) is used at the start in which G represents a phenyl radical substituted in position 4 by one of the following radicals: amino, (methylamino), (dimethylamino) or its N-oxide, (diethylamino), (dipropylamino), (N-methyl ethylamino), (N-methyl isopropylamino), (N-methyl isobutylamino), (N-methyl isopentylamino), 1-pyrrolidinyl, [2-(dimethylamino) N-methyl ethylamino], [N-methyl 2-(1-pyrrolidinyl) ethylamino], [N-methyl 2-(4-morpholinyl) ethylamino], (4-methyl 1-piperazinyl), formyl, acetyl, methoxy, phenoxy, [2-(dimethylamino) ethoxy], [2-(1-pyrrolidinyl) ethoxy], [2-(4-morpholinyl) ethoxy], (methylthio), (ethylthio), (isopentylthio), [2-(dimethylamino) ethylthio], [2-(1-pyrrolidinyl) ethylthio] [2-(4-morpholinyl) ethylthio], (trimethylsilyl), methyl, isopropyl, [(dimethylamino) methyl], or by one of the fluorine, chlorine or bromine atoms.

5. Process according to any one of claims 1 to 4, characterized in that a product of formula ($II_A$) or ($II_B$) or ($II'_A$) or ($II'_B$) is used at the start in which $R_1$ represents a methyl radical in position 13beta and $R_2$ and $R_3$ each represent a hydrogen atom.

**Claims for the following Contracting State : GR**

1. Process for preparing the products of general formula (I):

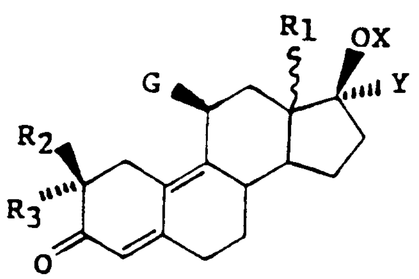

(I)

in which $R_1$ represents an aliphatic hydrocarbon radical containing 1 to 8 carbon atoms, $R_2$ and $R_3$, identical or different, represent a hydrogen atom or an alkyl radical containing 1 to 4 carbon atoms, G represents a hydrocarbon group containing 1 to 18 carbon atoms and optionally one or more identical or different heteroatoms, linked to the steroid nucleus by a carbon atom and
either X represents $X_A$, $X_A$ being a hydrogen atom, an alkyl radical containing 1 to 8 carbon atoms, an arylalkyl radical containing 7 to 15 carbon atoms or an acyl radical containing 1 to 8 carbon atoms and in this case Y represents $Y_A$, $Y_A$ being a

-B-O-CO-A-Z

group in which B represents a saturated or unsaturated, linear or branched bivalent aliphatic radical, containing 1 to 8 carbon atoms, A represents either a saturated or unsaturated, linear or branched bivalent aliphatic radical containing 1 to 6 carbon atoms and optionally interrupted or terminated by a bivalent aromatic radical, or A represents a bivalent aromatic radical, and Z represents one of the -COOH or $-SO_3H$ functions, these functions being able to be optionally salified in the form of an alkali

metal or alkaline-earth salt, an ammonium salt or an amine salt, or X represents $X_B$, $X_B$ being a

-CO-A-Z

group in which A and Z are as defined previously and Y then represents $Y_B$, $Y_B$ being one of the groups chosen from $-C{\equiv}C-R_4$, $-CH=CH-R_4$ or $-CH_2-CH_2-R_4$ in which $R_4$ represents a hydrogen atom, a halogen atom, a trialkylsilyl radical containing 3 to 12 carbon atoms, a linear or branched alkyl radical containing 1 to 6 carbon atoms or a phenyl radical, the said alkyl and phenyl radicals being optionally substituted, the wavy line in position 13 means that the $R_1$ radical can be found in alpha or beta position, characterized in that

A - the products of formula ($II_A$) or formula ($II_B$):

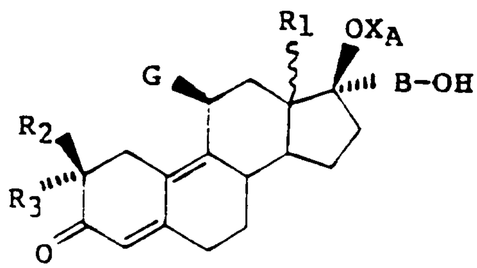

($II_A$)

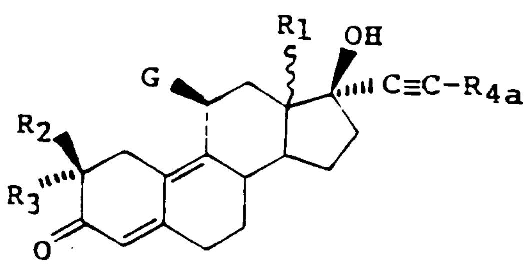

($II_B$)

in which $R_1$, $R_2$, $R_3$ and G have the meaning indicated in claim 1 and $R_{4a}$ has the values indicated in claim 1 for $R_4$ as well as these values in which the reactive functions are protected, are subjected, in a neutral solvent and in the presence of a base,

a) to the action of a product of formula ($III_1$):

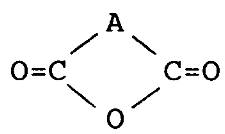

($III_1$)

in order to obtain after if necessary deprotection of the protected reactive functions and if desired salification of the carboxy function, the products of general formula (I) corresponding to formulae ($I_{A1}$) and ($I_{B1}$) respectively:

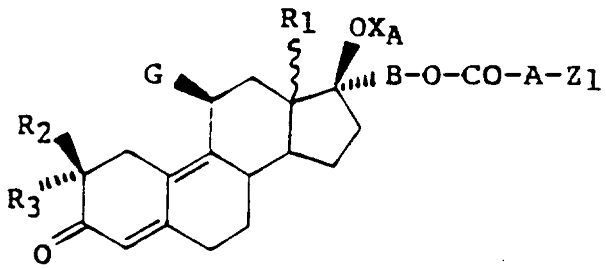

$(I_{A1})$

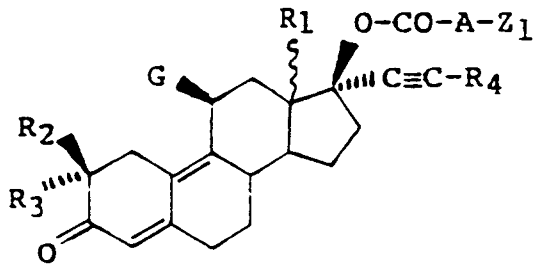

$(I_{B1})$

in which $Z_1$ represents an optionally salified carboxy radical,
b) to the action of a product of formula $(III_2)$:

HOOC-A-U $(III_2)$

or also a functional derivative of this acid in which U represents one of the -COOH, $-COOR_5$ groups in which $R_5$ represents an alkyl radical containing 1 to 6 carbon atoms or an arylalkyl radical containing 7 to 12 carbon atoms, or -SH, in order to obtain, after if necessary deprotection of the protected reactive functions, the products of formulae $(IV_A)$ and $(IV_B)$ respectively:

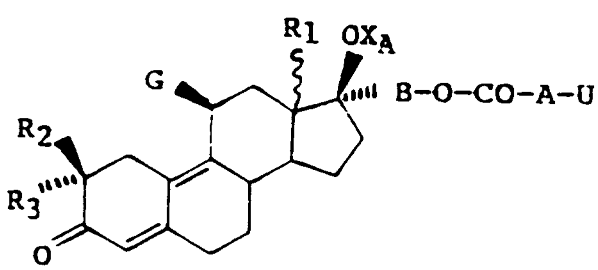

$(IV_A)$

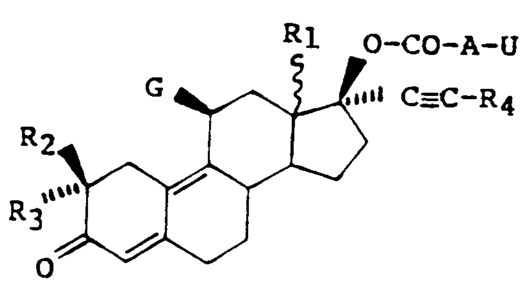

$(IV_B)$

which products of formulae $(IV_A)$ and $(IV_B)$:

- in the case where U represents the free carboxy group, correspond, after optional salification, to the products of formulae $(I_{A1})$ and $(I_{B1})$,
- in the case where U represents the $-COOR_5$ group, correspond to the products of formulae $(IV_C)$ and $(IV_D)$:

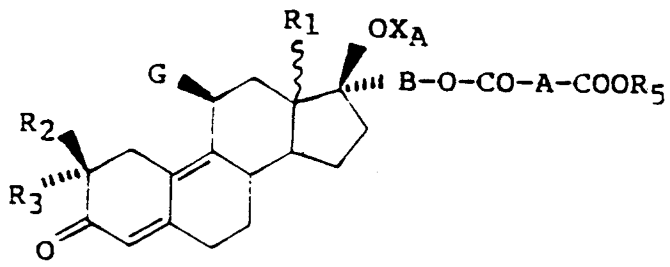

($IV_C$)

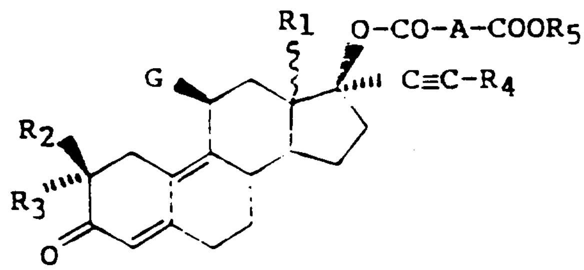

($IV_D$)

which products of formulae ($IV_C$) and ($IV_D$) are hydrolyzed or saponified in order to obtain the products of formulae ($I_{A1}$) and ($I_{B1}$) respectively,

- in the case where U represents the -SH group, correspond to the products of formulae ($IV_E$) and ($IV_F$):

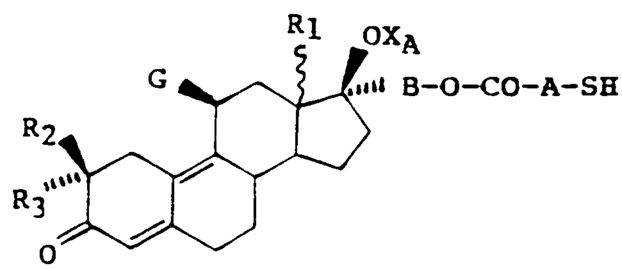

($IV_E$)

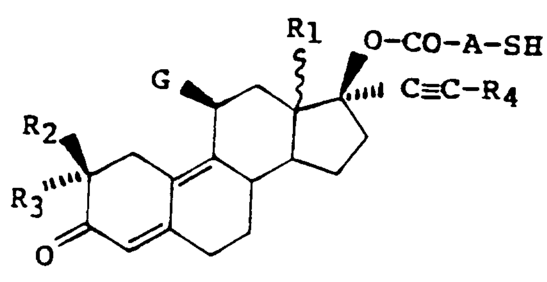

($IV_F$)

which products of formulae ($IV_E$) and ($IV_F$) are oxidized and if desired salified in order to obtain the products of general formula (I) corresponding to formulae ($I_{A2}$) and ($I_{B2}$) respectively:

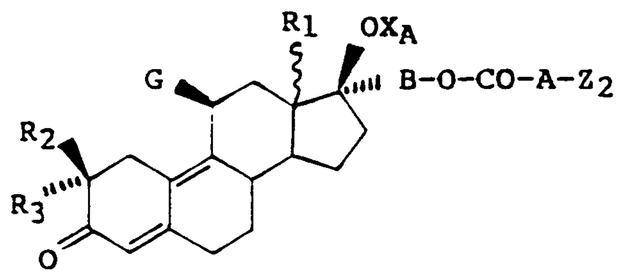

$(I_{A2})$

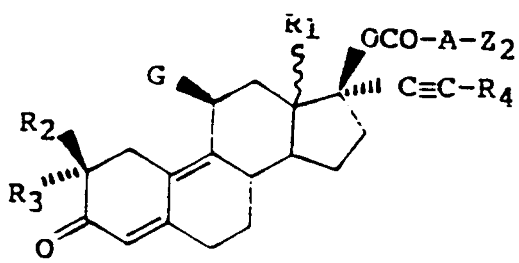

$(IB_2)$

in which $Z_2$ represents an optionally salified sulpho group,

c) or to the action of a product of formula $(III_3)$:

$HOOC\text{-}A\text{-}SO_2Cl$ $(III_3)$

or also a functional derivative of this acid, in order to obtain if necessary after deprotection of the reactive functions contained in $R_{4a}$ and if desired salification, the products of formulae $(I_{A2})$ and $(I_{B2})$ respectively;

in that:

B - the products of formulae $(I_{B1})$, $(I_{B2})$ and $(IV'_D)$ are subjected if desired:

a) <u>either</u> to a hydrogenation agent of the triple bonds in order to obtain the products of formulae $(I_{B3})$, $(I_{B4})$ and $(V_D)$ respectively:

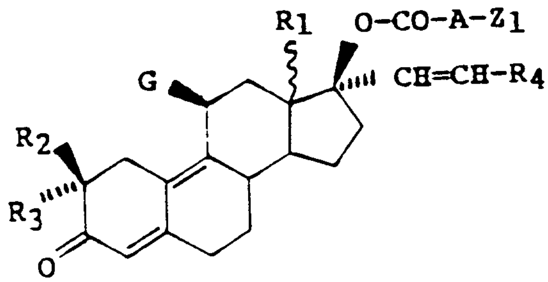

$(I_{B3})$

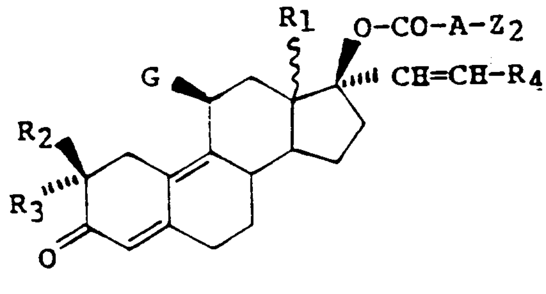

$(I_{B4})$

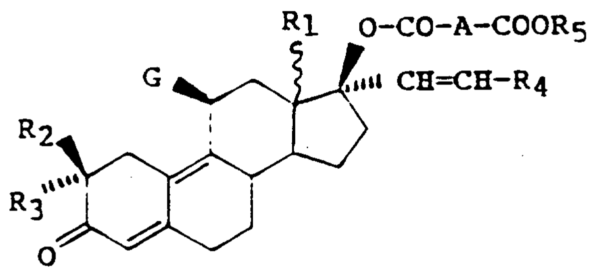

$(V_D)$

which products are if desired subjected to a hydrogenation agent of the double bonds in order to obtain the products of formulae $(I_{B5})$, $(I_{B6})$ and $(VI_D)$ respectively:

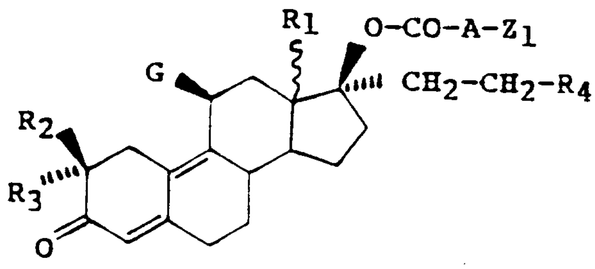

$(I_{B5})$

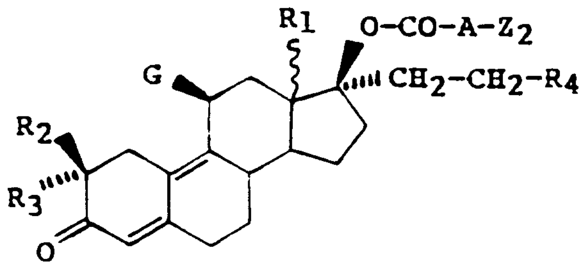

$(I_{B6})$

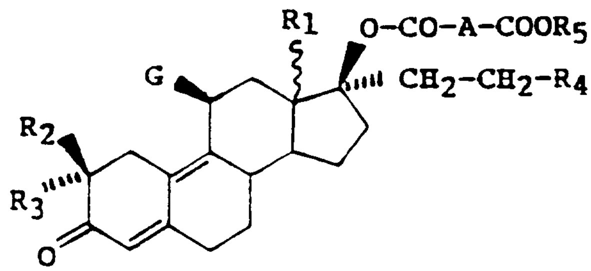

$(VI_D)$

or to a direct hydrogenation agent of the triple bonds into single bonds in order to obtain the products of formulae ($I_{B5}$), ($I_{B6}$) and ($VI_D$) respectively;

b) the products of formulae ($V_D$) and ($VI_D$) are either hydrolyzed, or saponified, in order to obtain the products of formulae ($I_{B3}$) and ($I_{B5}$) respectively.

**2.** Process for preparing the products of general formula (I) as defined in claim 1, corresponding to formula (I'):

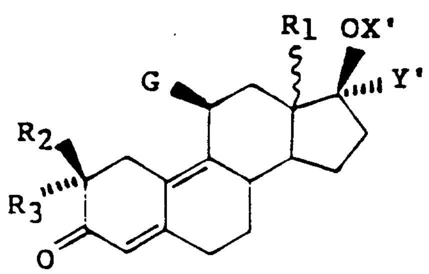

(I')

in which

either X' represents a hydrogen atom and in this case Y' represents $Y'_A$, $Y'_A$ being a group:

-B'-O-CO-A'-Z'

in which B' represents one of the bivalent radicals $-CH=CH-CH_2-$ or $C\equiv C-CH_2-$, A' represents one of the bivalent radicals $-(CH_2)_n-$ in which n can take one of the values 2 to 6 or an ortho-, meta- or para-phenylene radical and Z' represents the carboxy or sulpho functions or their sodium salt,

or X' represents $X'_B$, $X'_B$ being a group:

$-CO-(CH_2)_n-Z'$

in which n and Z' are as defined previously and Y' then represents $Y'_B$, $Y'_B$ being one of the $-C{\equiv}C-R'_4$ or $-CH=CH-R'_4$ groups in which $R'_4$ represents a hydrogen atom, a halogen atom, a trimethylsilyl radical, or a methyl radical optionally substituted by one or more halogen atoms, a hydroxy radical or by an alklyloxy, alkylthio or alkylamino group containing 1 to 4 carbon atoms, a dialkylamino group containing 2 to 8 carbon atoms or a trialkylsilyl group containing 3 to 12 carbon atoms, characterized in that:

- a) either the product of formula ($II'_A$) or ($II'_B$):

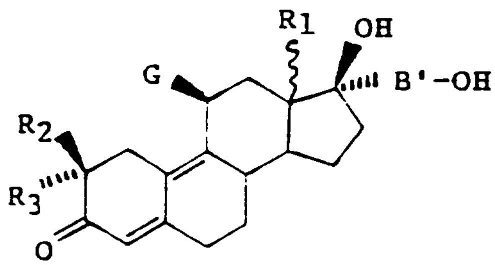

($II'_A$)

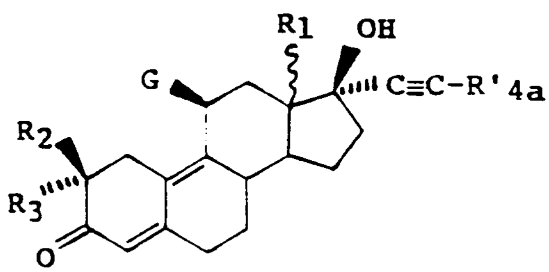

($II'_B$)

in which $R_1$, $R_2$, $R_3$ and G have the values indicated in claim 2 and $R'_{4a}$ has the values indicated in claim 2 for $R'_4$ as well as those values in which the hydroxylated functions are protected, in a neutral solvent and in the presence of a base, is subjected to the action of a product of formula ($III'_1$):

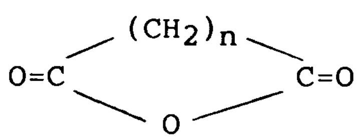

($III'_1$)

in which n takes one of the values from 2 to 6, in order to obtain, after if necessary deprotection of the protected hydroxy function contained in $R'_4$ and if desired, salification of the free carboxy function by the action of a sodium bicarbonate solution, the product of formula ($I'_{A1}$) or ($I'_{B1}$) respectively:

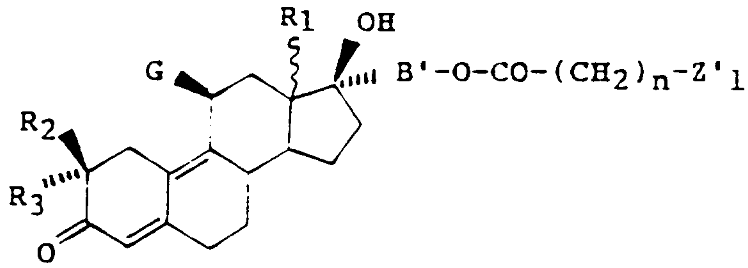

(I'A1)

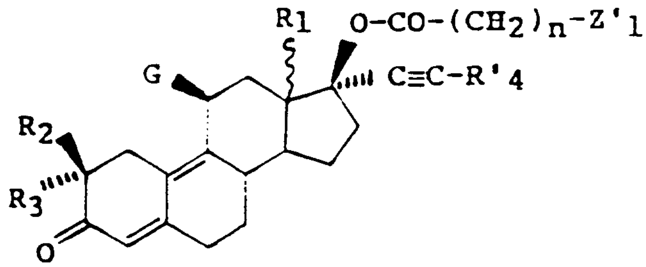

(I'B1)

in which Z'$_1$ represents a free carboxy group or its sodium salt;

b) or the product of formula (II'$_A$) as defined above is subjected, in the presence of a nitrogenous base, to the action of a product of formula (III'$_3$):

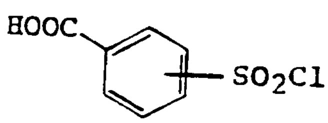

(III'3)

in order to obtain, if necessary and if desired, after salification by a sodium bicarbonate solution, a product of formula (I'$_{A2}$):

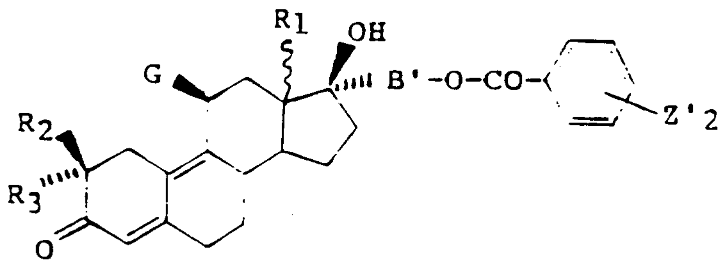

(I'A2)

in which Z'$_2$ represents a sulpho group or its sodium salt; which product of formula (I'$_{B1}$) is subjected, if desired, to the action of hydrogen in the presence of a catalyst in order to obtain the products of formula (I'$_{B3}$):

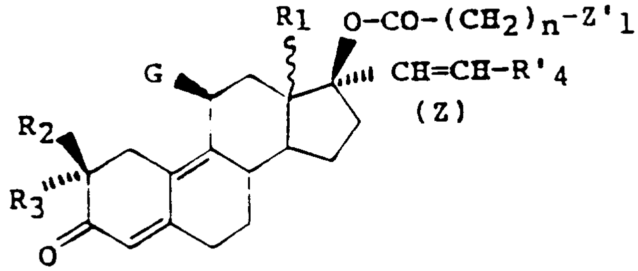

(I'B3)

**3.** Process according to claim 1 or 2, characterized in that a product of formula ($II_A$) or ($II_B$) or ($II'_A$) or ($II'_B$) is used at the start in which G represents an aryl radical substituted by a halogen atom, an aryl radical, an acyl radical containing 1 to 8 carbon atoms or by one of the $-NH_2$, -NHR' or -NR'R" functions, in which R' and R", identical or different, represent a phenyl radical, or a primary, secondary or tertiary alkyl radical containing 1 to 8 carbon atoms, optionally containing one or more heteroatoms, chosen from oxygen, nitrogen and sulphur atoms, or optionally substituted by a heterocycle, or R' and R" represent with the nitrogen atom to which they are linked, an optionally substituted heterocyclic radical and optionally containing another heteroatom chosen from nitrogen, oxygen and sulphur atoms, or by one of the -OR''' or -SR''' functions in which R''' represents a phenyl radical or a primary, secondary or tertiary alkyl radical containing 1 to 8 carbon atoms, optionally containing one or more heteroatoms, chosen from oxygen, nitrogen, sulphur atoms or optionally substituted by a heterocycle, or G represents a 2, 3 or 4-pyridyl radical.

**4.** Process according to claim 3, characterized in that a product of formula ($II_A$) or ($II_B$) or ($II'_A$) or ($II'_B$) is used at the start in which G represents a phenyl radical substituted in position 4 by one of the following radicals: amino, (methylamino), (dimethylamino) or its N-oxide, (diethylamino), (dipropylamino), (N-methyl ethylamino), (N-methyl isopropylamino), (N-methyl isobutylamino), (N-methyl isopentylamino), 1-pyrrolidinyl, [2-(dimethylamino) N-methyl ethylamino], [N-methyl 2-(1-pyrrolidinyl) ethylamino], [N-methyl 2-(4-morpholinyl) ethylamino], (4-methyl 1-piperazinyl), formyl, acetyl, methoxy, phenoxy, [2-(dimethylamino) ethoxy], [2-(1-pyrrolidinyl) ethoxy], [2-(4-morpholinyl) ethoxy], (methylthio), (ethylthio), (isopentylthio), [2-(dimethylamino) ethylthio], [2-(1-pyrrolidinyl) ethylthio] [2-(4-morpholinyl) ethylthio], (trimethylsilyl), methyl, isopropyl, [(dimethylamino) methyl], or by one of the fluorine, chlorine or bromine atoms.

**5.** Process according to any one of claims 1 to 4, characterized in that a product of formula ($II_A$) or ($II_B$) or ($II'_A$) or ($II'_B$) is used at the start in which $R_1$ represents a methyl radical in position 13beta and $R_2$ and $R_3$ each represent a hydrogen atom.

**6.** Process according to any one of claims 1 to 5, characterized in that one of the products is prepared of which the names follow:
- sodium and 21-chloro 11beta-[4-(dimethylamino) phenyl] 3-oxo 19-nor 17alpha-pregna-4,9-dien-20-yn-17beta-yl succinate,
- sodium and 11beta-[4-(methylthio) phenyl] 3-oxo 17alpha-(1-propynyl) estra-4,9-dien-17beta-yl succinate.

**7.** Preparation process for pharmaceutical compositions, characterized in that at least one of the products of formula (I) or at least one of their pharmaceutically acceptable salts as defined in claim 1 is used as active ingredient in a form intended for this use.

**8.** Preparation process for pharmaceutical compositions, characterized that at least one of the products of formula (I') or at least one of their pharmaceutically acceptable salts as defined in claim 2 is used as active ingredient in a form intended for this use.

**9.** Process according to claim 8, characterized in that the active ingredient is chosen from the group constituted by the products named in claim 6 and their pharmaceutically acceptable salts.

**10.** As new industrial products, the products of formulae ($IV_C$), ($IV_D$), ($IV_E$), ($IV_F$), ($V_D$) and ($VI_D$).

**Patentansprüche**

**Patentansprüche für folgende Vertragsstaaten : AT, BE, CH, DE, DK, FR, GB, IT, LI, LU, NL, SE**

1. Die Produkte der allgemeinen Formel (I):

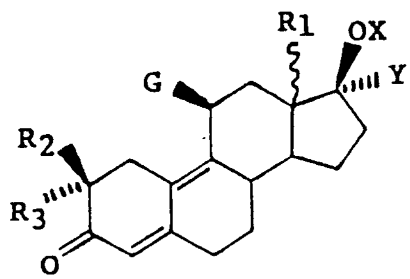

(I)

worin $R_1$ einen aliphatischen Kohlenwasserstoffrest, enthaltend 1 bis 8 Kohlenstoffatome, bedeutet, $R_2$ und $R_3$, die identisch oder verschieden sind, bedeuten ein Wasserstoffatom oder einen Alkylrest mit 1 bis 4 Kohlenstoffatomen, G bedeutet eine Kohlenwasserstoffgruppe mit 1 bis 18 Kohlenstoffatomen und gegebenenfalls einem oder mehreren identischen oder verschiedenen Heteroatom(en), gebunden an den Steroidkern durch ein Kohlenstoffatom, und
entweder X bedeutet $X_A$, wobei $X_A$ ein Wasserstoffatom, ein Alkylrest mit 1 bis 8 Kohlenstoffatomen, ein Arylalkylrest mit 7 bis 15 Kohlenstoffatomen oder ein Acylrest mit 1 bis 8 Kohlenstoffatomen ist, und Y bedeutet in diesem Fall $Y_A$,wobei $Y_A$ eine Gruppe

-B-O-CO-A-Z

ist, worin B einen gesättigten oder ungesättigten geraden oder verzweigten zweiwertigen aliphatischen Rest mit 1 bis 8 Kohlenstoffatomen bedeutet, A bedeutet entweder einen gesättigten oder ungesättigten, geraden oder verzweigten zweiwertigen aliphatischen Rest mit 1 bis 6 Kohlenstoffatomen, und gegebenenfalls unterbrochen oder beendet durch einen zweiwertigen aromatischen Rest, oder A bedeutet einen zweiwertigen aromatischen Rest, und Z bedeutet eine der Funktionen -COOH oder $-SO_3H$, wobei diese Funktionen gegebenenfalls als Salz in Form eines Alkali- oder Erdalkalimetallsalzes, eines Ammoniumsalzes oder eines Aminsalzes vorliegen können,
oder X bedeutet $X_B$, wobei $X_B$ eine Gruppe

-CO-A-Z

ist, worin A und Z wie vorstehend definiert sind, und Y bedeutet dann $Y_B$, wobei $Y_B$ eine der Gruppen ausgewählt aus $-C\equiv C-R_4$, $-CH=CH-R_4$ oder $-CH_2-CH_2-R_4$ ist, worin $R_4$ ein Wasserstoffatom, ein Halogenatom, einen Trialkylsilylrest mit 3 bis 12 Kohlenstoffatomen, einen geraden oder verzweigten Alkylrest mit 1 bis 6 Kohlenstoffatomen oder einen Phenylrest bedeutet, wobei diese Alkyl- und Phenylreste gegebenenfalls substituiert sind, die Wellenlinie in 13-Stellung bedeutet, daß der Rest $R_1$ in alpha- oder beta-Stellung sein kann.

2. Produkte der allgemeinen Formel (I), wie in Anspruch 1 definiert, entsprechend der Formel (I'):

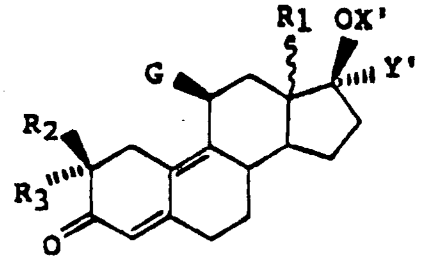

(I')

worin

entweder X' ein Wasserstoffatom bedeutet und Y' in diesem Fall $Y'_A$ bedeutet, wobei $Y'_A$ eine Gruppe

-B'-O-CO-A'-Z'

ist, worin B' einen der zweiwertigen Reste -CH = CH-$CH_2$- oder -C≡C-$CH_2$- darstellt, A' einen der zweiwertigen Reste $-(CH_2)_n-$ bedeutet, worin n einen der Werte von 2 bis 6 bedeutet oder einen ortho-, meta- oder para-Phenylenrest und Z' bedeutet die Carboxy- oder Sulfofunktionen oder deren Natriumsalz;
oder X' bedeutet $X'_B$, wobei $X'_B$ eine Gruppe:

$-CO-(CH_2)_n-Z'$

ist, worin n und Z' wie vorstehend definiert sind und Y' bedeutet dann $Y'_B$, wobei $Y'_B$ eine der Gruppen -C≡C-$R'_4$ oder -CH = CH-$R'_4$ ist, worin $R'_4$ ein Wasserstoffatom, ein Halogenatom, einen Trimethylsilylrest oder einen Methylrest bedeutet, gegebenenfalls substituiert durch ein oder mehrere Halogenatome, einen Hydroxyrest oder durch eine Alkyloxy-, Alkylthio- oder Alkylaminogruppe, enthaltend 1 bis 4 Kohlenstoffatome, Dialkylamino, enthaltend 2 bis 8 Kohlenstoffatome oder Trialkylsilyl, enthaltend 3 bis 12 Kohlenstoffatome.

**3.** Die Produkte der allgemeinen Formel (I), wie in den Ansprüchen 1 und 2 definiert, worin G einen Arylrest bedeutet, substituiert durch ein Halogenatom, einen Arylrest, einen Acylrest enthaltend 1 bis 8 Kohlenstoffatome oder durch eine der Funktionen $-NH_2$, -NHR' oder -NR'R'', worin R' und R'', die identisch oder verschieden sind, einen Phenylrest darstellen oder einen primären, sekundären oder tertiären Alkylrest mit 1 bis 8 Kohlenstoffatomen, enthaltend gegebenenfalls ein oder mehrere Heteroatome, ausgewählt aus den Sauerstoff-, Stickstoff- oder Schwefelatomen, oder gegebenenfalls substituiert durch einen Heterocyclus, oder R' und R'' bedeuten mit dem Stickstoffatom, mit dem sie verbunden sind, einen heterocyclischen Rest, gegebenenfalls substituiert und gegebenenfalls enthaltend ein anderes Heteroatom, ausgewählt unter den Stickstoff-, Sauerstoff- und Schwefelatomen, oder auch durch eine der Funktionen -OR''' oder -SR''', worin R''' einen Phenylrest oder einen primären, sekundären oder tertiären Alkylrest mit 1 bis 8 Kohlenstoffatome darstellt, gegebenenfalls enthaltend ein oder mehrere Heteroatome, ausgewählt unter den Sauerstoff-, Stickstoff- oder Schwefelatomen, oder gegebenenfalls substituiert durch einen Heterocyclus, oder G bedeutet einen 2-, 3- oder 4-Pyridylrest.

**4.** Die Produkte der allgemeinen Formel (I), wie in Anspruch 3 definiert, worin G einen Phenylrest bedeutet, der in 4-Stellung substituiert ist durch einen Rest Amino, (Methylamino), (Dimethylamino) oder sein N-Oxid, (Diethylamino), (Dipropylamino), (N-Methylethylamino), (N-Methylisopropylamino), (N-Methylisobutylamino), (N-Methylisopentylamino), 1-Pyrrolidinyl, [2-(Dimethylamino)-N-methylethylamino], [N-Methyl-2-(1-pyrrolidinyl)-ethylamino], N-Methyl-2-(4-morpholinyl)-ethylamino, (4-Methyl-1-piperazinyl), Formyl, Acetyl, Methoxy, Phenoxy, [2-(Dimethylamino)ethoxy], [2-(1-Pyrrolidinyl)-ethoxy], [2-(4-Morpholinyl)-ethoxy], (Methylthio), (Ethylthio), (Isopentylthio), [2-(Dimethylamino)-ethylthio], [2-(1-Pyrrolidinyl)-ethylthio], [2-(4-Morpholinyl)-ethylthio], (Trimethylsilyl), Methyl, Isopropyl, [(Dimethylamino)-methyl], oder durch eines der Atome Fluor, Chlor oder Brom.

**5.** Die Produkte der allgemeinen Formel (I), wie in einem der Ansprüche 1 bis 4 definiert, worin $R_1$ einen Methylrest in 13-beta-Stellung und $R_2$ und $R_3$ jeweils ein Wasserstoffatom bedeuten.

**6.** Die Produkte der allgemeinen Formel (I), wie in Anspruch 5 definiert, deren Namen folgendermaßen sind:
- Natrium-21-chloro-11-beta-[4-(dimethylamino)-phenyl]-3-oxo-19-nor-17-alpha-pregna-4,9-dien-20-in-17-beta-yl-succinat,
- Natrium-11-beta-[4-(methylthio)-phenyl]-3-oxo-17-alpha-(1-propinyl)-estra-4,9-dien-17-beta-yl-succinat.

**7.** Verfahren zur Herstellung der Produkte der allgemeinen Formel (I), wie in Anspruch 1 definiert, dadurch gekennzeichnet, daß:

A - die Produkte der Formel ($II_A$) oder der Formel ($II_B$):

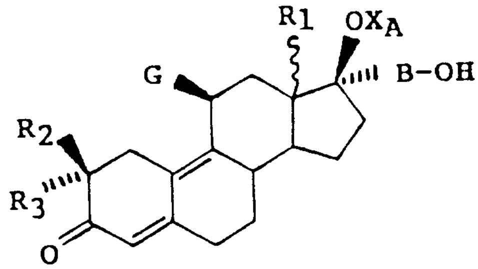

($II_A$)

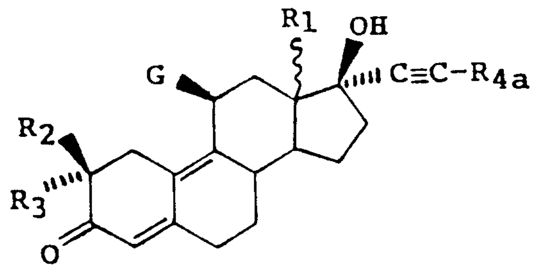

($II_B$)

worin $R_1$, $R_2$, $R_3$ und G die in Anspruch 1 angegebene Bedeutung haben und $R_{4a}$ die in Anspruch 1 für $R_4$ angegebenen Werte aufweist sowie diese Werte, worin die reaktiven Funktionen geschützt sind, in einem neutralen Lösungsmittel und in Gegenwart einer Base unterworfen werden

a) der Einwirkung eines Produkts der Formel ($III_1$):

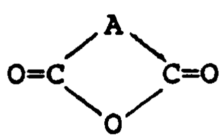

($III_1$)

um, falls notwendig, nach Entfernung der Schutzgruppen der geschützten reaktiven Funktionen und gewünschtenfalls Salzbildung der Carboxyfunktion, die Produkte der allgemeinen Formel (I) zu erhalten entsprechend den Formeln ($I_{A1}$) und ($I_{B1}$):

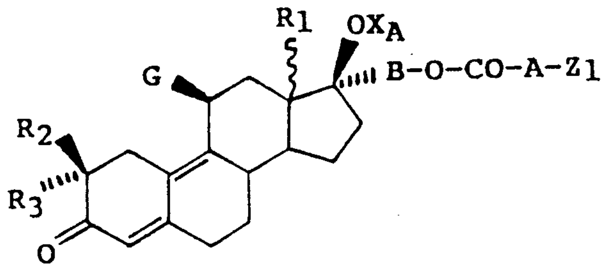

($I_{A1}$)

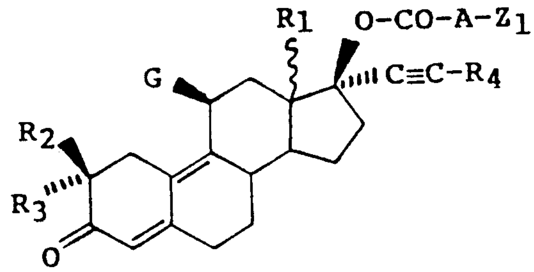

($I_{B1}$)

worin $Z_1$ einen Carboxyrest gegebenenfalls in Salzform bedeutet,
b) der Einwirkung eines Produkts der Formel ($III_2$):

HOOC-A-U ($III_2$)

oder auch eines funktionellen Derivats dieser Säure, worin U eine der Gruppen -COOH, -$COOR_5$, worin $R_5$ einen Alkylrest mit 1 bis 6 Kohlenstoffatomen oder einen Arylalkylrest mit 7 bis 12 Kohlenstoffatomen bedeutet, oder -SH darstellt, um, falls notwendig, nach Schutzgruppenabspaltung der geschützten reaktiven Funktionen, die Produkte der Formeln ($IV_A$) bzw. ($IV_B$) zu erhalten:

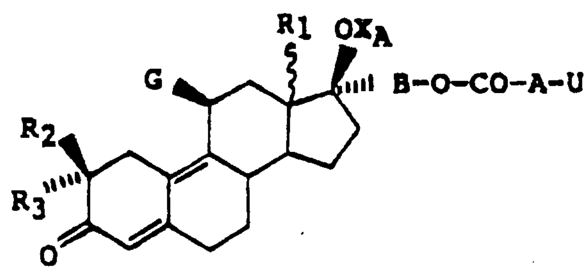

($IV_A$)

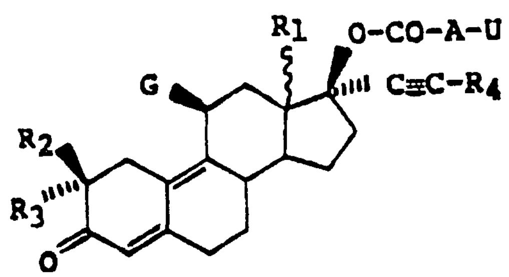

($IV_B$)

Produkte der Formeln ($IV_A$) und ($IV_B$), die:

- im Falle, daß U die freie Carboxygruppe bedeutet, nach eventueller Salzbildung den Produkten der Formeln ($I_{A1}$) und ($I_{B1}$) entsprechen,
- im Falle, daß U die Gruppe -$COOR_5$ bedeutet, den Produkten der Formeln ($IV_C$) und ($IV_D$) entsprechen:

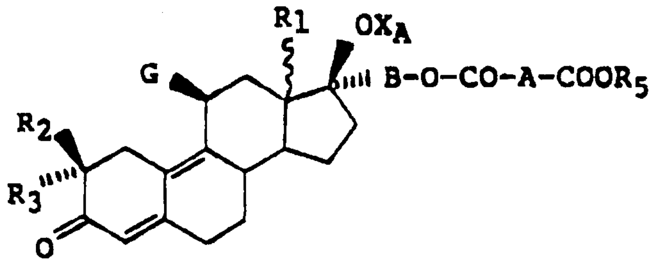

(IV_C)

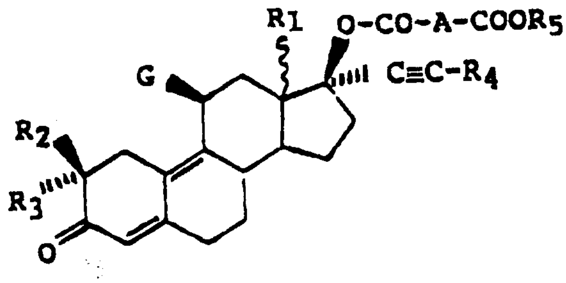

(IV_D)

Produkte der Formeln ($IV_C$) und ($IV_D$), welche hydrolysiert oder verseift werden, um die Produkte der Formeln ($I_{A1}$) bzw. ($I_{B1}$) zu erhalten,

- im Falle, daß U die Gruppe -SH bedeutet, den Produkten der Formeln ($IV_E$) und ($IV_F$) entsprechen:

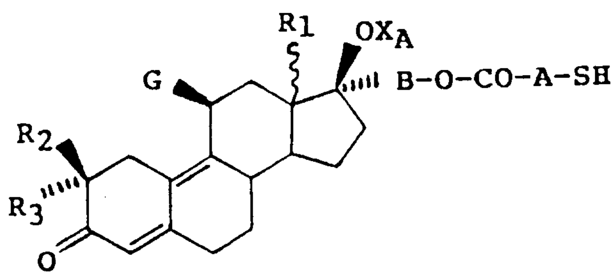

($IV_E$)

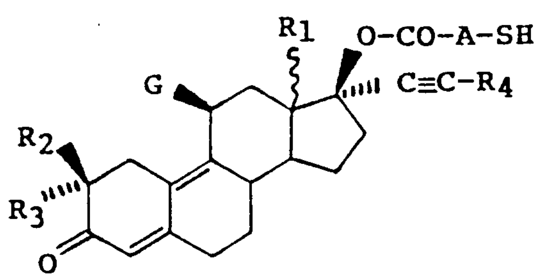

($IV_F$)

Produkte der Formeln ($IV_E$) und ($IV_F$), welche oxidiert und gewünschtenfalls in Salzform gebracht werden, um die Produkte der allgemeinen Formel (I) zu erhalten, entsprechend den Formeln ($I_{A2}$) bzw. ($I_{B2}$):

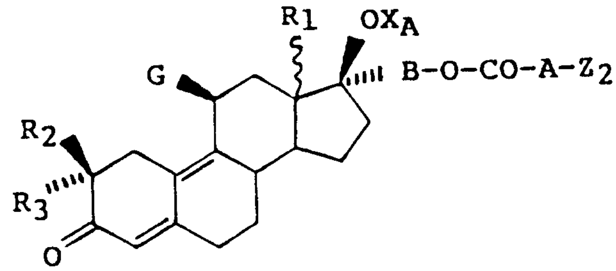

$(I_{A2})$

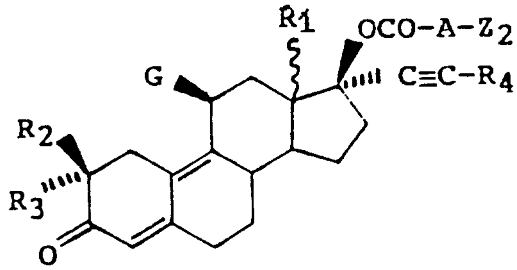

$(I_{B2})$

worin $Z_2$ eine Sulfogruppe, gegebenenfalls in Salzform, darstellt,

c) oder der Einwirkung eines Produkts der Formel ($III_3$):

$HOOC\text{-}A\text{-}SO_2Cl$ ($III_3$)

oder auch eines funktionellen Derivats dieser Säure, um, falls notwendig nach Schutzgruppenabspaltung der reaktiven Funktionen, die in $R_{4a}$ enthalten sind und gewünschtenfalls Salzbildung, die Produkte der Formeln ($I_{A2}$) bzw. ($I_{B2}$) zu erhalten;

und daß:

B - die Produkte der Formel ($I_{B1}$), ($I_{B2}$) und ($IV'_D$) gewünschtenfalls unterworfen werden:

a) <u>entweder</u> einem Hydrierungsmittel für die Dreifachbindungen, um die Produkte der Formeln ($I_{B3}$), ($I_{B4}$) bzw. ($V_D$) zu erhalten:

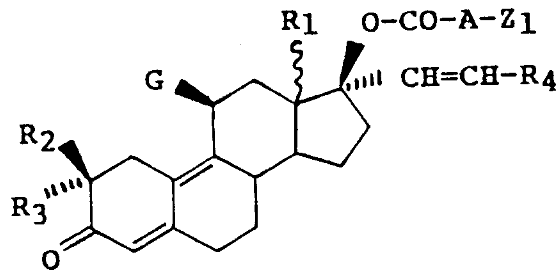

$(I_{B3})$

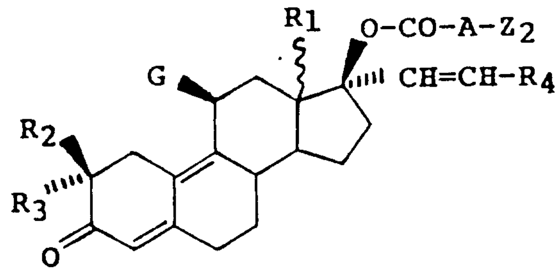

$(I_{B4})$

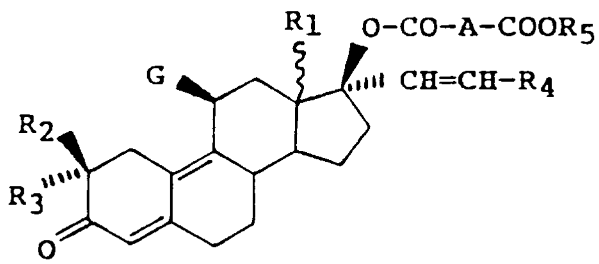

$(V_D)$

Produkte, welche gewünschtenfalls einem Hydrierungsmittel für Doppelbindungen unterworfen werden, um Produkte der Formel $(I_{B5})$, $(I_{B6})$ bzw. $(VI_D)$ zu erhalten:

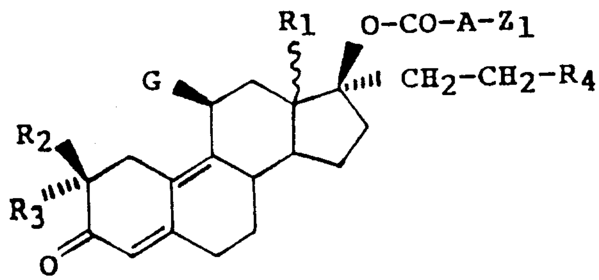

$(I_{B5})$

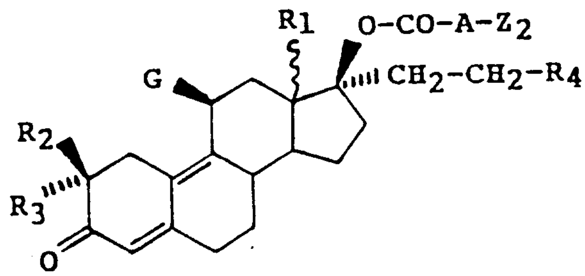

$(I_{B6})$

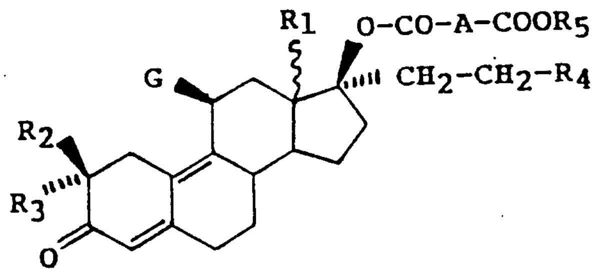

$(VI_D)$

oder einem direkten Hydrierungsmittel für Dreifachbindungen zu Einfachbindungen, um die Produkte der Formeln $(I_{B5})$,$(I_{B6})$ bzw. $(VI_D)$ zu erhalten;

b) die Produkte der Formeln $(V_D)$ und $(VI_D)$ werden entweder hydrolysiert oder verseift, um die Produkte der Formeln $(I_{B3})$ bzw. $(I_{B5})$ zu erhalten.

**8.** Verfahren gemäß Anspruch 7 zur Herstellung der Produkte der Formel (I'), wie in Anspruch 2 definiert, dadurch gekennzeichnet, daß man:

a) entweder das Produkt der Formel $(II'_A)$ oder $(II'_B)$:

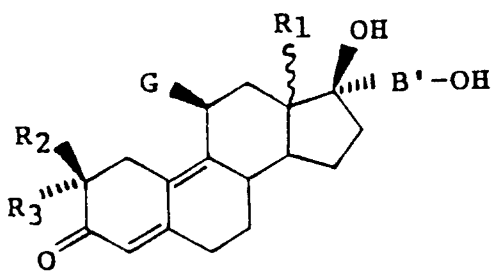

$(II'_A)$

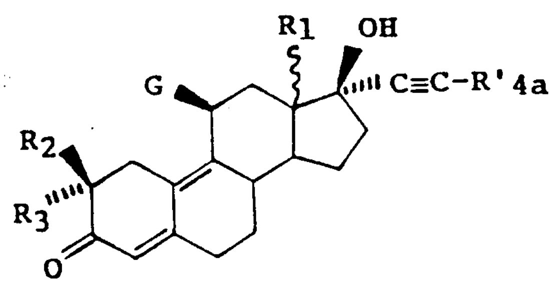

$(II'_B)$

worin $R_1$, $R_2$, $R_3$ und G die in Anspruch 2 angegebenen Werte haben und $R'_{4a}$ die in Anspruch 2 für $R'_4$ angegebenen Werte hat sowie diese Werte, worin die Hydroxylfunktionen geschützt sind, in einem neutralen Lösungsmittel und in Gegenwart einer Base der Einwirkung eines Produkts der Formel $(III'_1)$:

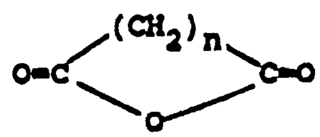

(III'$_1$)

unterwirft worin n einen der Werte 2 bis 6 hat, um, falls notwendig nach Schutzgruppenabspaltung der in R'$_4$ enthaltenen geschützten Hydroxylfunktion und gewünschtenfalls Salzbildung der freien Carboxylfunktion durch Einwirkung einer Natriumbicarbonatlösung, das Produkt der Formel (I'$_{A1}$) bzw. (I'$_{B1}$) zu erhalten:

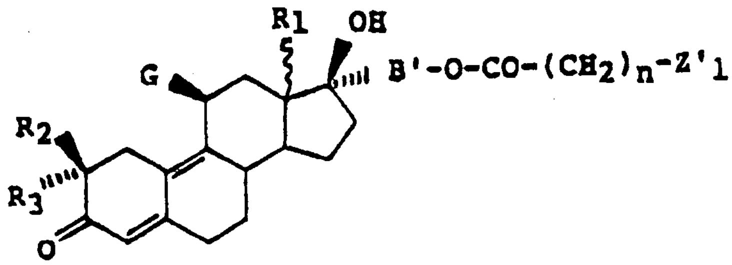

(I'$_{A1}$)

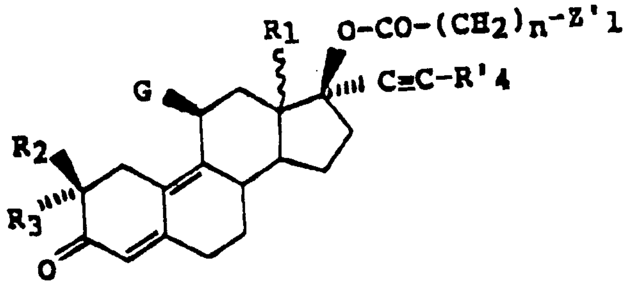

(I'$_{B1}$)

worin Z'$_1$ eine freie Carboxylgruppe oder ihr Natriumsalz darstellt;
b) oder man unterwirft das Produkt der Formel (II'$_A$), wie vorstehend definiert, der Einwirkung eines Produkts der Formel (III'$_3$):

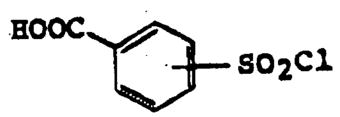

(III'$_3$)

in Gegenwart einer Stickstoffbase, um, falls notwendig und gewünscht, Salzbildung mit einer Natriumbicarbonatlösung, ein Produkt der Formel (I'$_{A2}$) zu erhalten:

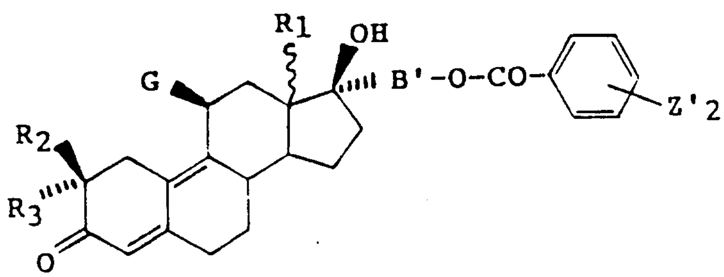

(I'$_{A2}$)

worin Z'$_2$ eine Sulfogruppe oder deren Natriumsalz bedeutet; Produkte der Formel (I'$_{B1}$), die man

gewünschtenfalls der Einwirkung von Wasserstoff in Gegenwart eines Katalysators unterwirft, um die Produkte der Formel ($I'_{B3}$) zu erhalten:

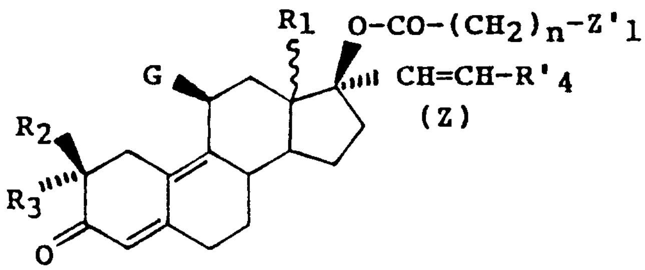

($I'_{B3}$)

**9.** Als Arzneimittel die Produkte der allgemeinen Formel (I), wie in Anspruch 1 definiert.

**10.** Als Arzneimittel die Produkte der allgemeinen Formel (I), wie in einem der Ansprüche 2 bis 6 definiert.

**11.** Pharmazeutische Zusammensetzungen, die als Wirkstoff mindestens eines der gemäß Anspruch 9 oder 10 definierten Arzneimittel enthalten.

**12.** Als neue industrielle Produkte die Produkte der Formeln ($IV_C$), ($IV_D$), ($IV_E$), ($IV_F$), ($V_D$) und ($VI_D$).

**Patentansprüche für folgenden Vertragsstaat : ES**

**1.** Verfahren zur Herstellung der Produkte der allgemeinen Formel (I):

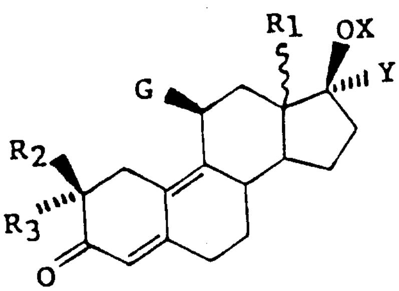

(I)

worin $R_1$ einen aliphatischen Kohlenwasserstoffrest, enthaltend 1 bis 8 Kohlenstoffatome, bedeutet, $R_2$ und $R_3$, die identisch oder verschieden sind, bedeuten ein Wasserstoffatom oder einen Alkylrest mit 1 bis 4 Kohlenstoffatomen, G bedeutet eine Kohlenwasserstoffgruppe mit 1 bis 18 Kohlenstoffatomen und gegebenenfalls einem oder mehreren identischen oder verschiedenen Heteroatom(en), gebunden an den Steroidkern durch ein Kohlenstoffatom, und
entweder X bedeutet $X_A$, wobei $X_A$ ein Wasserstoffatom, ein Alkylrest mit 1 bis 8 Kohlenstoffatomen, ein Arylalkylrest mit 7 bis 15 Kohlenstoffatomen oder ein Acylrest mit 1 bis 8 Kohlenstoffatomen ist, und Y bedeutet in diesem Fall $Y_A$,wobei $Y_A$ eine Gruppe

-B-O-CO-A-Z

ist, worin B einen gesättigten oder ungesättigten, geraden oder verzweigten zweiwertigen aliphatischen Rest mit 1 bis 8 Kohlenstoffatomen bedeutet, A bedeutet entweder einen gesättigten oder ungesättigten, geraden oder verzweigten zweiwertigen aliphatischen Rest mit 1 bis 6 Kohlenstoffatomen, und gegebenenfalls unterbrochen oder beendet durch einen zweiwertigen aromatischen Rest, oder A bedeutet einen zweiwertigen aromatischen Rest, und Z bedeutet eine der Funktionen -COOH oder $-SO_3H$, wobei diese Funktionen gegebenenfalls als Salz in Form eines Alkali- oder Erdalkalimetallsalzes, eines Ammoniumsalzes oder eines Aminsalzes vorliegen können,

oder X bedeutet $X_B$, wobei $X_B$ eine Gruppe

-CO-A-Z

ist, worin A und Z wie vorstehend definiert sind, und Y bedeutet dann $Y_B$, wobei $Y_B$ eine der Gruppen ausgewählt aus $-C{\equiv}C-R_4$, $-CH=CH-R_4$ oder $-CH_2-CH_2-R_4$ ist, worin $R_4$ ein Wasserstoffatom, ein Halogenatom, einen Trialkylsilylrest mit 3 bis 12 Kohlenstoffatomen, einen geraden oder verzweigten Alkylrest mit 1 bis 6 Kohlenstoffatomen oder einen Phenylrest bedeutet, wobei diese Alkylreste und das Phenyl gegebenenfalls substituiert sind, die Wellenlinie in 13-Stellung bedeutet, daß der Rest $R_1$ sich in alpha- oder beta-Stellung befinden kann, dadurch gekennzeichnet, daß

A - die Produkte der Formel ($II_A$) oder der Formel ($II_B$):

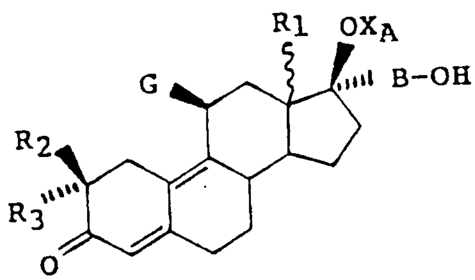

($II_A$)

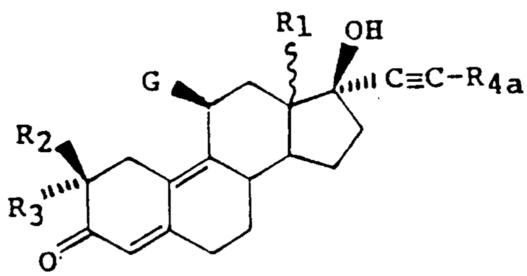

($II_B$)

worin $R_1$, $R_2$, $R_3$ und G die in Anspruch 1 angegebene Bedeutung haben und $R_{4a}$ die in Anspruch 1 für $R_4$ angegebenen Werte aufweist sowie diese Werte, worin die reaktiven Funktionen geschützt sind, in einem neutralen Lösungsmittel und in Gegenwart einer Base unterworfen werden

a) der Einwirkung eines Produkts der Formel ($III_1$):

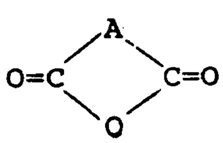

($III_1$)

um, falls notwendig nach Entfernung der Schutzgruppen der geschützten reaktiven Funktionen und gewünschtenfalls Salzbildung der Carboxyfunktion, die Produkte der allgemeinen Formel (I) zu erhalten entsprechend den Formeln ($I_{A1}$) und ($I_{B1}$):

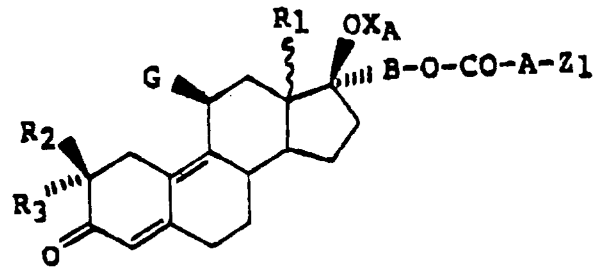

$(I_{A1})$

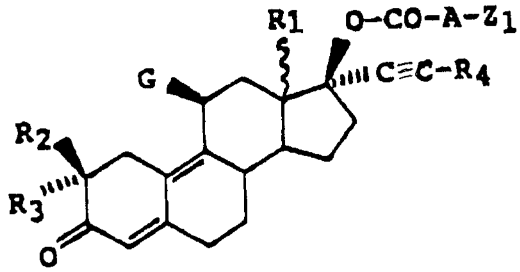

$(I_{B1})$

worin $Z_1$ einen Carboxyrest gegebenenfalls in Salzform bedeutet,
b) der Einwirkung eines Produkts der Formel ($III_2$):

HOOC-A-U ($III_2$)

oder auch eines funktionellen Derivats dieser Säure, worin U eine der Gruppen -COOH, -$COOR_5$, worin $R_5$ einen Alkylrest mit 1 bis 6 Kohlenstoffatomen oder einen Arylalkylrest mit 7 bis 12 Kohlenstoffatomen bedeutet, oder -SH darstellt, um, falls notwendig nach Schutzgruppenabspaltung der geschützten reaktiven Funktionen, die Produkte der Formeln ($IV_A$) bzw. ($IV_B$) zu erhalten:

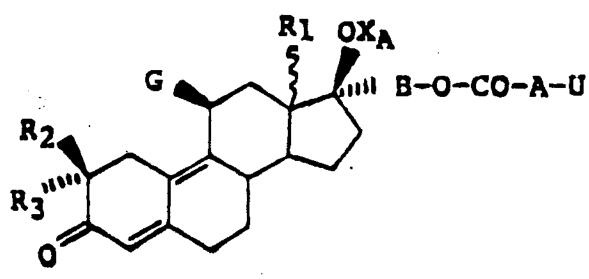

$(IV_A)$

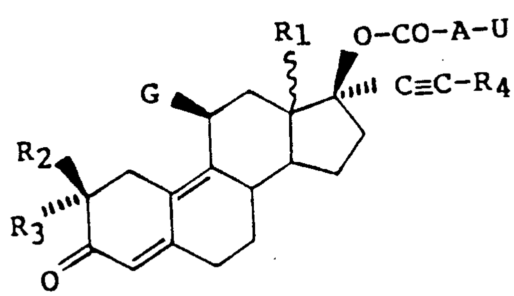

$(IV_B)$

Produkte der Formeln ($IV_A$) und ($IV_B$), die:
- im Falle, daß U die freie Carboxygruppe bedeutet, nach eventueller Salzbildung den Produkten der Formeln ($I_{A1}$) und ($I_{B1}$) entsprechen,

- im Falle, daß U die Gruppe -$COOR_5$ bedeutet, den Produkten der Formeln ($IV_C$) und ($IV_D$) entsprechen:

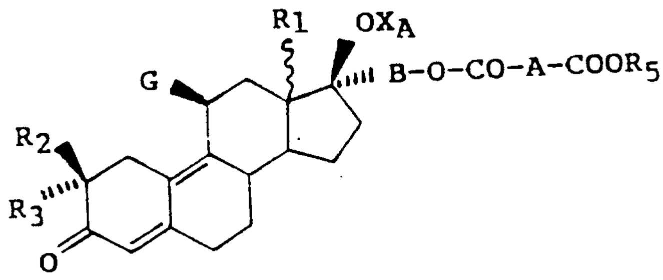

($IV_C$)

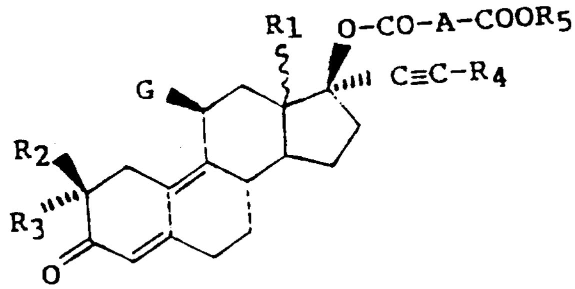

($IV_D$)

Produkte der Formeln ($IV_C$) und ($IV_D$), welche hydrolysiert oder verseift werden, um die Produkte der Formeln ($I_{A1}$) bzw. ($I_{B1}$) zu erhalten,

- im Falle, daß U die Gruppe -SH bedeutet, den Produkten der Formeln ($IV_E$) und ($IV_F$) entsprechen:

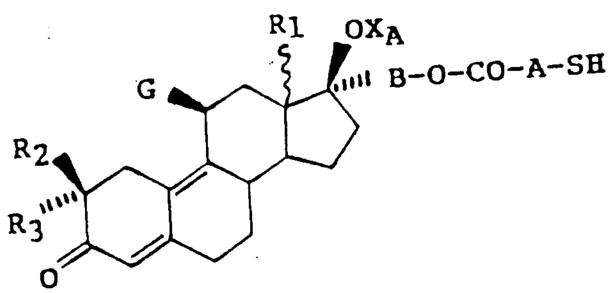

($IV_E$)

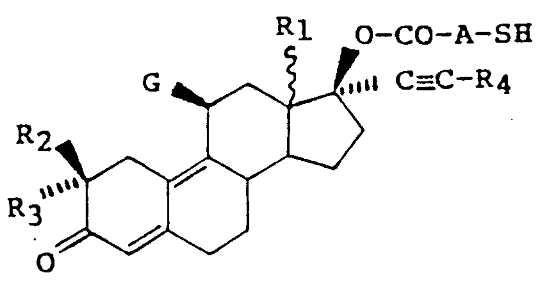

($IV_F$)

Produkte der Formeln ($IV_E$) und ($IV_F$), welche oxidiert und gewünschtenfalls in Salzform gebracht werden, um die Produkte der allgemeinen Formel (I) zu erhalten, entsprechend den Formeln ($I_{A2}$) bzw. ($I_{B2}$):

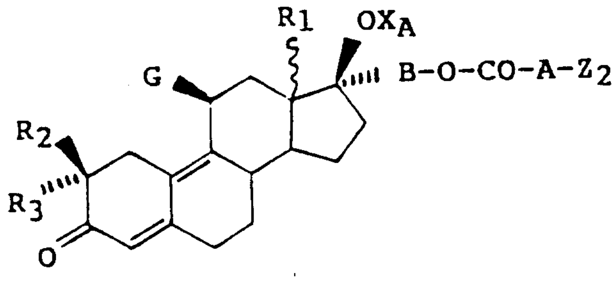

$(I_{A2})$

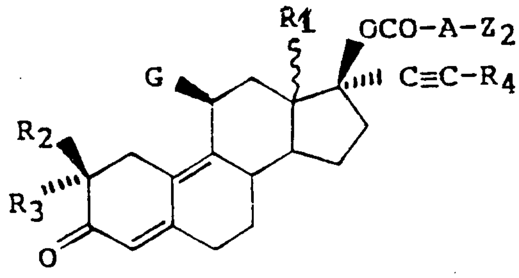

$(I_{B2})$

worin $Z_2$ eine Sulfogruppe, gegebenenfalls in Salzform, darstellt,

c) oder der Einwirkung eines Produkts der Formel ($III_3$):

$HOOC\text{-}A\text{-}SO_2Cl$ ($III_3$)

oder auch eines funktionellen Derivats dieser Säure, um, falls notwendig nach Schutzgruppenabspaltung der reaktiven Funktionen, die in $R_{4a}$ enthalten sind und gewünschtenfalls Salzbildung, die Produkte der Formeln ($I_{A2}$) bzw. ($I_{B2}$) zu erhalten;

und daß:

B - die Produkte der Formel ($I_{B1}$), ($I_{B2}$) und ($IV'_D$) gewünschtenfalls unterworfen werden:

a) entweder einem Hydrierungsmittel für die Dreifachbindungen, um die Produkte der Formeln ($I_{B3}$), ($I_{B4}$) bzw. ($V_D$) zu erhalten:

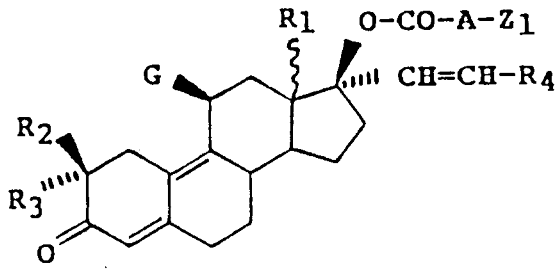

($I_{B3}$)

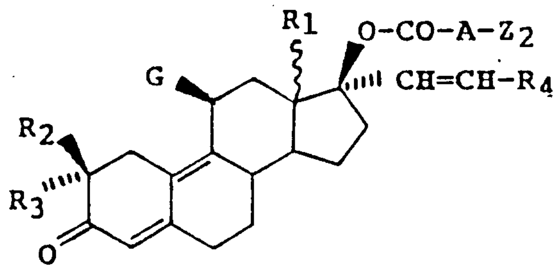

($I_{B4}$)

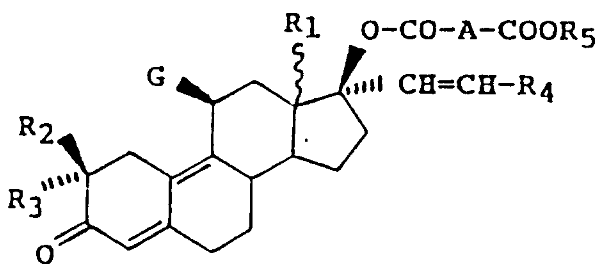

($V_D$)

Produkte, welche gewünschtenfalls einem Hydrierungsmittel für Doppelbindungen unterworfen werden, um Produkte der Formel ($I_{B5}$), ($I_{B6}$) bzw. ($VI_D$) zu erhalten:

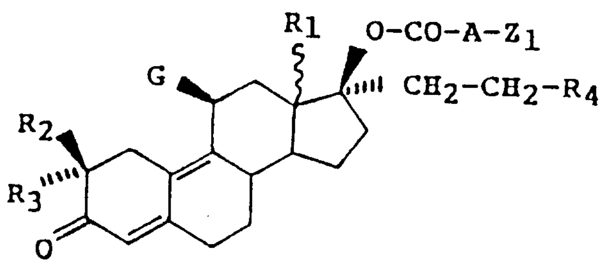

($I_{B5}$)

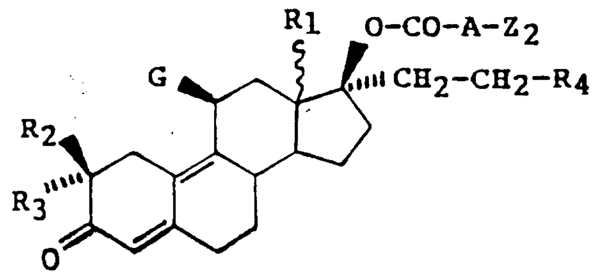

$(I_{B6})$

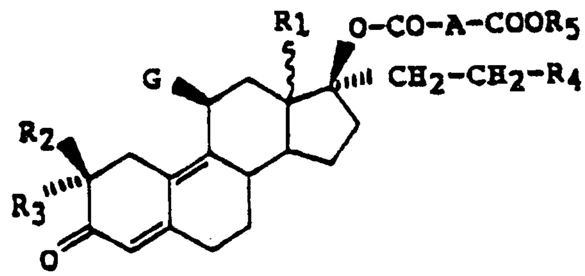

$(VI_D)$

oder einem direkten Hydrierungsmittel für Dreifachbindungen zu Einfachbindungen, um die Produkte der Formeln $(I_{B5})$,$(I_{B6})$ bzw. $(VI_D)$ zu erhalten;

b) die Produkte der Formeln $(V_D)$ und $(VI_D)$ werden entweder hydrolysiert oder verseift, um die Produkte der Formeln $(I_{B3})$ bzw. $(I_{B5})$ zu erhalten.

**2.** Verfahren zur Herstellung der Produkte der allgemeinen Formel (I), wie in Anspruch 1 definiert, entsprechend der Formel (I'):

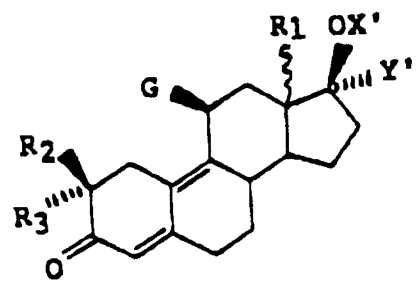

(I')

worin

entweder X' ein Wasserstoffatom bedeutet und Y' in diesem Fall $Y'_A$ bedeutet, wobei $Y'_A$ eine Gruppe

-B'-O-CO-A'-Z'

ist, worin B' einen der zweiwertigen Reste $-CH=CH-CH_2-$ oder $-C{\equiv}C-CH_2-$ darstellt, A' einen der zweiwertigen Reste $-(CH_2)_n-$ bedeutet, worin n einen der Werte von 2 bis 6 bedeutet oder einen ortho-, meta- oder para-Phenylenrest und Z' bedeutet die Carboxy- oder Sulfofunktionen oder deren Natriumsalz;

oder X' bedeutet $X'_B$, wobei $X'_B$ eine Gruppe:

$-CO-(CH_2)_n-Z'$

ist, worin n und Z' wie vorstehend definiert sind und Y' bedeutet dann $Y'_B$, wobei $Y'_B$ eine der Gruppen $-C{\equiv}C-R'_4$ oder $-CH=CH-R'_4$ ist, worin $R'_4$ ein Wasserstoffatom, ein Halogenatom, einen Trimethylsilylrest oder einen Methylrest bedeutet, gegebenenfalls substituiert durch ein oder mehrere Halogenatome, einen Hydroxyrest oder durch eine Alkyloxy-, Alkylthio- oder Alkylaminogruppe, enthaltend 1 bis 4

Kohlenstoffatome, Dialkylamino, enthaltend 2 bis 8 Kohlenstoffatome oder Trialkylsilyl, enthaltend 3 bis 12 Kohlenstoffatome, dadurch gekennzeichnet, daß man:

a) entweder das Produkt der Formel ($II'_A$) oder ($II'_B$):

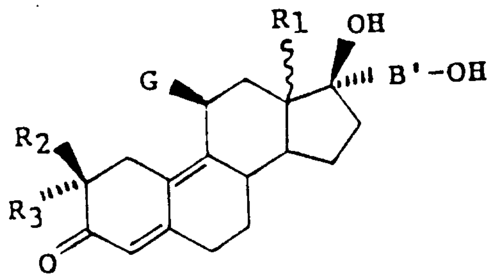

($II'_A$)

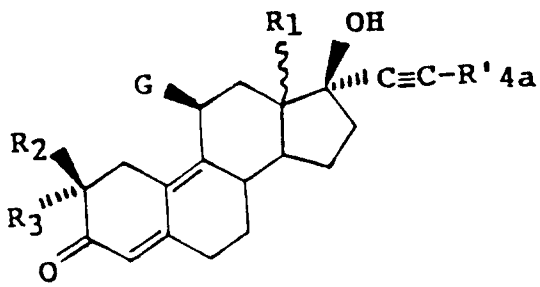

($II'_B$)

worin $R_1$, $R_2$, $R_3$ und G die in Anspruch 2 angegebenen Werte haben und $R'_{4a}$ den in Anspruch 2 für $R'_4$ angegebenen Wert hat sowie diese Werte, worin die Hydroxylfunktionen geschützt sind, in einem neutralen Lösungsmittel und in Gegenwart einer Base der Einwirkung eines Produkts der Formel ($III'_1$):

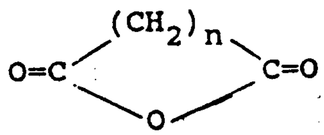

($III'_1$)

unterwirft, worin n einen der Werte 2 bis 6 hat, um, falls notwendig nach Schutzgruppenabspaltung der in $R'_4$ enthaltenen geschützten Hydroxylfunktion und gewünschtenfalls Salzbildung der freien Carboxylfunktion durch Einwirkung einer Natriumbicarbonatlösung, das Produkt der Formel ($I'_{A1}$) bzw. ($I'_{B1}$) zu erhalten:

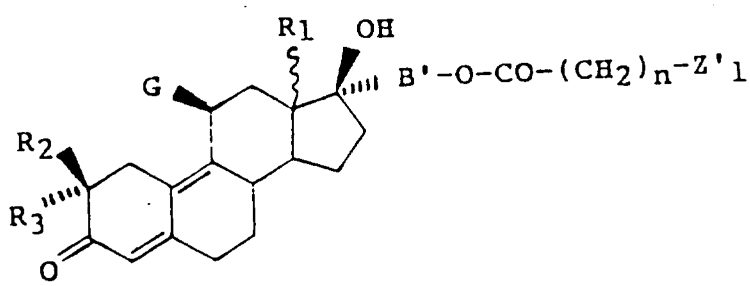

($I'_{A1}$)

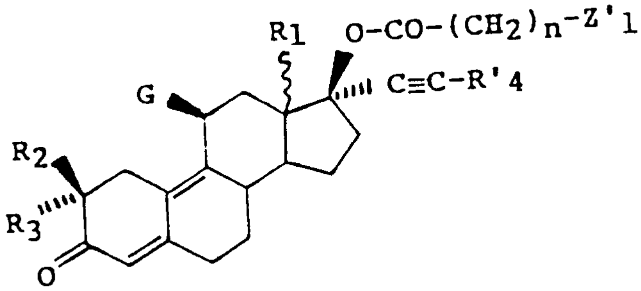

(I'$_{B1}$)

worin Z'$_1$ eine freie Carboxylgruppe oder ihr Natriumsalz darstellt;

b) oder man unterwirft das Produkt der Formel (II'$_A$), wie vorstehend definiert, der Einwirkung eines Produkts der Formel (III'$_3$):

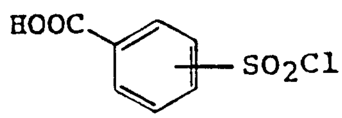

(III'$_3$)

in Gegenwart einer Stickstoffbase, um, falls notwendig und gewünscht, Salzbildung mit einer Natriumbicarbonatlösung, ein Produkt der Formel (I'$_{A2}$) zu erhalten:

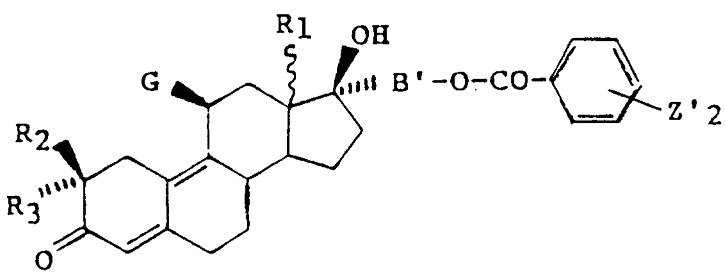

(I'$_{A2}$)

worin Z'$_2$ eine Sulfogruppe oder deren Natriumsalz bedeutet; Produkte der Formel (I'$_{B1}$), die man gewünschtenfalls der Einwirkung von Wasserstoff in Gegenwart eines Katalysators unterwirft, um die Produkte der Formel (I'$_{B3}$) zu erhalten:

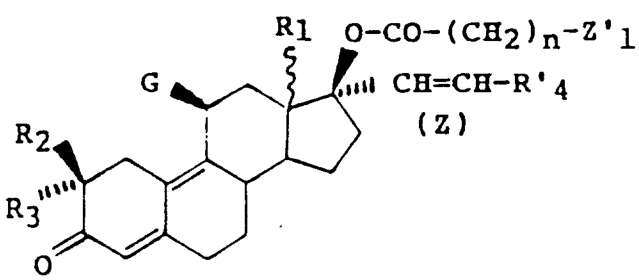

(I'$_{B3}$)

**3.** Verfahren gemäß Anspruch 1 oder 2, dadurch gekennzeichnet, daß man zu Beginn ein Produkt der Formel (II$_A$) oder (II$_B$) oder (II'$_A$) oder (II'$_B$) verwendet, worin G bedeutet einen Arylrest, substituiert durch ein Halogenatom, einen Arylrest, einen Acylrest mit 1 bis 8 Kohlenstoffatomen, oder durch eine der Funktionen -NH$_2$, -NHR' oder -NR'R'', worin R' und R'', die identisch oder verschieden sind, einen Phenylrest darstellen oder einen primären, sekundären oder tertiären Alkylrest mit 1 bis 8 Kohlenstoffatomen, enthaltend gegebenenfalls ein oder mehrere Heteroatome, ausgewählt unter den Sauerstoff-, Stickstoff- oder Schwefelatomen, oder gegebenenfalls substituiert durch einen Heterocyclus oder R' und R" bedeuten mit dem Stickstoffatom, an das sie gebunden sind, einen heterocyclischen Rest, gegebenenfalls substituiert und gegebenenfalls enthaltend ein weiteres Heteroatom ausgewählt unter den Stickstoff-, Sauerstoff- und Schwefelatomen, oder auch durch eine der Funktionen -OR''' oder -SR''' worin R''' einen Phenylrest oder einen primären, sekundären oder tertiären Alkylrest mit 1 bis 8 Kohlenstoffatomen bedeutet, enthaltend gegebenenfalls ein oder mehrere Heteroatom(e) ausgewählt unter den Sauerstoff-, Stickstoff- und Schwefelatomen oder gegebenenfalls substituiert durch einen Heterocyclus, oder G bedeutet einen 2-, 3- oder 4-Pyridylrest.

**4.** Verfahren gemäß Anspruch 3, dadurch gekennzeichnet, daß man zu Beginn ein Produkt der Formel ($II_A$) oder ($II_B$) oder ($II'_A$) oder ($II'_B$) verwendet, worin G einen Phenylrest bedeutet, substituiert in 4-Stellung durch einen Rest Amino, (Methylamino), (Dimethylamino) oder sein N-Oxid, (Diethylamino), (Dipropylamino), (N-Methylethylamino), (N-Methylisopropylamino), (N-Methylisobutylamino), (N-Methylisopentylamino), 1-Pyrrolidinyl, [2-(Dimethylamino)-N-methylethylamino ], [N-Methyl-2-(1-pyrrolidinyl)-ethylamino], [N-Methyl-2-(4-morpholinyl)-ethylamino], (4-Methyl-1-piperazinyl),Formyl, Acetyl, Methoxy, Phenoxy, [2-(Dimethylamino)ethoxy], [2-(1-Pyrrolidinyl)-ethoxy], [2-(4-Morpholinyl)-ethoxy], (Methylthio), (Ethylthio), (Isopentylthio), [2-(Dimethylamino)-ethylthio],[2-(1-Pyrrolidinyl)-ethylthio], [2-(4-Morpholinyl)-ethylthio], (Trimethylsilyl), Methyl, Isopropyl, [(Dimethylamino)-methyl], oder durch eines der Fluor-, Chlor- oder Bromatome.

**5.** Verfahren gemäß einem der Ansprüche 1 bis 4, dadurch gekennzeichnet, daß man zu Beginn ein Produkt der Formel ($II_A$) oder ($II_B$) oder ($II'_A$) oder ($II'_B$) verwendet, worin $R_1$ einen Methylrest in 13-beta-Stellung und $R_2$ und $R_3$ jeweils ein Wasserstoffatom bedeuten.

**Patentansprüche für folgenden Vertragsstaat : GR**

**1.** Verfahren zur Herstellung der Produkte der allgemeinen Formel (I):

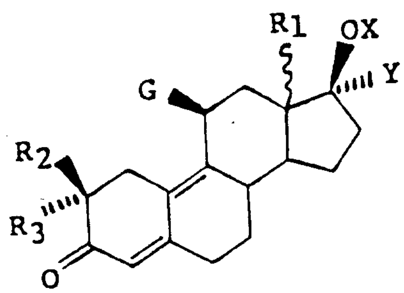

(I)

worin $R_1$ einen aliphatischen Kohlenwasserstoffrest, enthaltend 1 bis 8 Kohlenstoffatome, bedeutet, $R_2$ und $R_3$, die identisch oder verschieden sind, bedeuten ein Wasserstoffatom oder einen Alkylrest mit 1 bis 4 Kohlenstoffatomen, G bedeutet eine Kohlenwasserstoffgruppe mit 1 bis 18 Kohlenstoffatomen und gegebenenfalls einem oder mehreren identischen oder verschiedenen Heteroatom(en), gebunden an den Steroidkern durch ein Kohlenstoffatom, und
entweder X bedeutet $X_A$, wobei $X_A$ ein Wasserstoffatom, ein Alkylrest mit 1 bis 8 Kohlenstoffatomen, ein Arylalkylrest mit 7 bis 15 Kohlenstoffatomen oder ein Acylrest mit 1 bis 8 Kohlenstoffatomen ist, und Y bedeutet in diesem Fall $Y_A$,wobei $Y_A$ eine Gruppe

-B-O-CO-A-Z

ist, worin B einen gesättigten oder ungesättigten, geraden oder verzweigten zweiwertigen aliphatischen Rest mit 1 bis 8 Kohlenstoffatomen bedeutet, A bedeutet entweder einen gesättigten oder ungesättigten, geraden oder verzweigten zweiwertigen aliphatischen Rest mit 1 bis 6 Kohlenstoffatomen, und gegebenenfalls unterbrochen oder beendet durch einen zweiwertigen aromatischen Rest, oder A bedeutet einen zweiwertigen aromatischen Rest, und Z bedeutet eine der Funktionen -COOH oder $-SO_3H$, wobei diese Funktionen gegebenenfalls als Salz in Form eines Alkali- oder Erdalkalimetallsalzes, eines Ammoniumsalzes oder eines Aminsalzes vorliegen können,
oder X bedeutet $X_B$, wobei $X_B$ eine Gruppe

-CO-A-Z

ist, worin A und Z wie vorstehend definiert sind, und Y bedeutet dann $Y_B$, wobei $Y_B$ eine der Gruppen ausgewählt aus $-C{\equiv}C-R_4$, $-CH=CH-R_4$ oder $-CH_2-CH_2-R_4$ ist, worin $R_4$ ein Wasserstoffatom, ein Halogenatom, einen Trialkylsilylrest mit 3 bis 12 Kohlenstoffatomen, einen geraden oder verzweigten Alkylrest mit 1 bis 6 Kohlenstoffatomen oder einen Phenylrest bedeutet, wobei diese Alkylreste und das Phenyl gegebenenfalls substituiert sind, die Wellenlinie in 13-Stellung bedeutet, daß der Rest $R_1$ sich in alpha- oder beta-Stellung befinden kann, dadurch gekennzeichnet, daß

A - die Produkte der Formel ($II_A$) oder der Formel ($II_B$):

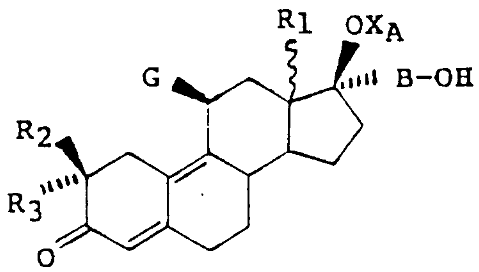

($II_A$)

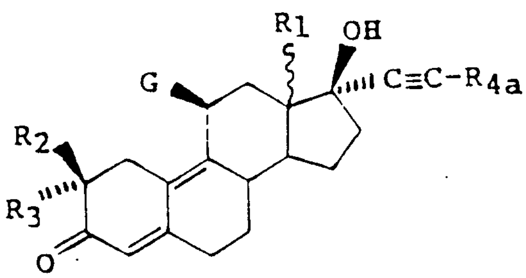

($II_B$)

worin $R_1$, $R_2$, $R_3$ und G die in Anspruch 1 angegebene Bedeutung haben und $R_{4a}$ die in Anspruch 1 für $R_4$ angegebenen Werte aufweist sowie diese Werte, worin die reaktiven Funktionen geschützt sind, in einem neutralen Lösungsmittel und in Gegenwart einer Base unterworfen werden

a) der Einwirkung eines Produkts der Formel ($III_1$):

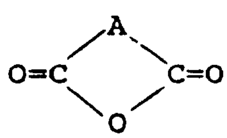

($III_1$)

um, falls notwendig nach Entfernung der Schutzgruppen der geschützten reaktiven Funktionen und gewünschtenfalls Salzbildung der Carboxyfunktion, die Produkte der allgemeinen Formel (I) zu erhalten entsprechend den Formeln ($I_{A1}$) und ($I_{B1}$):

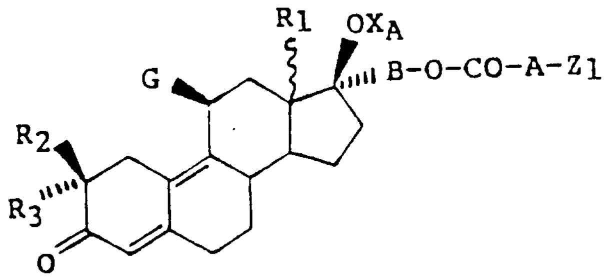

($I_{A1}$)

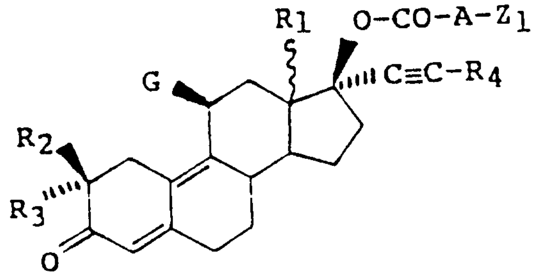

($I_{B1}$)

worin $Z_1$ einen Carboxyrest gegebenenfalls in Salzform bedeutet,
b) der Einwirkung eines Produkts der Formel ($III_2$):

HOOC-A-U ($III_2$)

oder auch eines funktionellen Derivats dieser Säure, worin U eine der Gruppen -COOH, $-COOR_5$, worin $R_5$ einen Alkylrest mit 1 bis 6 Kohlenstoffatomen oder einen Arylalkylrest mit 7 bis 12 Kohlenstoffatomen bedeutet, oder -SH darstellt, um, falls notwendig nach Schutzgruppenabspaltung der geschützten reaktiven Funktionen, die Produkte der Formeln ($IV_A$) bzw. ($IV_B$) zu erhalten:

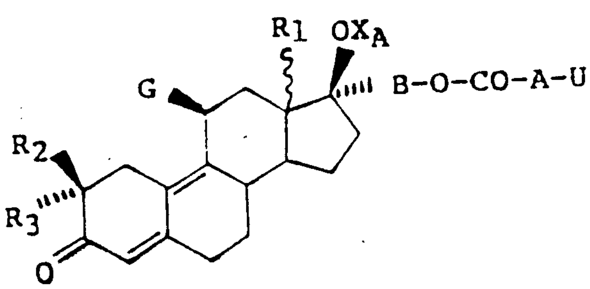

($IV_A$)

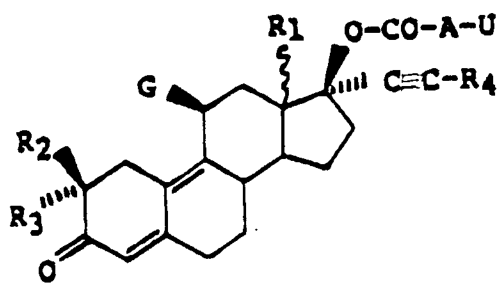

($IV_B$)

Produkte der Formeln ($IV_A$) und ($IV_B$), die:

- im Falle, daß U die freie Carboxygruppe bedeutet, nach eventueller Salzbildung den Produkten der Formeln ($I_{A1}$) und ($I_{B1}$) entsprechen,

- im Falle, daß U die Gruppe $-COOR_5$ bedeutet, den Produkten der Formeln ($IV_C$) und ($IV_D$) entsprechen:

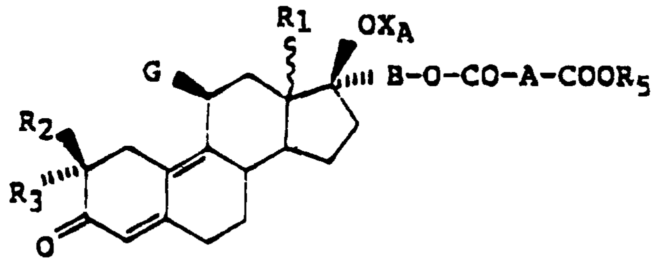

($IV_C$)

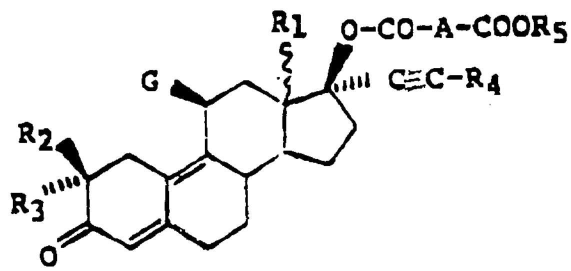

($IV_D$)

Produkte der Formeln ($IV_C$) und ($IV_D$), welche hydrolysiert oder verseift werden, um die Produkte der Formeln ($I_{A1}$) bzw. ($I_{B1}$) zu erhalten,

- im Falle, daß U die Gruppe -SH bedeutet, den Produkten der Formeln ($IV_E$) und ($IV_F$) entsprechen:

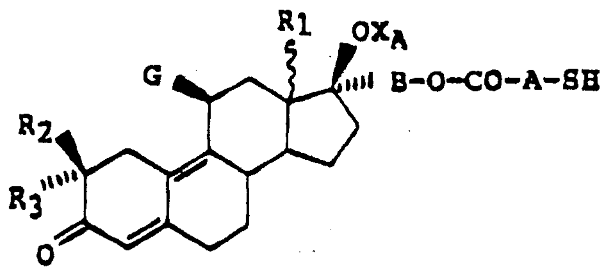

($IV_E$)

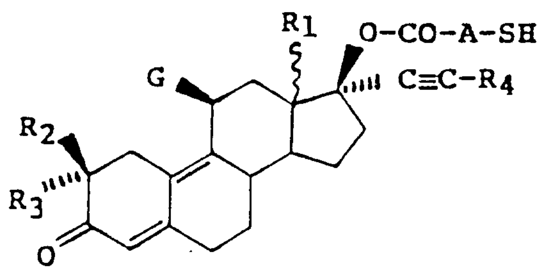

($IV_F$)

Produkte der Formeln ($IV_E$) und ($IV_F$), welche oxidiert und gewünschtenfalls in Salzform gebracht werden, um die Produkte der allgemeinen Formel (I) zu erhalten, entsprechend den Formeln ($I_{A2}$) bzw. ($I_{B2}$):

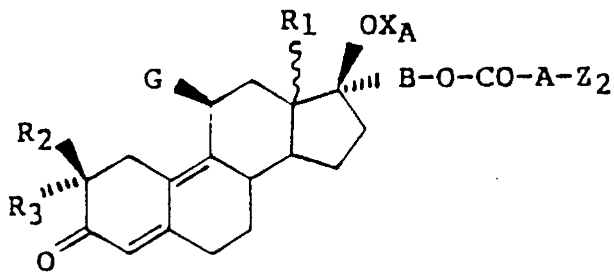

$(I_{A2})$

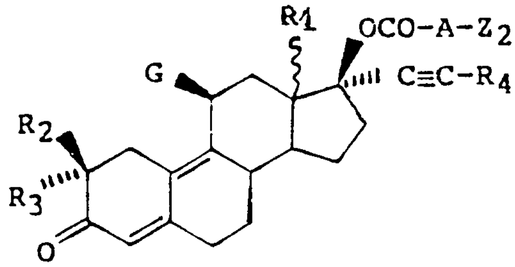

$(I_{B2})$

worin $Z_2$ eine Sulfogruppe, gegebenenfalls in Salzform, bedeutet,

c) oder der Einwirkung eines Produkts der Formel ($III_3$):

HOOC-A-$SO_2$Cl ($III_3$)

oder auch eines funktionellen Derivats dieser Säure, um, falls notwendig nach Schutzgruppenabspaltung der reaktiven Funktionen, die in $R_{4a}$ enthalten sind und gewünschtenfalls Salzbildung, die Produkte der Formeln ($I_{A2}$) bzw. ($I_{B2}$) zu erhalten;

und daß:

B - die Produkte der Formel ($I_{B1}$), ($I_{B2}$) und ($IV'_D$) gewünschtenfalls unterworfen werden:

a) entweder einem Hydrierungsmittel für die Dreifachbindungen, um die Produkte der Formeln ($I_{B3}$), ($I_{B4}$) bzw. ($V_D$) zu erhalten:

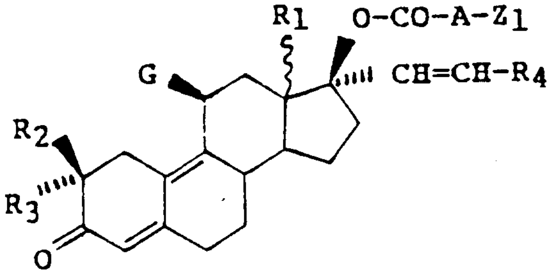

($I_{B3}$)

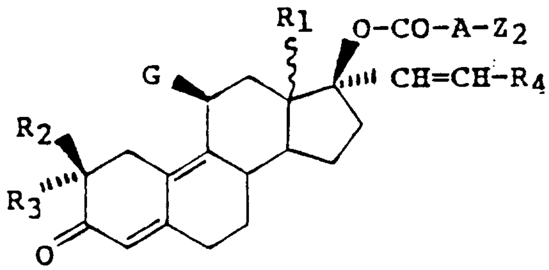

($I_{B4}$)

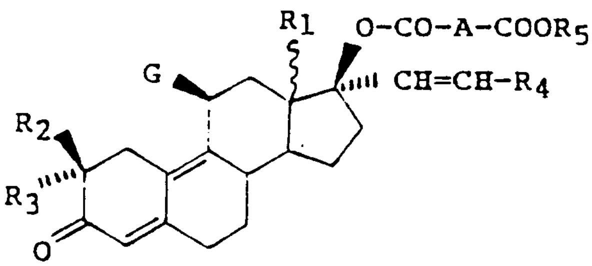

($V_D$)

Produkte, welche gewünschtenfalls einem Hydrierungsmittel für Doppelbindungen unterworfen werden, um Produkte der Formel ($I_{B5}$), ($I_{B6}$) bzw. ($VI_D$) zu erhalten:

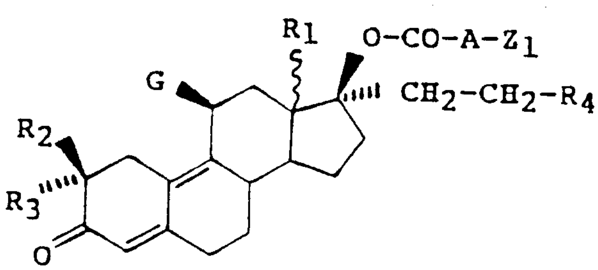

($I_{B5}$)

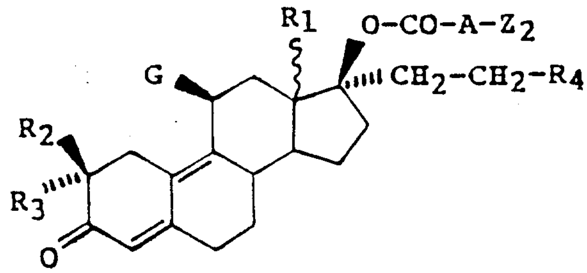

$(I_{B6})$

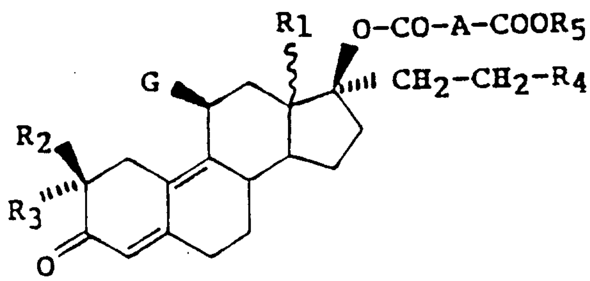

$(VI_D)$

oder einem direkten Hydrierungsmittel für Dreifachbindungen zu Einfachbindungen, um die Produkte der Formeln $(I_{B5})$,$(I_{B6})$ bzw. $(VI_D)$ zu erhalten;

b) die Produkte der Formeln $(V_D)$ und $(VI_D)$ werden entweder hydrolysiert oder verseift, um die Produkte der Formeln $(I_{B3})$ bzw. $(I_{B5})$ zu erhalten.

**2.** Verfahren zur Herstellung der Produkte der allgemeinen Formel (I), wie in Anspruch 1 definiert, entsprechend der Formel (I'):

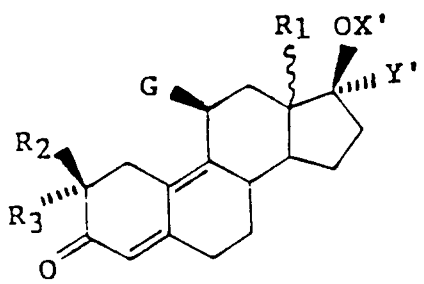

(I')

worin

entweder X' ein Wasserstoffatom bedeutet und Y' in diesem Fall $Y'_A$ bedeutet, wobei $Y'_A$ eine Gruppe

-B'-O-CO-A'-Z'

ist, worin B' einen der zweiwertigen Reste $-CH=CH-CH_2-$ oder $-C\equiv C-CH_2-$ darstellt, A' einen der zweiwertigen Reste $-(CH_2)_n-$ bedeutet, worin n einen der Werte von 2 bis 6 bedeutet oder einen ortho-, meta- oder para-Phenylenrest und Z' bedeutet die Carboxy- oder Sulfofunktionen oder deren Natriumsalz;

oder X' bedeutet $X'_B$, wobei $X'_B$ eine Gruppe:

$-CO-(CH_2)_n-Z'$

ist, worin n und Z' wie vorstehend definiert sind und Y' bedeutet dann $Y'_B$, wobei $Y'_B$ eine der Gruppen

$-C{\equiv}C-R'_4$ oder $-CH=CH-R'_4$ ist, worin $R'_4$ ein Wasserstoffatom, ein Halogenatom, einen Trimethylsilylrest oder einen Methylrest bedeutet, gegebenenfalls substituiert durch ein oder mehrere Halogenatome, einen Hydroxyrest oder durch eine Alkyloxy-, Alkylthio- oder Alkylaminogruppe, enthaltend 1 bis 4 Kohlenstoffatome, Dialkylamino, enthaltend 2 bis 8 Kohlenstoffatome oder Trialkylsilyl, enthaltend 3 bis 12 Kohlenstoffatome, dadurch gekennzeichnet, daß man:

a) entweder das Produkt der Formel ($II'_A$) oder ($II'_B$):

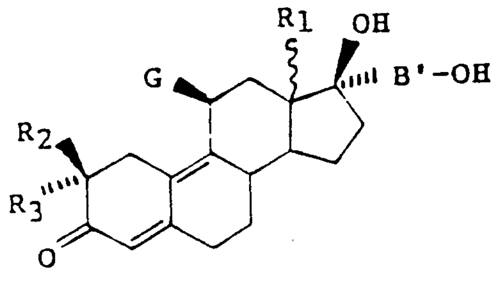

($II'_A$)

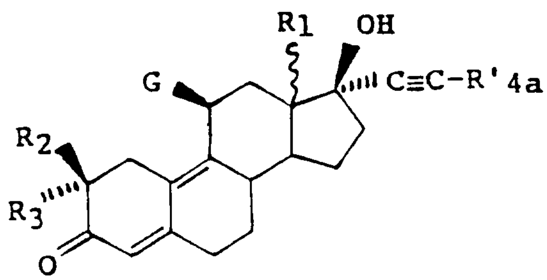

($II'_B$)

worin $R_1$, $R_2$, $R_3$ und G die in Anspruch 2 angegebenen Bedeutungen haben und $R'_{4a}$ die in Anspruch 2 für $R'_4$ angegebenen Bedeutungen hat sowie diese Werte, worin die Hydroxylfunktionen geschützt sind, in einem neutralen Lösungsmittel und in Gegenwart einer Base der Einwirkung eines Produkts der Formel ($III'_1$):

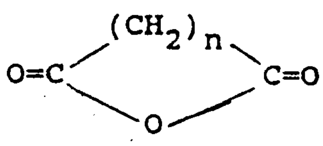

($III'_1$)

unterwirft, worin n einen der Werte 2 bis 6 hat, um, falls notwendig nach Schutzgruppenabspaltung der in $R'_4$ enthaltenen geschützten Hydroxylfunktion und gewünschtenfalls Salzbildung der freien Carboxylfunktion durch Einwirkung einer Natriumbicarbonatlösung, das Produkt der Formel ($I'_{A1}$) bzw. ($I'_{B1}$) zu erhalten:

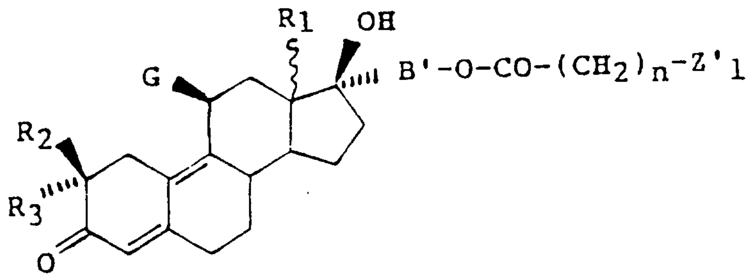

$(I'_{A1})$

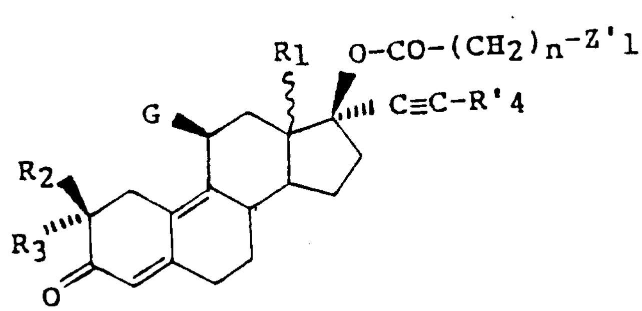

$(I'_{B1})$

worin $Z'_1$ eine freie Carboxylgruppe oder ihr Natriumsalz darstellt;

b) oder man unterwirft das Produkt der Formel $(II'_A)$, wie vorstehend definiert, der Einwirkung eines Produkts der Formel $(III'_3)$:

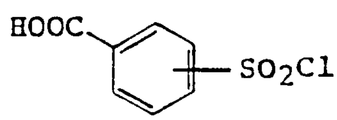

$(III'_3)$

in Gegenwart einer Stickstoffbase, um, falls notwendig und gewünscht, Salzbildung mit einer Natriumbicarbonatlösung, ein Produkt der Formel $(I'_{A2})$ zu erhalten:

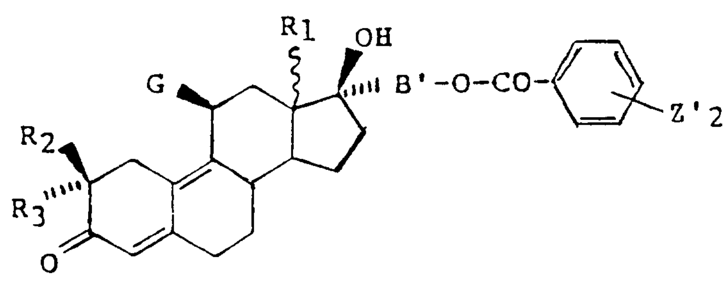

$(I'_{A2})$

worin $Z'_2$ eine Sulfogruppe oder deren Natriumsalz bedeutet; Produkt der Formel $(I'_{B1})$, das man gewünschtenfalls der Einwirkung von Wasserstoff in Gegenwart eines Katalysators unterwirft, um die Produkte der Formel $(I'_{B3})$ zu erhalten:

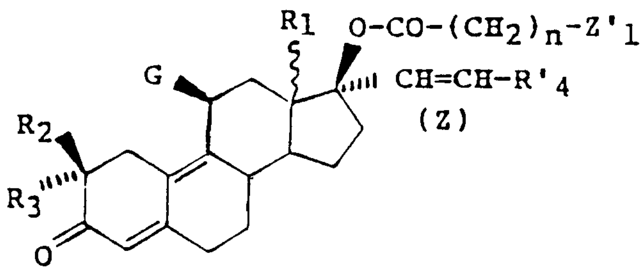

(I'$_{B3}$)

3. Verfahren gemäß Anspruch 1 oder 2, dadurch gekennzeichnet, daß man zu Beginn ein Produkt der Formel ($II_A$) oder ($II_B$) oder ($II'_A$) oder ($II'_B$) verwendet, worin G einen Arylrest, substituiert durch ein Halogenatom, einen Arylrest, einen Acylrest mit 1 bis 8 Kohlenstoffatomen, oder durch eine der Funktionen $-NH_2$, -NHR' oder -NR'R" bedeutet, worin R' und R", die identisch oder verschieden sind, einen Phenylrest darstellen oder einen primären, sekundären oder tertiären Alkylrest mit 1 bis 8 Kohlenstoffatomen, enthaltend gegebenenfalls ein oder mehrere Heteroatome, ausgewählt unter den Sauerstoff-, Stickstoff- oder Schwefelatomen, oder gegebenenfalls substituiert durch einen Heterocyclus, oder R' und R" bedeuten mit dem Stickstoffatom, an das sie gebunden sind, einen heterocyclischen Rest, gegebenenfalls substituiert und gegebenenfalls enthaltend ein weiteres Heteroatom ausgewählt unter den Stickstoff-, Sauerstoff- und Schwefelatomen, oder auch durch eine der Funktionen -OR''' oder -SR''', worin R''' einen Phenylrest oder einen primären, sekundären oder tertiären Alkylrest mit 1 bis 8 Kohlenstoffatomen bedeutet, enthaltend gegebenenfalls ein oder mehrere Heteroatom(e) ausgewählt unter den Sauerstoff-, Stickstoff- und Schwefelatomen oder gegebenenfalls substituiert durch einen Heterocyclus, oder G bedeutet einen 2-, 3- oder 4-Pyridylrest.

4. Verfahren gemäß Anspruch 3, dadurch gekennzeichnet, daß man zu Beginn ein Produkt der Formel ($II_A$) oder ($II_B$) oder ($II'_A$) oder ($II'_B$) verwendet, worin G einen Phenylrest bedeutet, substituiert in 4-Stellung durch einen Rest Amino, (Methylamino), (Dimethylamino) oder sein N-Oxid, (Diethylamino), (Dipropylamino), (N-Methylethylamino), (N-Methylisopropylamino), (N-Methylisobutylamino), (N-Methylisopentylamino), 1-Pyrrolidinyl, [2-(Dimethylamino)-N-methylethylamino], [N-Methyl-2-(1-pyrrolidinyl)-ethylamino], [N-Methyl-2-(4-morpholinyl)-ethylamino], (4-Methyl-1-piperazinyl),Formyl, Acetyl, Methoxy, Phenoxy, [2-(Dimethylamino)-ethoxy], [2-(1-Pyrrolidinyl)-ethoxy], [2-(4-Morpholinyl)-ethoxy], (Methylthio), (Ethylthio), (Isopentylthio), [2-(Dimethylamino)-ethylthio],[2-(1-Pyrrolidinyl)-ethylthio], [2-(4-Morpholinyl)-ethylthio], (Trimethylsilyl), Methyl, Isopropyl, (Dimethylamino)-methyl , oder durch eines der Fluor-, Chlor- oder Bromatome.

5. Verfahren gemäß einem der Ansprüche 1 bis 4, dadurch gekennzeichnet, daß man zu Beginn ein Produkt der Formel ($II_A$) oder ($II_B$) oder ($II'_A$) oder ($II'_B$) verwendet, worin $R_1$ einen Methylrest in 13-beta-Stellung und $R_2$ und $R_3$ jeweils ein Wasserstoffatom bedeuten.

6. Verfahren gemäß einem der Ansprüche 1 bis 5, dadurch gekennzeichnet, daß man eines der Produkte mit folgender Bezeichnung herstellt:
   - Natrium-21-chloro-11-beta-[4-(dimethylamino)-phenyl]-3-oxo-19-nor-17-alpha-pregna-4,9-dien-20-in-17-beta-yl-succinat;
   - Natrium-11-beta-[4-(methylthio)-phenyl]-3-oxo-17-alpha-(1-propinyl)-estra-4,9-dien-17-beta-yl-succinat.

7. Verfahren zur Herstellung von pharmazeutischen Zusammensetzungen, dadurch gekennzeichnet, daß man als Wirkstoff mindestens eines der Produkte der Formel (I) oder mindestens eines seiner pharmazeutisch annehmbaren Salze, wie in Anspruch 1 definiert, in einer für diesen Verwendungszweck bestimmten Form einsetzt.

8. Verfahren zur Herstellung von pharmazeutischen Zusammensetzungen, dadurch gekennzeichnet, daß man als Wirkstoff mindestens eines der Produkte der Formel (I') oder mindestens eines seiner pharmazeutisch annehmbaren Salze, wie in Anspruch 2 definiert, in einer für diesen Verwendungszweck bestimmten Form einsetzt.

**9.** Verfahren gemäß Anspruch 8, dadurch gekennzeichnet, daß der Wirkstoff ausgewählt ist aus der Gruppe bestehend aus den in Anspruch 6 bezeichneten Produkten und ihren pharmazeutisch annehmbaren Salzen.

**10.** Als neue industrielle Produkte die Produkte der Formeln ($IV_C$), ($IV_D$), ($IV_E$), ($IV_F$), ($V_D$) und ($VI_D$).